# EUROPEAN PATENT APPLICATION

(11) **EP 4 692 071 A1**
(43) Date of publication of application: **11.02.2026**
(21) Application number: 23930718.4
(22) Date of filing: 05.07.2023
(51) Int. Cl.: C07D 311/04, C07C 217/76, C07C 217/94, C07D 311/78, C07D 405/04, C07D 407/04, C07D 413/04, C07F 7/10, C09K 9/02, G02C 7/02, G02C 7/10, C07D 215/06, C07D 265/36, C07D 311/94, C07D 311/96, C09B 57/00

(54) **PHOTOCHROMIC COMPOUND, NAPHTHOL DERIVATIVE, CURABLE COMPOSITION, OPTICAL ARTICLE, LENS, AND EYEGLASS**

(30) Priority: 27.03.2023 JP 2023049959; 09.05.2023 JP 2023077412; 09.05.2023 JP 2023077411
(71) Applicant: TOKUYAMA CORPORATION, Yamaguchi 745-8648 (JP)
(72) Inventor: MIYAZAKI Masayuki, Shunan-shi, Yamaguchi 745-8648 (JP)
(74) Representative: Papula Oy
(86) International application number: PCT/JP2023/024969
(87) International publication number: WO 2024/202086

(57) **Abstract**

The purpose of the present invention is to provide: a photochromic compound having excellent temperature dependency and an excellent color fading speed; a naphthol derivative that can be an intermediate for the photochromic compound; and a curable composition, an optical article, a lens, and an eyeglass each including the photochromic compound. An embodiment of the present invention provides a photochromic compound having a skeleton represented by formula (1). In formula (1), M is C, Si, or Ge. Ring A is a substituted or unsubstituted aromatic ring, a substituted or unsubstituted aromatic heterocyclic ring, or a substituted or unsubstituted condensed polycyclic ring in which an aromatic ring or an aromatic heterocyclic ring and the substituted or unsubstituted aromatic ring or the substituted or unsubstituted aromatic heterocyclic ring are condensed. Z¹ is a group represented by formula (1a) or a group represented by formula (1b).

## Description

### TECHNICAL FIELD

The present invention relates to a photochromic compound, a naphthol derivative, a curable composition, an optical article, a lens, and eyeglasses.

### BACKGROUND ART

Photochromic compounds refer to compounds that can reversibly become two types of isomers with different absorption spectra by irradiation with light including ultraviolet ray, such as sunlight or mercury lamp light. Generally, when a colorless compound in a decolored state is irradiated with ultraviolet ray, the compound rapidly changes in color and isomerizes into a colored state (chromogenic reaction). Photochromic compounds have been researched and developed as materials for photochromic lenses.

In applications for such photochromic lenses, photochromic compounds require the following properties in some cases.
(I) Low coloring degree in the visible light region before UV irradiation (hereinafter referred to as "initial coloring").
(II) High rate at which the color optical density reaches saturation after the start of UV irradiation (hereinafter referred to as "color optical density").
(III) High rate at which the color optical density reaches saturation after the start of UV irradiation (hereinafter also referred to as "high chromogenic sensitivity").
(IV) High rate at which the compound returns to its original state after the stop of UV irradiation (hereinafter referred to as "color fading speed").
(V) High durability against the aforementioned repeated reversible actions.
(VI) High solubility in the lens matrix material, and high dispersibility in a cured product.

As photochromic compounds that can satisfy the above requirements, chromene compounds having an indeno(2,1-f)naphth(1,2-b)pyran structure as a basic skeleton are known. For example, a chromene compound represented by formula (A) below (Patent Document 1), a chromene compound represented by formula (B) below (Patent Document 2), and a chromene compound represented by formula (C) below (Patent Document 3) are known.

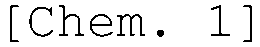

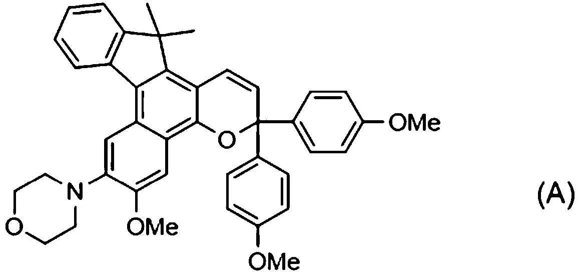

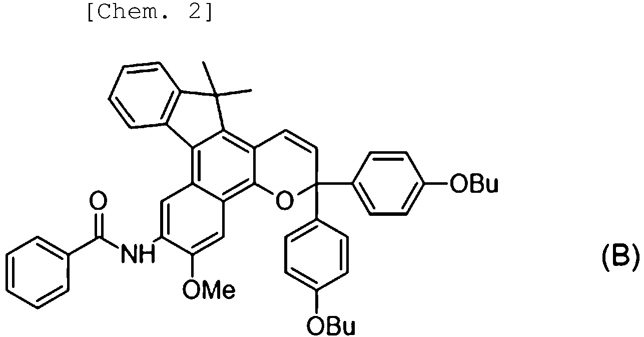

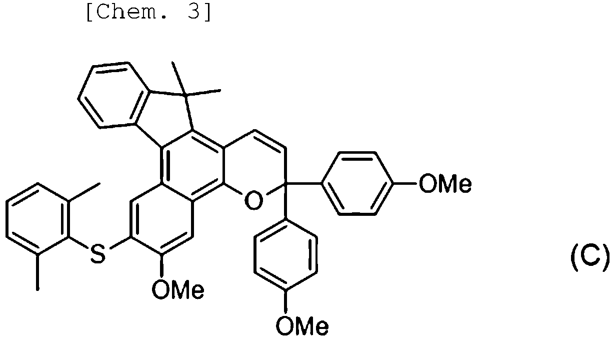

In recent years, the performance required for photochromic compounds is becoming more sophisticated. In addition to the above properties (I) to (VI), photochromic compounds are required to exhibit a high color optical density even at a high temperature e.g. even in summer. Hereinafter, such a property is also referred to as "low temperature dependence" in some cases. Generally, a high color optical density can be exhibited even at a high temperature by improving a thermal stability of a colored state, but a color fading speed is decreased. Thus, there is a trade-off relationship between the color optical density at a high temperature and the color fading speed. Photochromic compounds that can achieve both a high color optical density at a high temperature and the property (IV) have been required.

### Citation List

### Patent Documents

Patent Document 1: PCT International Publication No. WO2001/19813
Patent Document 2: PCT International Publication No. WO2019/228604
Patent Document 3: PCT International Publication No. WO2013/042800
Patent Document 4: PCT International Publication No. WO2015/035325
Patent Document 5: PCT International Publication No. WO2018/235771
Patent Document 6: PCT International Publication No. WO2012/102410
Patent Document 7: PCT International Publication No. WO2011/053615
Patent Document 8: PCT International Publication No. WO2011/034202
Patent Document 9: PCT International Publication No. WO2019/013249
Patent Document 10: PCT International Publication No. WO2016/143910
Patent Document 11: PCT International Publication No. WO2019/228604
Patent Document 12: PCT International Publication No. WO2019/013249
Patent Document 13: PCT International Publication No. WO2016/143910

### Non-Patent Documents

Non-Patent Document 1: Journal of Organic Chemistry, 69(10), pp. 3282-3293; 2004
Non-Patent Document 2: Synthetic Communications, 23(16), pp. 2241-2249 (1993)
Non-Patent Document 3: J. Am. Chem. Soc., 132(41), pp. 14324-14326 (2010)
Non-Patent Document 4: Org. Lett. 16, pp. 6492-6495 (2014)

### DISCLOSURE OF THE INVENTION

### Problems to be Solved by the Invention

An object of the present invention is to provide a photochromic compound excellent in temperature dependency and color fading speed, a naphthol derivative that can serve as an intermediate of this photochromic compound, as well as a curable composition, an optical article, a lens, and eyeglasses including this photochromic compound.

### Means for Solving the Problems

The present disclosure relates to a photochromic compound having a skeleton represented by formula (1) or (1A) below.

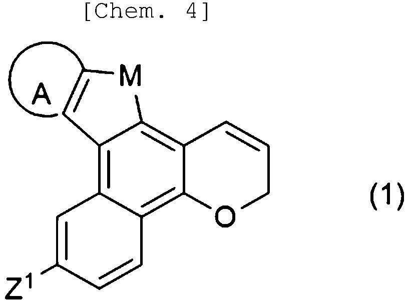

In formula (1), M is C, Si, or Ge. Ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Z¹ is a group represented by formula (1a) below or a group represented by formula (1b) below.

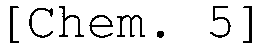

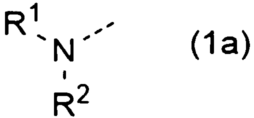

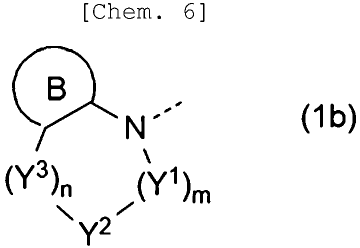

In formula (1a), R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents. R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below.

-Q¹-(X¹Q²)a-X²Q³ (2a)

In formula (2a), Q¹ is an alkylene group or a haloalkylene group,
Q² is an alkylene group or a haloalkylene group, and Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and a is 0, or 1 or more and 3 or less.

In formula (1b), ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Y¹ is a substituted or unsubstituted methylene group. Y² is a substituted or unsubstituted methylene group, O, S, SO₂, NR⁶⁰⁰, R⁶⁰¹C=CR⁶⁰², or CC. R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group. Y³ is a substituted or unsubstituted methylene group.
m is an integer of 1 to 4, n is an integer of 0 to 4, and m+n is an integer of 2 or more. 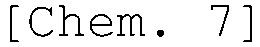 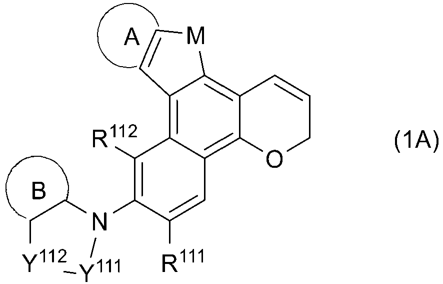 In formula (1A), M, ring A, and ring B are each independently the same as those in formula (1).

In formula (1A), Y¹¹¹ is a substituted or unsubstituted methylene group. Y¹¹² is a substituted or unsubstituted methylene group, an oxygen atom, a sulfur atom, NR⁶⁰⁰, or SO₂. R⁶⁰⁰ is the same as described above in formula (1b).

In formula (1A), R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) above.

Also, the present disclosure relates to a curable composition. The curable composition includes the photochromic compound described above, and at least one selected from a group consisting of a radical polymerizable monomer, a cationic polymerizable monomer, a compound having a polymerizable group, and a (thio)urethane(urea) polymer.

Also, the present disclosure relates to a cured product. The cured product is a cured product of the above-described curable composition.

Also, the present disclosure relates to an optical article. The optical article includes the above-described cured product of the curable composition.

Also, the present disclosure relates to a lens. The lens includes the above-described photochromic compound.

Also, the present disclosure relates to eyeglasses. The eyeglasses include the above-described lens.

Also, the present disclosure relates to a naphthol derivative. The naphthol derivative has a skeleton represented by formula (6) or (6A) below.

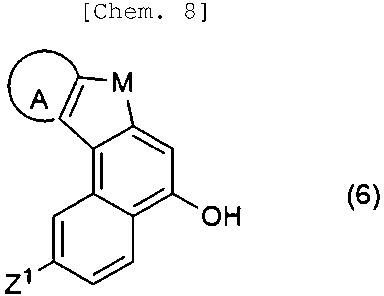

In formula (6), M, ring A, and Z¹ are each independently the same as those in formula (1) above.

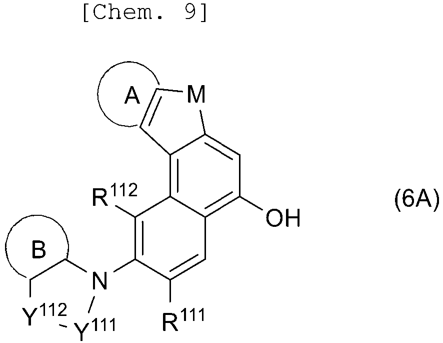

In formula (6A), M, ring A, ring B, Y¹¹¹, Y¹¹², R¹¹¹, and R¹¹² are each independently the same as those in formula (1A) above.

### Effects of the Invention

The present invention provides a photochromic compound excellent in temperature dependency and color fading speed, a naphthol derivative that can serve as an intermediate of this photochromic compound, as well as a curable composition, an optical article, a lens, and eyeglasses including this photochromic compound.

### BRIEF DESCRIPTION OF THE DRAWINGS

FIG. 1 is a graph presenting an example of a relationship between a color fading half-life at 23°C and a high-temperature color development rate of each photochromic laminate in Examples and Comparative Examples; and
FIG. 2 is a graph presenting another example of the relationship between the color fading half-life at 23°C and the high-temperature color development rate of each photochromic laminate in Examples and Comparative Examples.

### PREFERRED MODE FOR CARRYING OUT THE INVENTION

### [Photochromic Compound]

According to the embodiment, a photochromic compound having a skeleton represented by formula (1) below is provided.

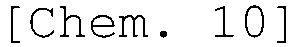

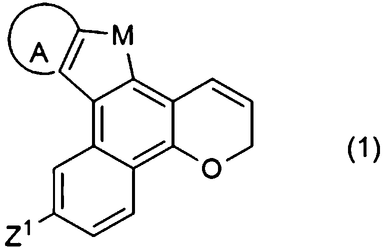

In formula (1), M is C, Si, or Ge. Ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Z¹ is a group represented by formula (1a) below or a group represented by formula (1b) below.

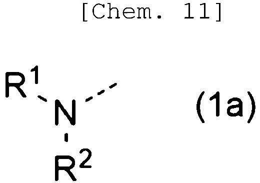

In formula (1a), R¹ is a substituted or unsubstituted aryl group or a substituted or unsubstituted heteroaryl group. R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below.

-Q¹-(X¹Q²) a-X²Q³ (2a)

In formula (2a), Q¹ is an alkylene group or a haloalkylene group. Q² is an alkylene group or a haloalkylene group. Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.
a is an integer of 0, or 1 or more and 3 or less.

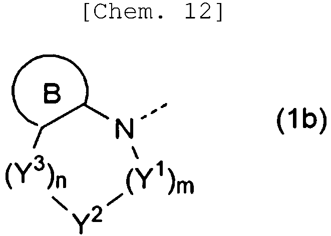

In formula (1b), ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Y¹ is a substituted or unsubstituted methylene group. Y² is a substituted or unsubstituted methylene group, O, S, SO₂, NR⁶⁰⁰, R⁶⁰¹C=CR⁶⁰², or CC. R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group. Y³ is a substituted or unsubstituted methylene group or an oxygen atom.
m is an integer of 1 to 4, n is an integer of 1 to 4, and m+n is an integer of 2 or more.

This photochromic compound is excellent in temperature dependency and color fading speed. Although the reason for this is not yet clear, the inventors consider that the reason is as follows.

For the substituent Z¹ of the photochromic compound according to the embodiment, formula (1a) or (1b) above is used. It is considered that formulas (1a) and (1b) above have a structure with an aryl group or heteroaryl group directly bonded to a nitrogen atom, and therefore can be conjugated with the skeleton represented by formula (1) and the substituent Z¹ via an orbit p on a nitrogen atom. Compounds having such a structure are considered to have an extended conjugation compared to compounds with the above formulas (1a) and (1b) not substituted. This extension increases the absorbancy even in a decolored state, suggesting that the color optical density can be improved even at a high temperature. Furthermore, electrons are delocalized by a resonance structure, suggesting that the durability can be improved.

Additionally, there is a tendency that, when the substitution position to which the substituent Z¹ binds is substituted with a substituent having a high electron-donating ability, the color fading speed of the photochromic compound decreases. As described above, the substituent Z¹ has an aryl group or heteroaryl group bonded to a nitrogen atom, suggesting that the electron-donating ability is decreased by the conjugation. Thus, in the photochromic compound having the substituent Z¹, the decrease in the color fading speed is expected to be suppressed.

Based on the above description, a cured product excellent in both the temperature dependency and the color fading speed as well as the durability can be achieved by using the photochromic compound according to the embodiment. Thus, such a photochromic compound is suitable for optical articles such as sunglasses that is used in environments with a significant temperature change.

The photochromic compound having the skeleton represented by formula (1) will be explained below in detail.

### <M>

In formula (1), M is C, Si, or Ge. M is preferably C.

### <Ring A>

In formula (1), ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Examples of the aromatic hydrocarbon ring include a benzene ring, a cyclotetradecaheptaene ring, and the like.

Examples of the aromatic heterocyclic ring include a furan ring, a thiophene ring, a pyridine ring, and the like.

Examples of the fused polycylic ring include a naphthalene ring, a fluorene ring, an anthracene ring, a phenanthrene ring, a tetracene ring, a pentacene ring, a benzopyrene ring, a chrysene ring, a pyrene ring, a triphenylene ring, a perylene ring, a benzofuran ring, a benzothiophene ring, a quinoline ring, an isoquinoline ring, an indole ring, a pyrimidine ring, a quinazoline ring, a pyridazine ring, a cinnoline ring, a phthalazine ring, a 1,2,3-, 1,2,4-, or 1,3,5-triazine ring, a carbazole ring, a benzoxazole ring, an isothiazole ring, and the like.

Above all, a benzene ring, a naphthalene ring, a fluorene ring, a phenanthrene ring, a pyrene ring, a furan ring, a thiophene ring, or a pyridine ring are preferable, a benzene ring, a naphthalene ring, a fluorene ring, or a phenanthrene ring are more preferable, and a benzene ring is most preferable.

### <Z¹>

In formula (1), Z¹ is a group represented by formula (1a) or (1b) below. The dashed line indicates a hand that binds to a carbon atom at position 7 in the skeleton represented by formula (1).

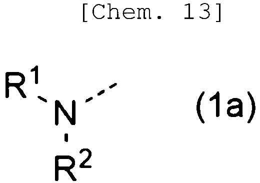

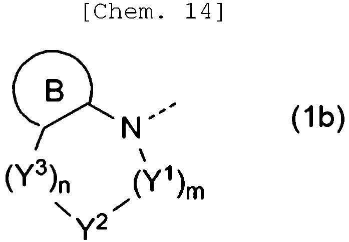

### (R¹)

R¹ is a substituted or unsubstituted aryl group, a substituted or unsubstituted heteroaryl ring, or a substituted or unsubstituted fused polycylic ring in which an aromatic ring or an aromatic heterocyclic ring is fused with these substituents. The substituent that may be included in a group that may be R¹ will be described later.

The number of carbon atoms in the substituted or unsubstituted aryl group is e.g. 5 or more and 12 or less, and preferably 6 or more and 10 or less. The heteroatom of the substituted or unsubstituted heteroaryl group includes at least one selected from a group consisting of oxygen atom, sulfur atom, nitrogen atom, and phosphorus atom. The number of heteroatoms in the substituted or unsubstituted heteroaryl group is e.g. 1 or more and 3 or less, preferably 1 or 2. The number of carbon atoms in the substituted or unsubstituted heteroaryl group is e.g. 4 or more and 11 or less, and preferably 5 or more and 9 or less.

R¹ is preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group, more preferably a substituted or unsubstituted phenyl group, a substituted or unsubstituted 1-naphthyl group, or a substituted or unsubstituted 2-naphthyl group.

The substituent that may be included in R¹ is preferably at least one selected from a group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of substituents is e.g. 1 or more and 3 or less.

### (R²)

R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below.

R² is preferably a hydrogen atom, a substituted or unsubstituted alkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below, more preferably a substituted or unsubstituted alkyl group, a substituted or unsubstituted aryl group, or a group represented by formula (2a) below.

The alkyl group is preferably an unsubstituted alkyl group having 1 or more and 10 or less carbon atoms, and more preferably an unsubstituted alkyl group having 1 or more and 6 or less carbon atoms. Specific examples of the alkyl group include a methyl group, an ethyl group, a propyl group, a butyl group, a pentyl group, and a hexyl group.

Preferably, the haloalkyl group has 1 to 6 carbon atoms. The number of halogen atoms is preferably 1 or more and 10 or less, and more preferably 2 or more and 5 or less. The haloalkyl group having 1 to 6 carbon atoms is preferably an alkyl group substituted with a fluorine atom, a chlorine atom, or a bromine atom. Preferably, the haloalkyl group has a perfluoromethyl group on its terminal. Examples of a suitable haloalkyl group include a trifluoromethyl group, a trifluoroethyl group, a trifluoropropyl group, a tetrafluoroethyl group, a chloromethyl group, a 2-chloroethyl group, a bromomethyl group, and the like.

The cycloalkyl group is preferably a cycloalkyl group having 3 to 8 carbon atoms (cycloalkyl group having 3 to 8 carbon atoms constituting a ring). Examples of the cycloalkyl group having 3 to 8 carbon atoms include a cyclopropyl group, a cyclobutyl group, a cyclopentyl group, a cyclohexyl group, and the like. Note that, although the cycloalkyl group may have substituents, the number of carbon atoms constituting the ring (3 to 8 carbon atoms) does not include the number of carbon atoms in the substituents.

The alkoxy group is preferably an alkoxy group having 1 to 6 carbon atoms. Examples of a suitable alkoxy group having 1 to 6 carbon atoms include a methoxy group, an ethoxy group, an n-propoxy group, an isopropoxy group, an n-butoxy group, a sec-butoxy group, a tert-butoxy group, and the like.

As the aryl group, the same groups as those listed for R¹ as examples may be used. The aryl group is preferably a phenyl group or a naphthyl group.

As the heteroaryl group, the same groups as those listed for R¹ as examples may be used.

The substituent that may be included in R² is preferably at least one selected from a group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of substituents is e.g. 1 or more and 3 or less.

R² is preferably an alkyl group having 1 or more and 5 or less carbon atoms, a fluoroalkyl group having 1 or more and 5 or less carbon atoms, an alkoxy group having 1 or more and 5 or less carbon atoms, an alkoxyalkyl group having 2 or more and 6 or less carbon atoms, a phenyl group, or a phenyl group having a substituent. Preferably, the fluoroalkyl group having 1 or more and 5 or less carbon atoms has a perfluoromethyl group on its terminal.

In terms of increasing the color optical density, R² is preferably a substituted or unsubstituted aryl group or a substituted or unsubstituted heteroaryl group. It is considered that such a structure further extends the conjugation between the skeleton and the substituent Z¹ presented in formula (1).

(Group Represented by Formula (2a))

-Q¹-(X¹Q²) a-X²Q³ (2a)

In formula (2a), Q¹ is an alkylene group or a haloalkylene group.

The number of carbon atoms in the alkylene group is preferably 1 or more and 20 or less, more preferably 1 or more and 12 or less, even more preferably 1 or more and 7 or less, and most preferably 2 or more and 6 or less. As the halogen atom in the haloalkylene group, at least one selected from a group consisting of I, Cl, Br, and F may be used. The halogen atom is preferably at least one of Cl and F, more preferably F. In the haloalkylene group, it is preferable that the halogen atom is bonded to the terminal carbon atom, and it is more preferable that the terminal group is a perfluoromethyl group.

Q² is an alkylene group or a haloalkylene group. The preferable aspect of the alkylene group or haloalkylene group is the same as that described for Q¹. The number of carbon atoms in the alkylene group or haloalkylene group of Q² may be the same as or different from that in the alkylene group or haloalkylene group of Q¹.

Q³ is an alkyl group or a haloalkyl group, which may be linear or branched, but is preferably linear.

The carbon number in the alkyl group is preferably 1 or more and 20 or less, more preferably 1 or more and 12 or less, most preferably 1 or more and 7 or less. As the halogen atom of the haloalkyl group, at least one selected from a group consisting of I, Cl, Br, and F may be used. The halogen atom is preferably at least one of Cl and F, more preferably F.

Q³ is preferably a linear alkyl group.

X¹ and X² are each independently O, S, NR⁷⁰⁰, PR⁷⁰¹, or P(=O). X¹ and X² are preferably O, S, or NR⁷⁰⁰, most preferably O.

R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.

R⁷⁰⁰ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

R⁷⁰¹ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

a is 0, or 1 or more and 3 or less.

Formula (2a) is preferably an alkylenealkoxy group, an alkylenethioalkyl group, or an alkyleneoxyalkylenealkoxy group.

Specific examples of formula (2a) include -CH₂OCH₃, - CH₂SCH₃, -CH₂CH₂OCH₃, -CH₂CH₂OCH₂CH₃, -CH₂CH₂SCH₃, -CH₂CH₂CH₂OCH₃, - CH₂CH₂CH₂SCH₃, -CH₂CH₂OCH₂CH₂OCH₃, and -CH₂CH₂OCH₂CH₂OCH₂CH₃.

The group represented by formula (2a) is preferably - CH₂OCH₃, -CH₂CH₂OCH₃, -CH₂CH₂OCH₂CH₃, -CH₂CH₂CH₂OCH₃, - CH₂CH₂OCH₂CH₂OCH₃, or -CH₂CH₂OCH₂CH₂OCH₂CH₃.

Examples of suitable formula (1a) are as follows.

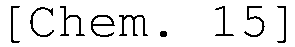

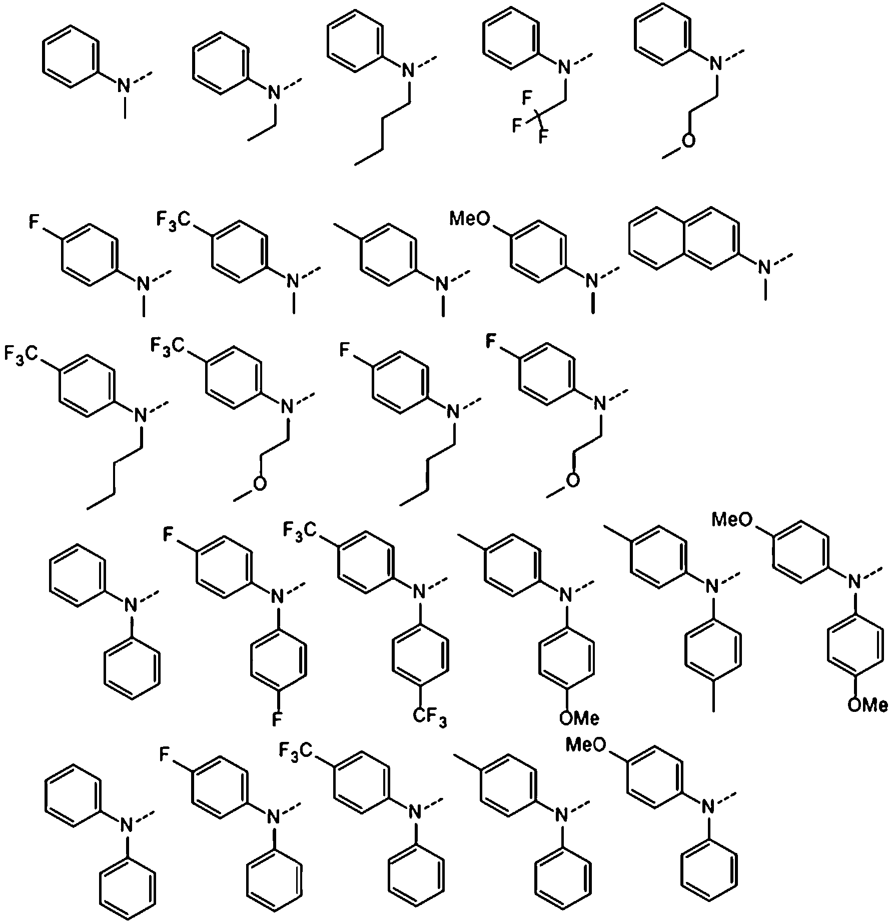

### <Formula (1b)>

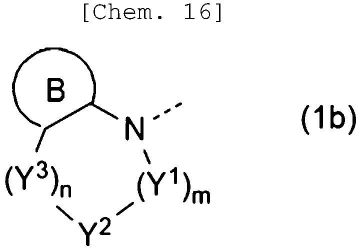

In formula (1b), ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Examples of a suitable ring B are the same as those listed for ring A.

Y¹ is a substituted or unsubstituted methylene group. The substituent is preferably a halogen atom, a haloalkyl group having 1 or more and 3 or less carbon atoms, or an alkyl group having 1 or more and 3 or less carbon atoms, and more preferably a fluorine atom or a methyl group. The number of the substituents is 1 or 2.

Y¹ is more preferably a methylene group substituted with a methyl group or a fluorine atom, or an unsubstituted methylene group, particularly preferably an unsubstituted methylene group.

Y² is a substituted or unsubstituted methylene group, O, S, SO₂, NR⁶⁰⁰, R⁶⁰¹C=CR⁶⁰², or CC.

R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group, preferably a hydrogen atom, a substituted or unsubstituted alkyl group, a substituted or unsubstituted cycloalkyl group, or a substituted or unsubstituted aryl group. As these groups, the same groups as those listed for R¹ or R² as examples may be used.

Preferably, the haloalkoxy group has 1 to 6 carbon atoms. The number of halogen atoms is preferably 1 or more and 10 or less, and more preferably 2 or more and 5 or less. The haloalkoxy group having 1 to 6 carbon atoms is preferably an alkoxy group substituted with a fluorine atom, a chlorine atom, or a bromine atom. Preferably, the haloalkoxy group has a perfluoromethyl group on its terminal. Examples of a suitable haloalkoxy group include a trifluoromethoxy group, a pentafluoroethoxy group, a heptafluoropropoxy group, a 2,2,2-trifluoroethoxy group, a 3,3,3-trifluoropropoxy group, a 2,2,3,3,3-pentafluoropropoxy group, and the like.

Y² is preferably a substituted or unsubstituted methylene group, O, S, SO₂, or NR⁶⁰⁰, more preferably an unsubstituted methylene group, O, S, or SO₂.

Y³ is a substituted or unsubstituted methylene group, and examples of suitable Y³ are the same as those listed for Y¹ as examples.

m is an integer of 1 to 4.
m is preferably 1 or 2.
n is an integer of 0 to 4.
n is preferably 0 or 1.
m+n is an integer of 2 or more.
m+n is preferably 2 or more and 4 or less, more preferably 2 or 3. When n is 0, Y² binds to ring B.

Examples of suitable formula (1b) are as follows.

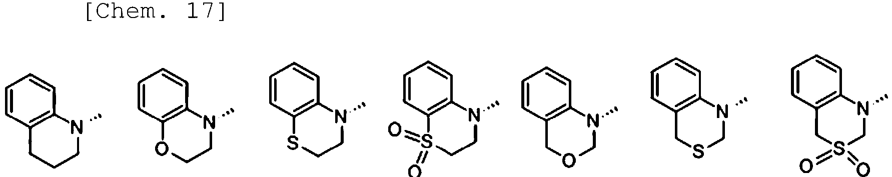

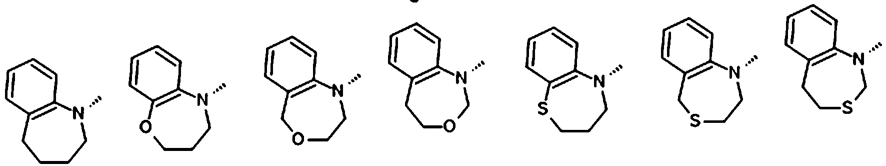

### <Compound Represented by Formula (2)>

Preferably, the photochromic compound according to the embodiment has a naphthopyran skeleton represented by formula (2) below. In formula (2) below, Z¹ and M are each the same as those in formula (1).

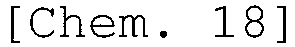

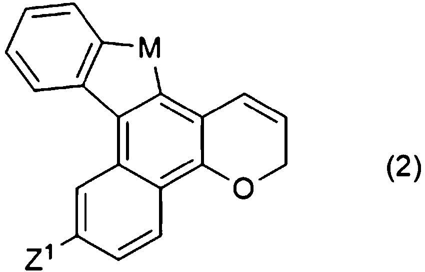

### <Skeleton Represented by Formula (3)>

Preferably, the photochromic compound according to the embodiment has a skeleton represented by formula (3) below. In formula (3) below, Z¹ and M are each the same as those in formula (1).

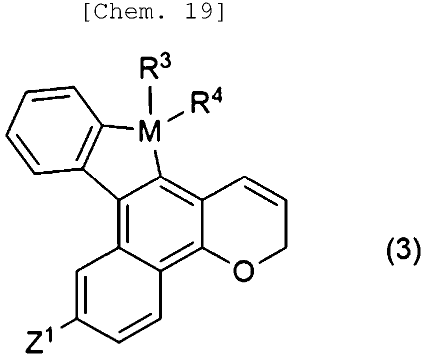

### <R³ and R⁴>

R³ and R⁴ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) above.

The same groups as those listed for R¹ or R² as examples may be used as the substituted or unsubstituted alkyl group, the haloalkyl group, the group represented by formula (2a), the substituted or unsubstituted cycloalkyl group, the substituted or unsubstituted alkoxy group, the substituted or unsubstituted aryl group, and the substituted or unsubstituted heteroaryl group.

The amino group may be a primary amino group (-NH₂), or a secondary or tertiary amino group with substituents that have been substituted for one or two hydrogen atoms. Examples of the substituents in the substituted amino group include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like. Examples of a suitable amino group include an amino group, a methylamino group, a dimethylamino group, an ethylamino group, a diethylamino group, a methylphenylamino group, a diphenylamino group, and the like.

Preferably, the heterocyclic group is constituted by 3 to 10 atoms. The heteroatom of the heterocyclic group is preferably at least one selected from a group consisting of oxygen atom, nitrogen atom, sulfur atom, and phosphorus atom. The number of heteroatoms is e.g. 1 or more and 5 or less, and preferably 1 or 2. Specific examples of the heterocyclic group include an aliphatic heterocyclic group such as a morpholino group, a piperidino group, a pyrrolidinyl group, a piperazino group, and an N-methylpiperazino group, an aromatic heterocyclic group such as an indolinyl group, and the like. The heterocyclic group may also be a 2,6-dimethylmorpholino group, a 2,6-dimethylpiperidino group, and a 2,2,6,6-tetramethylpiperidino group.

Preferably, the alkylthio group has 1 to 6 carbon atoms. Examples of the alkylthio group having 1 to 6 carbon atoms include a methylthio group, ethylthio group, an n-propylthio group, an isopropylthio group, an n-butylthio group, a sec-butylthio group, a t-butylthio group, and the like.

Preferably, the arylthio group has 6 to 10 carbon atoms. Examples of the arylthio group having 6 to 10 carbon atoms include a phenylthio group, a 1-naphthylthio group, a 2-naphthylthio group, and the like.

Preferably, the alkylcarbonyl group has 2 to 7 carbon atoms. Examples of the alkylcarbonyl group having 2 to 7 carbon atoms include an acetyl group and an ethylcarbonyl group.

Preferably, the alkoxycarbonyl group has 2 to 7 carbon atoms. Examples of the alkoxycarbonyl group having 2 to 7 carbon atoms include a methoxycarbonyl group and an ethoxycarbonyl group.

Examples of the halogen atom include a fluorine atom, a chlorine atom, a bromine atom, and an iodine atom.

Preferably, the aralkyl group has 7 to 11 carbon atoms. Examples of the aralkyl group having 7 to 11 carbon atoms include a benzyl group, a phenylethyl group, a phenylpropyl group, a phenylbutyl group, a naphthylmethyl group, and the like.

Preferably, the aralkoxy group has 7 to 11 carbon atoms. Examples of the aralkoxy group having 7 to 11 carbon atoms include a benzyloxy group a naphthylmethoxy group, and the like.

Preferably, the aryloxy group has 6 to 12 carbon atoms. Examples of the aryloxy group having 6 to 12 carbon atoms include a phenyloxy group a naphthyloxy group, and the like.

Preferably, the alkoxyalkylthio group has 2 to 9 carbon atoms. Examples of the alkoxyalkylthio group having 2 to 9 carbon atoms include a methoxymethylthio group, a methoxyethylthio group, a methoxy-n-propylthio group, a methoxy-n-butylthio group, an ethoxyethylthio group, an n-propoxypropylthio group, and the like.

Preferably, the haloalkylthio group has 1 to 6 carbon atoms. Examples of the haloalkylthio group having 1 to 6 carbon atoms include a trifluoromethylthio group, a tetrafluoroethylthio group, a chloromethylthio group, a 2-chloroethylthio group, a bromomethylthio group, and the like.

Preferably, the cycloalkylthio group has 3 to 8 carbon atoms. Examples of the cycloalkylthio group having 3 to 8 carbon atoms include a cyclopropylthio group, a cyclobutylthio group, a cyclopentylthio group, a cyclohexylthio group, and the like. Note that, although the cycloalkylthio group may have substituents, the number of carbon atoms (3 to 8 carbon atoms) does not include the number of carbon atoms in the substituents.

The silyl group may have substituents. Examples of the substituents of the substituted silyl group include, but are not particularly limited to, an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like.

The oxysilyl group may have substituents. Examples of the substituents of the substituted oxysilyl group include, but are not particularly limited to, an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, and the like.

Note that, the cycloalkyl group, the arylthio group, the aralkyl group, the aralkoxy group, the aryloxy group, the aryl group, the heteroaryl group, and the cycloalkylthio group may be unsubstituted. However, when these groups have substituents, it is preferable that 1 to 8 hydrogen atoms, particularly preferably 1 to 4 hydrogen atoms in groups constituting the ring are substituted with substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents will be described later.

R³ and R⁴ may be a hydrogen atom, a hydroxy group, a substituted or unsubstituted alkyl group, a haloalkyl group, a group represented by formula (2a), a substituted or unsubstituted cycloalkyl group, a substituted or unsubstituted alkoxy group, a formyl group, a hydroxycarbonyl group, a substituted or unsubstituted alkylcarbonyl group, a substituted or unsubstituted alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, or formula (X3) below.

Preferably, R³ and R⁴ are a substituted or unsubstituted alkyl group, a haloalkyl group, a group represented by formula (2a), or a group represented by formula (X3). When R³ and R⁴ are the aforementioned groups, the color fading speed of the photochromic compound tends to increase.

R³ and R⁴, together with M, may collectively constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring.

R³ and R⁴ preferably constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, more preferably constitute, together with M, a ring selected from a cyclopentane ring, a cyclohexane ring, a cycloheptane ring, a cyclooctane ring, a cyclononane ring, a cyclodecane ring, a cycloundecane ring, a cyclododecane ring, and a spirodicyclohexane ring. The ring may have 1 to 10 alkyl groups having 1 to 5 carbon atoms and cycloalkyl groups having 5 to 7 carbon atoms as substituents, and the cycloalkyl groups having 5 to 7 carbon atoms may be fused. Specifically, it is preferable that rings shown below are constituted.

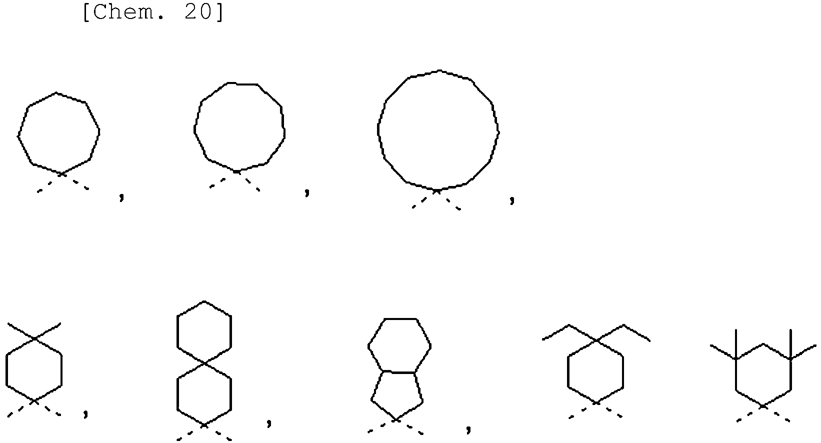

The substituents that may be included in groups R³ and R⁴ will be described later in detail. A substituent that may be included in this group is preferably a linear or branched alkyl group having 1 or more and 6 or less carbon atoms.

<Group Represented by Formula (X3)>

L¹-R⁴⁰⁰ (X3)

In formula (X3),
R⁴⁰⁰ is a hydrogen atom, an alkyl group, an aryl group, a polymerizable group, a photochromic group, or a silyl group having, as a substituent, an alkyl group, an alkoxy group, or an aryl group.

L¹ is a group represented by formula (X2) below.

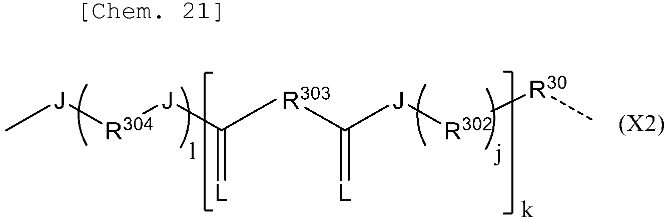

In formula (X2), R³⁰ is a group represented by formula (X2a) below.

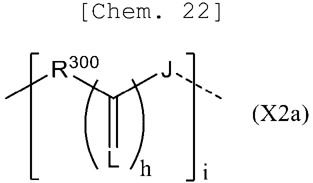

In formula (X2) and formula (X2a), J represents a divalent group. A plurality of J groups are each independently directly bonded, a substituted or unsubstituted methylene group, an oxygen atom, a sulfur atom, or NR³⁰¹. R³⁰¹ is a hydrogen atom or an alkyl group. R³⁰¹ is preferably an alkyl group having 1 to 20 carbon atoms. Preferably, this alkyl group has, as a substituent, a silyl group having an alkyl group with 1 to 10 carbon atoms, a polymerizable group, or a photochromic group.

Examples of the polymerizable group include radical polymerizable groups such as a vinyl group, a 1-chlorovinyl group, an allyl group, a styryl group, a (meth)acrylic group, a 2-(methacryloxy)ethylcarbamyl group, a 2-(methacryloxy)ethoxycarbonyl group, a crotyl group, and the like. Other examples thereof include an epoxy group, an episulfide group, a thietanyl group, OH group, SH group, NH₂ group, COOH group, NCO group, NCS group, and the like. The polymerizable group is preferably at least one selected from a group consisting of a (meth)acrylic group, a 2-(methacryloxy)ethylcarbamyl group, a 2-(methacryloxy)ethoxycarbonyl group, an epoxy group, OH group, SH group, NH₂ group, and COOH group.

The photochromic group is a group including a photochromic site. Representative examples of the photochromic group include naphthopyran, spirooxazine, spiropyran, fulgide, fulgimide, and diarylethene. As the photochromic group, indenonaphthopyran is preferable because it can express excellent photochromic properties, and above all, indeno[2,1-f]naphtho[1,2-b]pyran is particularly preferable.

Indeno[2,1-f]naphtho[1,2-b]pyran is preferably a group represented by formula (X₄) below.

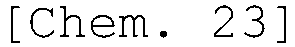

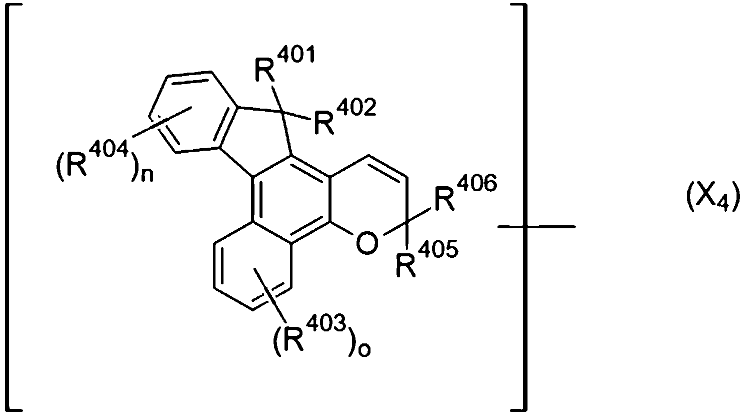

In formula (X₄), R⁴⁰¹ and R⁴⁰² may be the same groups as those used for R³ and R⁴ described above.

R⁴⁰³ and R⁴⁰⁴ may be each independently the same groups as those used for R³ and R⁴ described above.

R⁴⁰⁵ and R⁴⁰⁶ are each independently a substituted or unsubstituted aryl group, or a substituted or unsubstituted heteroaryl group. R⁴⁰⁵ and R⁴⁰⁶ may be the same as the groups used for R⁵ and R⁶ described later.

In formula (X₄), o is an integer of 0 to 4. n is an integer of 0 to 4. When o is 2 to 4, the plurality of R⁴⁰³ groups may be the same as or different from each other. When n is 2 to 4, the plurality of R⁴⁰⁴ groups may be the same as or different from each other.

At least one of the substituents on the aryl or heteroaryl group of R⁴⁰¹, R⁴⁰², R⁴⁰³, R⁴⁰⁴, and R⁴⁰⁵ binds to L¹.

A particularly suitable group represented by formula (X2) is represented by formulas below.

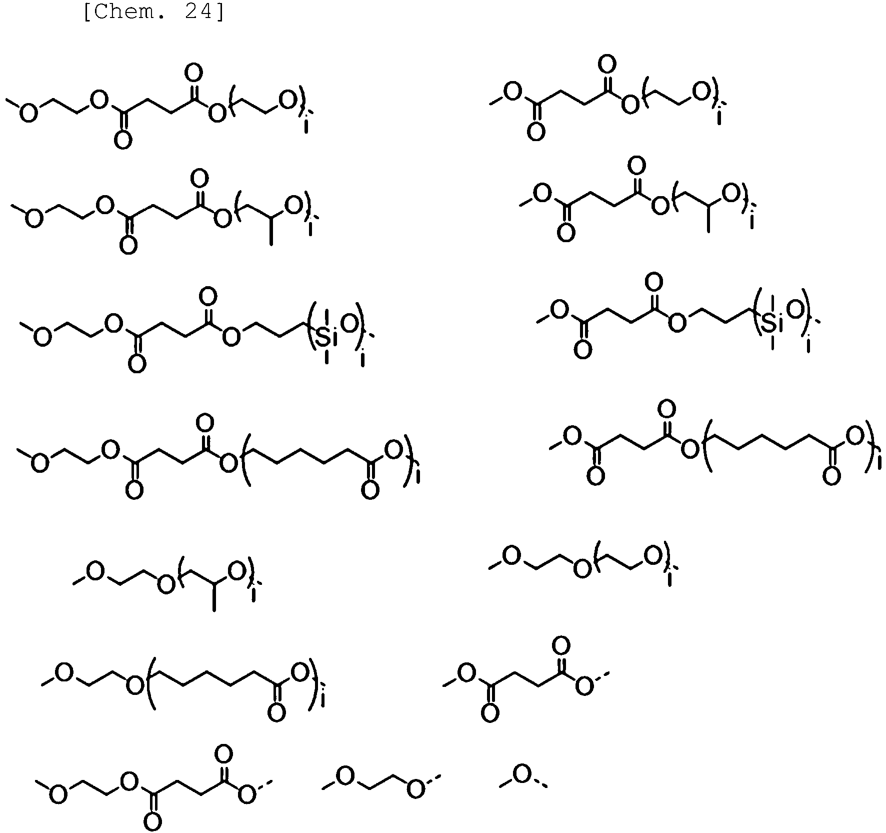

In formula (X2) and formula (X2a), L is an oxygen atom or a sulfur atom.

R³⁰⁰ is an alkylene group or a silylene group having an alkyl group or an aryl group as a substituent. R³⁰⁰ is preferably a silylene group having an alkylene group having 1 to 6 carbon atoms, or a silylene group with an alkyl group having 1 to 6 carbon atoms as a substituent.

R³⁰², R³⁰³, and R³⁰⁴ are each independently an alkylene group. R³⁰² is preferably an alkylene group having 1 to 6 carbon atoms. R³⁰³ is preferably an alkylene group having 1 to 6 carbon atoms. R³⁰⁴ is preferably an alkylene group having 1 to 6 carbon atoms.

h, j, k, and l are each independently an integer of 0 or 1.

In formula (X2a), i is an integer of 1 to 200. When i is 2 or more, the structures of the plurality of R³⁰ may be the same as or different from each other.
i is preferably a numerical number within a range of 5 to 100, more preferably 8 to 75, most preferably 10 to 70.

The dashed lines indicate bonds with R⁴⁰⁰,

### <Group Represented by Formula (X)>

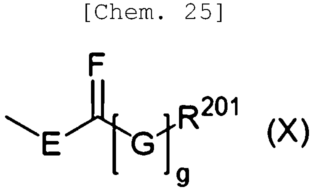

In formula (X), E is an oxygen atom or NR¹⁰¹. R¹⁰¹ is a hydrogen atom or an alkyl group. E is NR¹⁰¹, and R¹⁰¹ is preferably a hydrogen atom or an alkyl group having 1 to 6 carbon atoms.

F is an oxygen atom or a sulfur atom. F is preferably an oxygen atom.

G is an oxygen atom, a sulfur atom, or NR²⁰². R²⁰² is a hydrogen atom, an alkyl group, a cycloalkyl group, an aryl group, or a heteroaryl group. G is preferably NH.

g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than hydrogen atom. R²⁰¹ is preferably an alkyl group having 1 to 6 carbon atoms, an alkoxy group having 1 to 6 carbon atoms, or an aryl group having 6 to 12 carbon atoms.

Suitable groups represented by formula (X) are as follows.

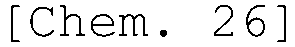

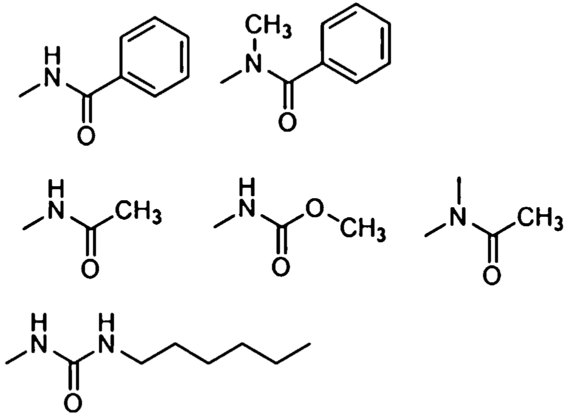

### (Skeleton Represented by Formula (7))

A particularly suitable skeleton of the photochromic compound according to the embodiment is represented by formula (7) below.

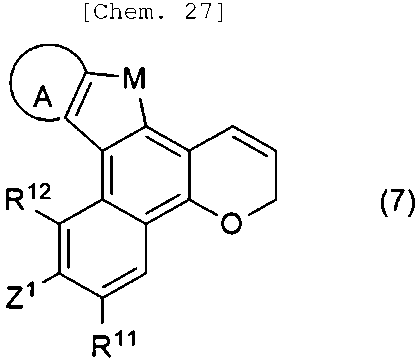

In formula (7), M, Z¹, and ring A are each the same as those in formula (1).

R¹¹ is a hydrogen atom, a halogen atom, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a substituted or unsubstituted alkylthio group, a substituted or unsubstituted arylthio group, a substituted or unsubstituted aralkyl group, a substituted or unsubstituted aralkoxy group, a substituted or unsubstituted aryloxy group, a substituted or unsubstituted aryl group, or a substituted or unsubstituted heteroaryl group. As these groups, the same groups as those listed for R¹ to R⁴ as examples may be used.

R¹² is a hydrogen atom, a halogen atom, a substituted or unsubstituted alkyl group, a haloalkyl group, a substituted or unsubstituted alkoxy group, a substituted or unsubstituted amino group, a substituted or unsubstituted alkylthio group, a substituted or unsubstituted arylthio group, a substituted or unsubstituted aralkyl group, a substituted or unsubstituted aralkoxy group, or a substituted or unsubstituted aryloxy group. As these groups, the same groups as those listed for R¹ to R⁴ as examples may be used.

The skeleton represented by formula (7) makes it possible to more effectively form a conjugation between the skeleton represented by formula (7) and the substituent Z¹ because a steric repulsion between the Z¹ group and substituents R¹¹ and R¹² at substitution positions adjacent to the Z¹ group is small.

R¹¹ and R¹² are each independently a hydrogen atom, a halogen atom, a substituted or unsubstituted alkyl group, a haloalkyl group, a substituted or unsubstituted alkoxy group, a substituted or unsubstituted amino group, a substituted or unsubstituted heterocyclic group, a substituted or unsubstituted arylthio group, or a substituted or unsubstituted aryloxy group, more preferably a hydrogen atom, a halogen atom, a substituted or unsubstituted alkyl group, a haloalkyl group, a substituted or unsubstituted alkoxy group, a substituted or unsubstituted amino group, or a substituted or unsubstituted heterocyclic group, particularly preferably a hydrogen atom, a halogen atom, a substituted or unsubstituted alkyl group, a haloalkyl group, or a substituted or unsubstituted alkoxy group.

The substituents that may be included in groups R¹¹ and R¹² will be described later in detail. Groups R¹¹ and R¹² are preferably unsubstituted.

### <Compound Represented by Formula (4)>

The photochromic compound according to the embodiment is preferably a compound represented by formula (4) below.

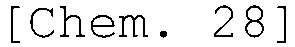

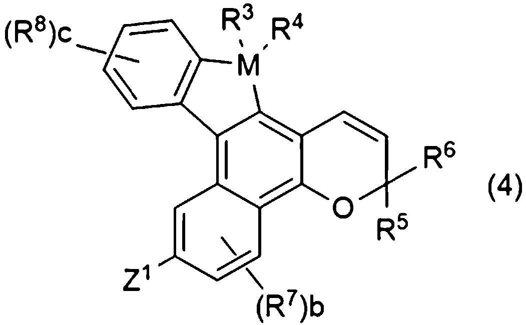

In formula (4), R³, R⁴, Z¹, and M are each the same as those in formula (3).

### <R⁵ and R⁶>

In formula (4), R⁵ and R⁶ are each independently a substituted or unsubstituted aryl group, or a substituted or unsubstituted heteroaryl group. As these groups, the same groups as those listed for R¹ as examples may be used.

Preferably, R⁵ and R⁶ are each independently a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group. Furthermore, it is preferable that at least one of R⁵ and R⁶ is a substituted or unsubstituted phenyl group, and it is more preferable that both R⁵ and R⁶ are substituted phenyl groups.

Preferably, the substituent of the phenyl group is a group represented by formula (2a), a hydroxy group, an alkyl group, a haloalkyl group, a substituted or unsubstituted cycloalkyl group, an alkoxy group, an amino group, a substituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, or a group represented by formula (X3). More preferably, the substituent is an alkyl group, a haloalkyl group, an alkoxy group, an amino group, a substituted amino group, a substituted or unsubstituted heterocyclic group, a cyano group, a halogen atom, an alkylthio group, a substituted or unsubstituted arylthio group, a substituted or unsubstituted aryloxy group, a substituted or unsubstituted aryl group, a substituted or unsubstituted heteroaryl group, an alkoxyalkylthio group, a haloalkylthio group, a substituted or unsubstituted silyl group, a substituted or unsubstituted oxysilyl group, or a group represented by formula (X3).

### <R⁷ and R⁸>

R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by formula (2a), a group represented by formula (X) below, or a group represented by formula (X3). As these groups, the same groups as those listed for R¹ to R⁴ as examples may be used.

In formula (4), b is an integer of 0 to 3. c is an integer of 0 to 4. When b is 2 to 3, a plurality of R⁷ groups may be the same as or different from each other. When b is 2 to 3 and there are a plurality of R⁷ groups adjacent to each other, two adjacent R⁷ groups, together with carbon atoms to which the R⁷ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. The combined adjacent R⁷ groups are located at the position 5 and the position 6 of the chromene compound.

When c is 2 to 4, a plurality of R⁸ groups may be the same as or different from each other. When c is 2 to 4 and there are a plurality of R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. The combined adjacent R⁸ groups are located at the position 9 and the position 10, the position 10 and the position 11, or the position 11 and the position 12 of the chromene compound.

R⁷ or R⁸ may constitute a ring constituted by 5 to 8 atoms including carbon atoms to which R⁷ or R⁸ bind. The ring may also have a substituent. The substituent includes a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, and a halogen atom. Specific examples of these substituents will be described later.

A suitable ring is exemplified by a ring represented by formula (X5) below.

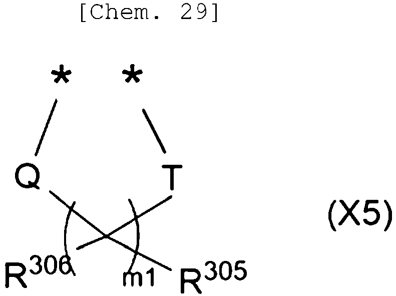

In formula (X5), Q and T are each independently a sulfur atom, a substituted or unsubstituted methylene group, an oxygen atom, or a group represented by NR³⁰⁷. R³⁰⁷ is a hydrogen atom, a hydroxy group, an alkyl group, a haloalkyl group, a cycloalkyl group, an alkoxy group, a substituted or unsubstituted aryl group, a substituted or unsubstituted heteroaryl group, or a group represented by formula (2a).

Preferably, R³⁰⁵ and R³⁰⁶ are each independently a hydroxy group, an alkyl group, a haloalkyl group, a cycloalkyl group, an alkoxy group, an amino group, a substituted amino group, a substituted or unsubstituted heterocyclic group, a cyano group, a nitro group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryl group, a thiol group, an alkylthio group, an alkoxyalkylthio group, a haloalkylthio group, a cycloalkylthio group, or a substituted or unsubstituted arylthio group.

R³⁰⁵ and R³⁰⁶ may constitute a substituted or unsubstituted aliphatic ring together with carbon atoms to which R³⁰⁵ and R³⁰⁶ bind. Specific examples of the aliphatic ring include a cyclopentane ring, a cyclohexane ring, and the like. In the aliphatic ring, 1 to 8 hydrogen atoms, particularly preferably 1 to 4 hydrogen atoms may be substituted with at least one group selected from a group consisting of a hydroxy group, an alkyl group, a haloalkyl group, a cycloalkyl group, an alkoxy group, an amino group, a substituted amino group, a heterocyclic group, a cyano group, a nitro group, and a halogen atom. Specific examples of these substituents will be described later.

In the formula, m1 is an integer of 1 to 4.

### <Photochromic Compound Represented by Formula (5)>

A particularly suitable chromene compound is exemplified by a compound represented by formula (5) below.

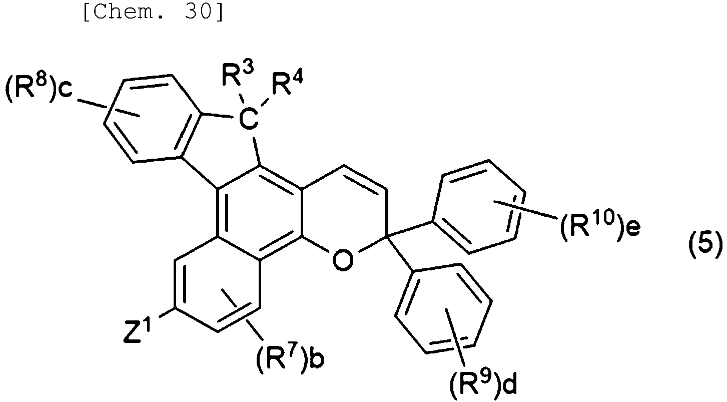

In formula (5),
R³, R⁴, R⁷, R⁸, Z¹, b, and c are each independently the same as those in formula (4).

### <R⁹ and R¹⁰>

R⁹ and R¹⁰ are each independently a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a), or a group represented by formula (X3).

d represents the number of R⁹ and is an integer of 0 to 5. When d is 2 or more, R⁹ groups may be the same as or different from each other.

When d is 2 to 5 and there are a plurality of R⁹ groups adjacent to each other, two adjacent R⁹ groups, together with carbon atoms to which the R⁹ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

e represents the number of R¹⁰ and is an integer of 0 to 5. When e is 2 or more, R¹⁰ groups may be the same as or different from each other.

When e is 2 to 5 and there are a plurality of R¹⁰ groups adjacent to each other, two adjacent R¹⁰ groups, together with carbon atoms to which the R¹⁰ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

Two adjacent R⁹ groups and two adjacent R¹⁰ groups can each independently constitute a ring group that may include at least one selected from a group consisting of oxygen atom, sulfur atom, carbon atom, and nitrogen atom. This ring group is preferably, but not particularly limited to, a ring constituted by 5 to 8 atoms including carbon atoms to which R⁹ and R¹⁰ groups bind. The ring may also have a substituent. Examples of the substituent include substituents selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group constituted by 3 to 8 atoms, a cyano group, a nitro group, and a halogen atom. Specific examples of these substituents are the same as those described above. As for the ring, it is preferable that R⁹ groups and R¹⁰ groups collectively constitute a ring represented by formula (X5).

### <Detailed Description of Substituents etc.>

Examples of the substituents that may be included in the groups R¹ to R¹⁰ described above include a hydroxy group, a cyano group, a halogen atom, a nitro group, a formyl group, a hydroxycarbonyl group, a thiol group, a group represented by formula (2a), a group represented by formula (X), a group represented by formula (X3), an alkyl group, a haloalkyl group, a cycloalkyl group, an alkoxy group, an amino group, a substituted amino group, a heterocyclic group, a halogen atom, an alkylthio group, an arylthio group, an alkylcarbonyl group, an alkoxycarbonyl group, an aralkyl group, an aralkoxy group, an aryloxy group, an aryl group, a heteroaryl group, an alkoxyalkylthio group, a haloalkylthio group, a cycloalkylthio group, a silyl group, and an oxysilyl group. As these groups, the same groups as those described in detail for R¹ to R⁴ may be used.

### <Specific Examples of Suitable Photochromic Compound>

A particularly suitable photochromic compound is specifically exemplified by photochromic compounds represented by the following formulas.

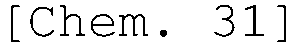

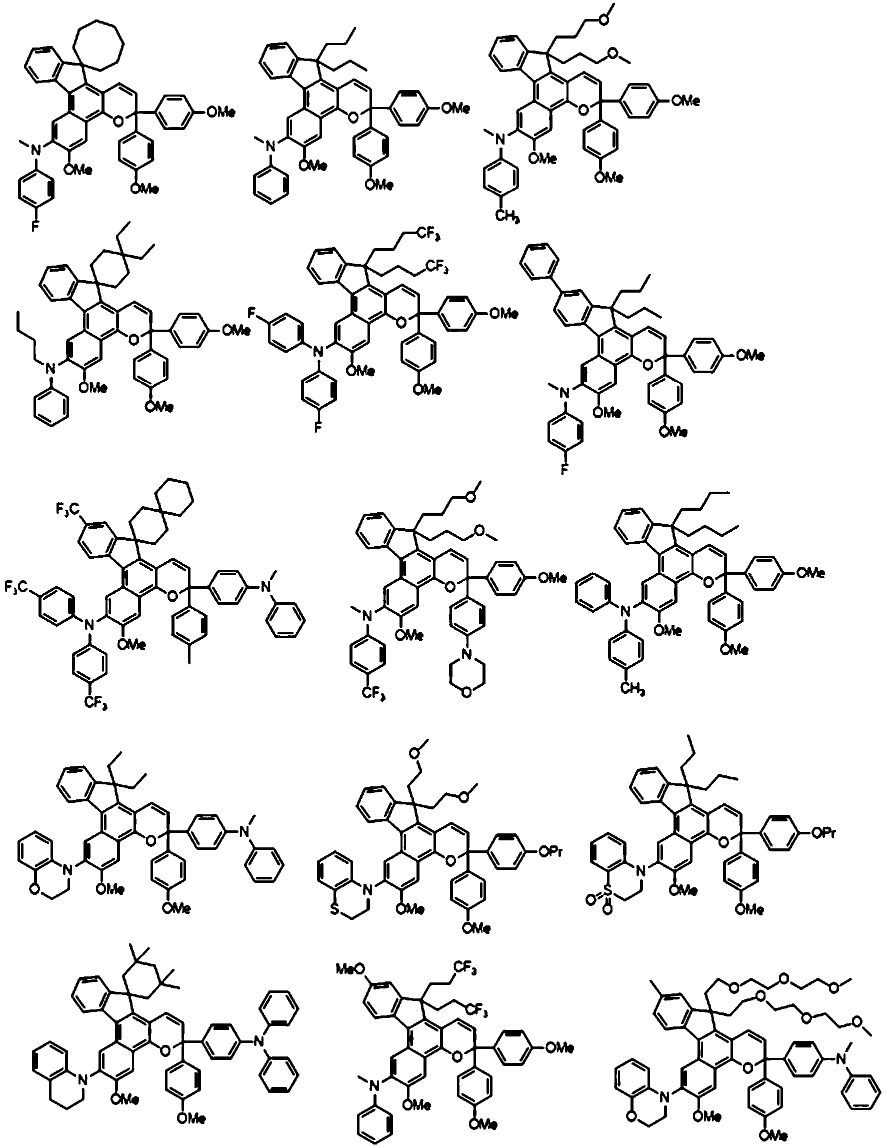

### [Naphthol Derivative]

The naphthol derivative according to the embodiment has a skeleton represented by formula (6) below. This naphthol derivative may be used as an intermediate for synthesizing the photochromic compound having the skeleton represented by formula (1) or formula (2) above.

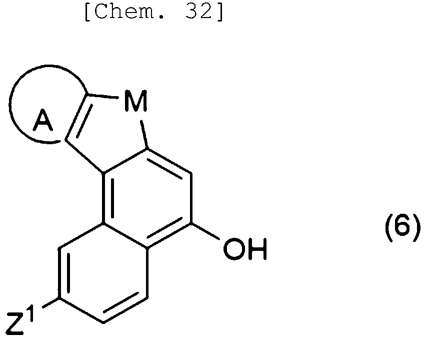

In formula (6), M, Z¹, and ring A are each independently the same as those in formula (1) above.

### <Naphthol Derivative Represented by Formula (6a)>

The naphthol derivative represented by formula (6a) may be used as an intermediate for synthesizing the photochromic compound represented by formula (3), formula (4), or formula (5) above.

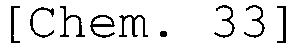

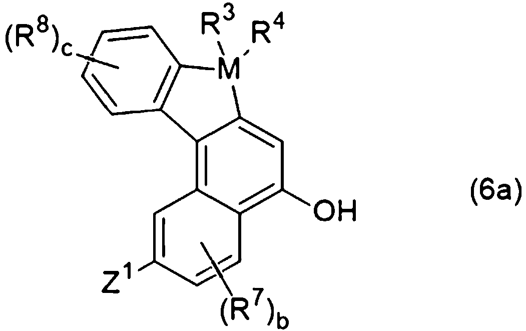

In formula (6a), M and Z¹ are each independently the same as those in formula (1) above. R³ and R⁴ are each independently the same as those in formula (3) above. R³, R⁴, c, and d are each independently the same as those in formula (4) above.

### <Specific Examples of Naphthol Derivative>

Specific examples of the naphthol derivative according to the embodiment include compounds represented by the following formulas.

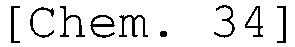

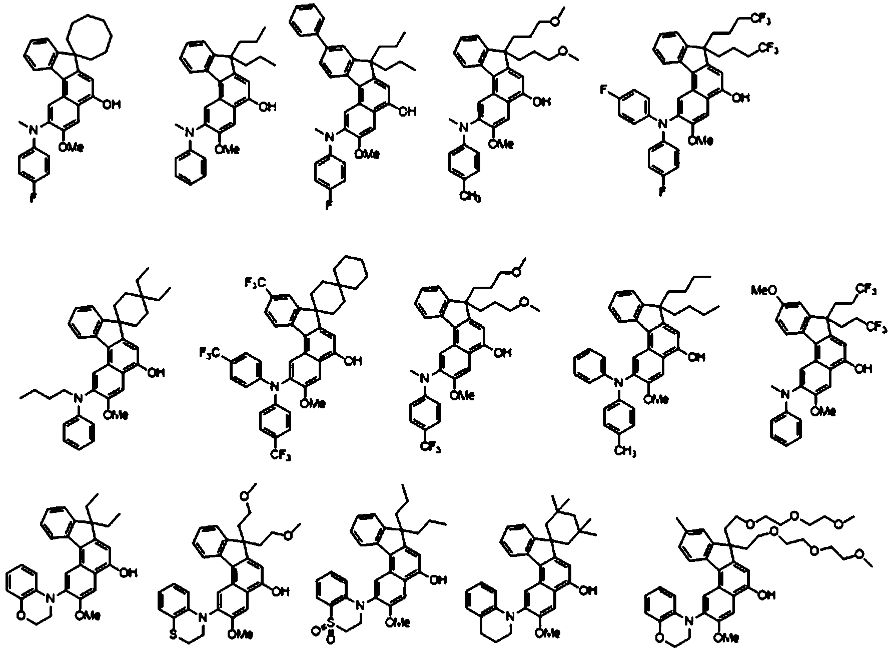

### <Compound Having Skeleton Represented by Formula (1A)>

According to the embodiment, a photochromic compound having a skeleton represented by formula (1A) below is provided.

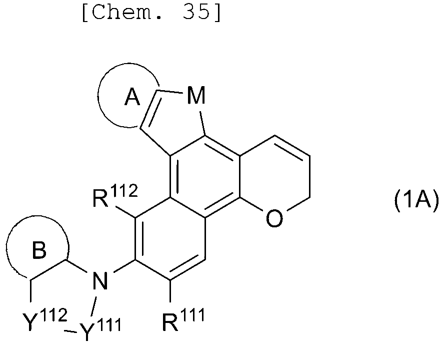

In formula (1A), M, ring A, and ring B are each independently the same as those in formula (1).

### <Y¹¹¹>

Y¹¹¹ is a substituted or unsubstituted methylene group. Y¹¹¹ is preferably an unsubstituted methylene group.

### <Y¹¹²>

Y¹¹² is a substituted or unsubstituted methylene group, an oxygen atom, a sulfur atom, NR⁶⁰⁰, or SO₂. R⁶⁰⁰ is the same as described above for formula (1b). Y¹¹² is preferably an unsubstituted methylene group or a methylene group having an alkyl group as a substituent.

### <R¹¹¹ and R¹¹²>

R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) above. As these groups, the same groups as those listed for R¹ to R⁸ as examples may be used. By using these groups as R¹¹¹ and R¹¹², the temperature dependency, the color fading speed, and the durability of the photochromic compound tend to increase. The substituents that may be included in the groups R¹¹¹ and R¹¹² will be described later in detail. Preferably, the substituent that may be included in the groups R¹¹¹ and R¹¹² is at least one selected from a group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of substituents is e.g. 1 or more and 3 or less. Preferably, R¹¹¹ and R¹¹² are each independently a hydrogen atom, a halogen atom, a substituted or unsubstituted alkyl group, a substituted or unsubstituted haloalkyl group, a substituted or unsubstituted alkoxy group, a substituted or unsubstituted amino group, a substituted or unsubstituted alkylthio group, a hydroxy group, a substituted or unsubstituted haloalkoxy group, or a group represented by formula (2a) below. R¹¹¹ is more preferably a substituted or unsubstituted alkyl group or a substituted or unsubstituted alkoxy group, even more preferably a methyl group or a methoxy group. R¹² is more preferably a hydrogen atom.

### <Compound Having Skeleton Represented by Formula (2A)>

Preferably, the photochromic compound according to the embodiment has a naphthopyran skeleton represented by formula (2A) below. In formula (2A) below, ring B, M, R¹¹¹, R¹¹², Y¹¹¹, and Y¹¹² are each the same as those in formula (1A) above.

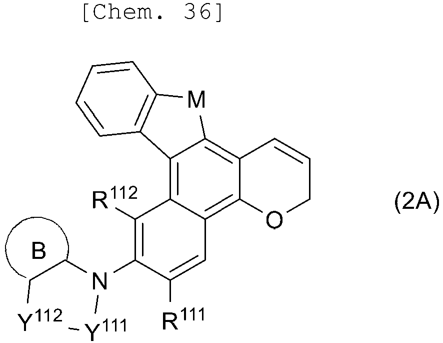

### <Compound Having Skeleton Represented by Formula (3A)>

Preferably, the photochromic compound according to the embodiment has a skeleton represented by formula (3A) below. In formula (3A) below, Y¹¹¹, Y¹¹², R¹¹¹, R¹¹², and M are each the same as those in formula (1A). R³ and R⁴ are each independently the same as those in formula (3).

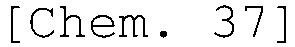

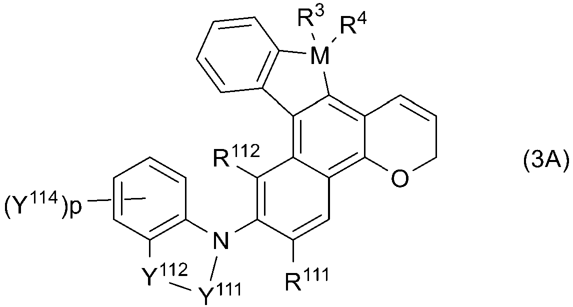

### <Y¹¹⁴>

Y⁴ is a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a substituted or unsubstituted haloalkylthio group, a substituted or unsubstituted cycloalkylthio group, a substituted or unsubstituted silyl group, a substituted or unsubstituted oxysilyl group, or a group represented by formula (2a). A substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a substituted or unsubstituted heterocyclic group, a substituted or unsubstituted arylthio group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) below may be the same as those listed for R¹¹¹ or R¹¹² as examples.

Y¹¹⁴ is preferably a substituted or unsubstituted alkyl group, a substituted or unsubstituted alkoxy group, a haloalkyl group, or a halogen atom, more preferably a linear alkyl group having 1 or more and 3 or less carbon atoms, a linear alkoxy group having 1 or more and 3 or less carbon atoms, a perfluoroalkyl group having 1 or more and 3 or less carbon atoms, or a fluorine atom.

p is an integer of 0 to 4.

p may be 0 or may be 1 to 3.

### <Compound Represented by Formula (4A)>

The photochromic compound according to the embodiment is preferably represented by formula (4A) below. In formula (4A), Y¹¹¹, Y¹¹², Y¹¹⁴, R³, R⁴, R¹¹¹, R¹¹², M, and p are each the same as those in formula (3A). R⁵, R⁶, R⁸, and c are the same as those in formula (4).

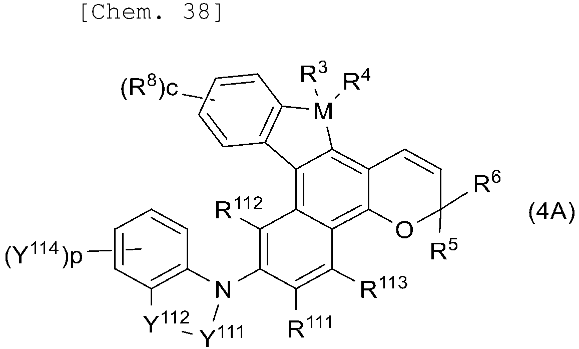

R¹¹³ is a hydrogen atom, a hydroxy group, a substituted or unsubstituted alkyl group, a substituted or unsubstituted haloalkyl group, a substituted or unsubstituted haloalkoxy group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a substituted or unsubstituted haloalkylthio group, a substituted or unsubstituted cycloalkylthio group, a substituted or unsubstituted silyl group, a substituted or unsubstituted oxysilyl group, a group represented by formula (2a) above, a group represented by formula (X) above, or a group represented by formula (X3) above. R¹¹³ is preferably a hydrogen atom.

R¹¹¹ and R¹¹³, together with carbon atoms to which R¹¹¹ and R¹¹³ bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

### <Photochromic Compound Represented by Formula (5A)>

A particularly suitable chromene compound is exemplified by a compound represented by formula (5A) below. In formula (5A), Y¹¹¹, Y¹¹², Y¹¹⁴, R³, R⁴, R⁸, R¹¹¹, R¹¹², R¹¹³, c, and p are each independently the same as those in formula (4A). R⁹, R¹⁰, d, and e are each independently the same as those in formula (5).

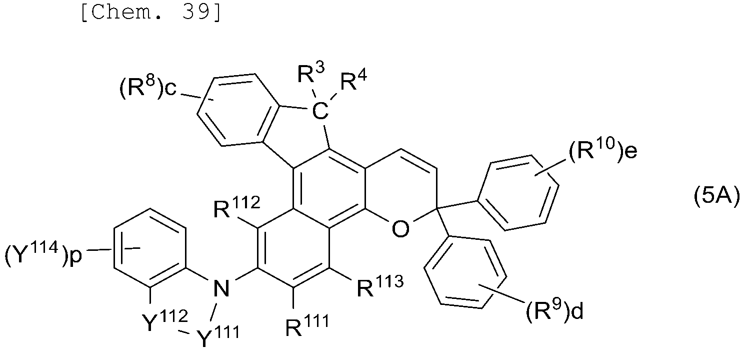

### <Detailed Description of Substituents etc.>

Examples of the substituents that may be included in the groups R¹¹¹ to R¹¹³ and Y¹¹¹ to Y¹¹⁴ described above include a hydroxy group, a cyano group, a halogen atom, a nitro group, a formyl group, a hydroxycarbonyl group, a thiol group, a group represented by formula (2a), a group represented by formula (X), a group represented by formula (X3), an alkyl group, a haloalkyl group, a cycloalkyl group, an alkoxy group, an amino group, a substituted amino group, a heterocyclic group, a halogen atom, an alkylthio group, an arylthio group, an alkylcarbonyl group, an alkoxycarbonyl group, an aralkyl group, an aralkoxy group, an aryloxy group, an aryl group, a heteroaryl group, an alkoxyalkylthio group, a haloalkylthio group, a cycloalkylthio group, a silyl group, and an oxysilyl group. As these groups, the same groups as those described in detail for R³ to R¹² may be used.

### <Specific Examples of Suitable Photochromic Compound>

A particularly suitable photochromic compound is specifically exemplified by photochromic compounds represented by the following formulas.

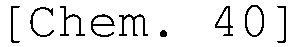

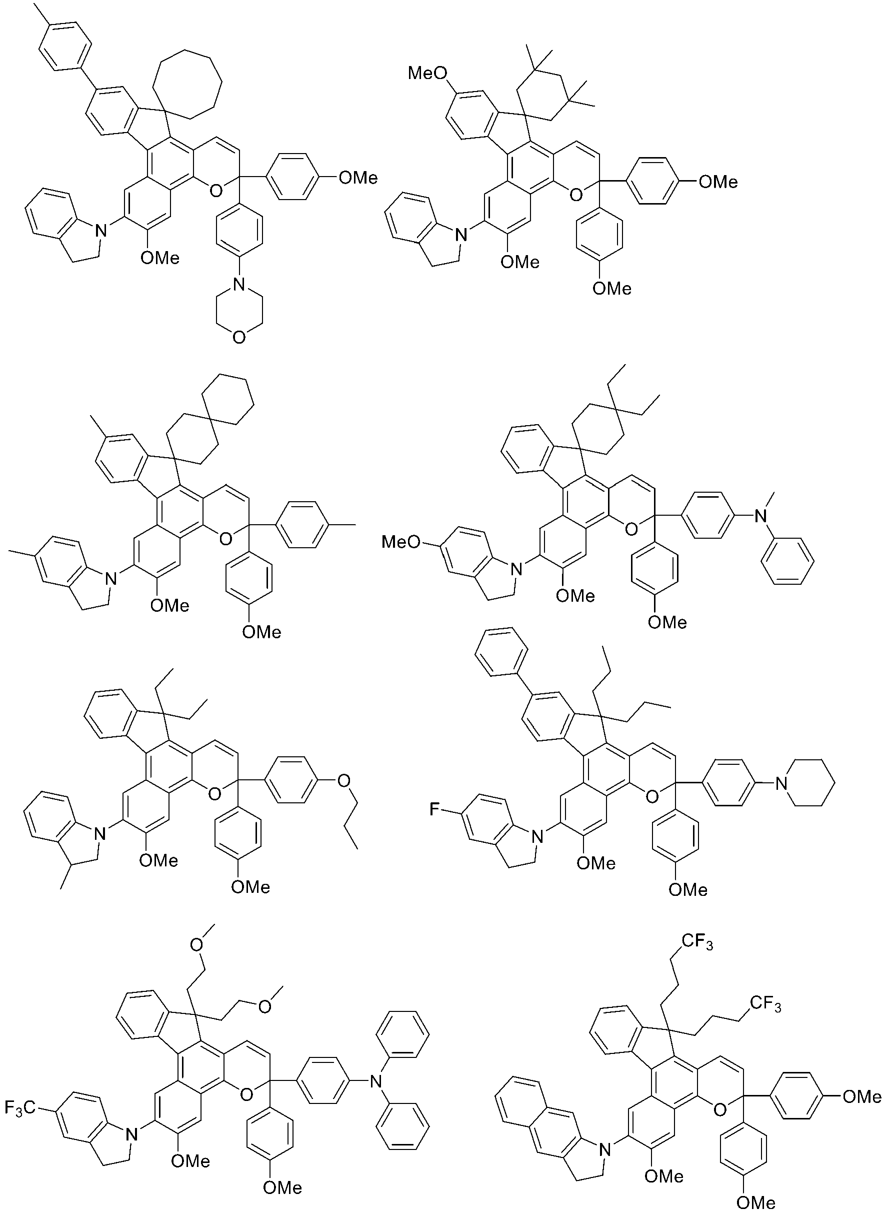

### [Naphthol Derivative]

The naphthol derivative according to the embodiment has a skeleton represented by formula (6A) below. This naphthol derivative may be used as an intermediate for synthesizing the photochromic compound having the skeletons represented by formula (1A) or formula (2A) above. In formula (6A), ring A, ring B, Y¹¹¹, Y¹¹², R¹¹¹, R¹¹², and M are each independently the same as those in formula (1A) above.

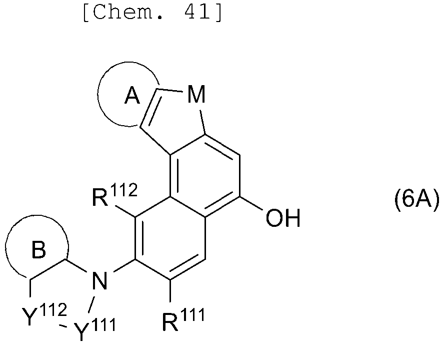

### <Naphthol Derivative Represented by Formula (6Aa)>

The naphthol derivative represented by formula (6Aa) may be used as an intermediate for synthesizing the photochromic compound represented by formula (3A), formula (4A), or formula (5A) above. In formula (6Aa), Y¹¹¹, Y¹¹², Y¹¹⁴, R³, R⁴, R¹¹¹, R¹¹², R¹¹³, c, and p are each independently the same as those in formula (3A), formula (4A), or formula (5A) above.

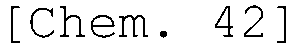

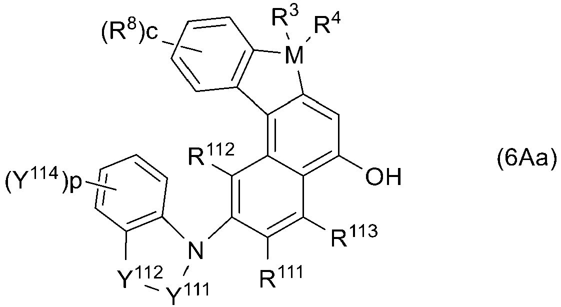

### <Specific Examples of Naphthol Derivative>

Specific examples of the naphthol derivative according to the embodiment include compounds represented by the following formulas.

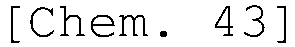

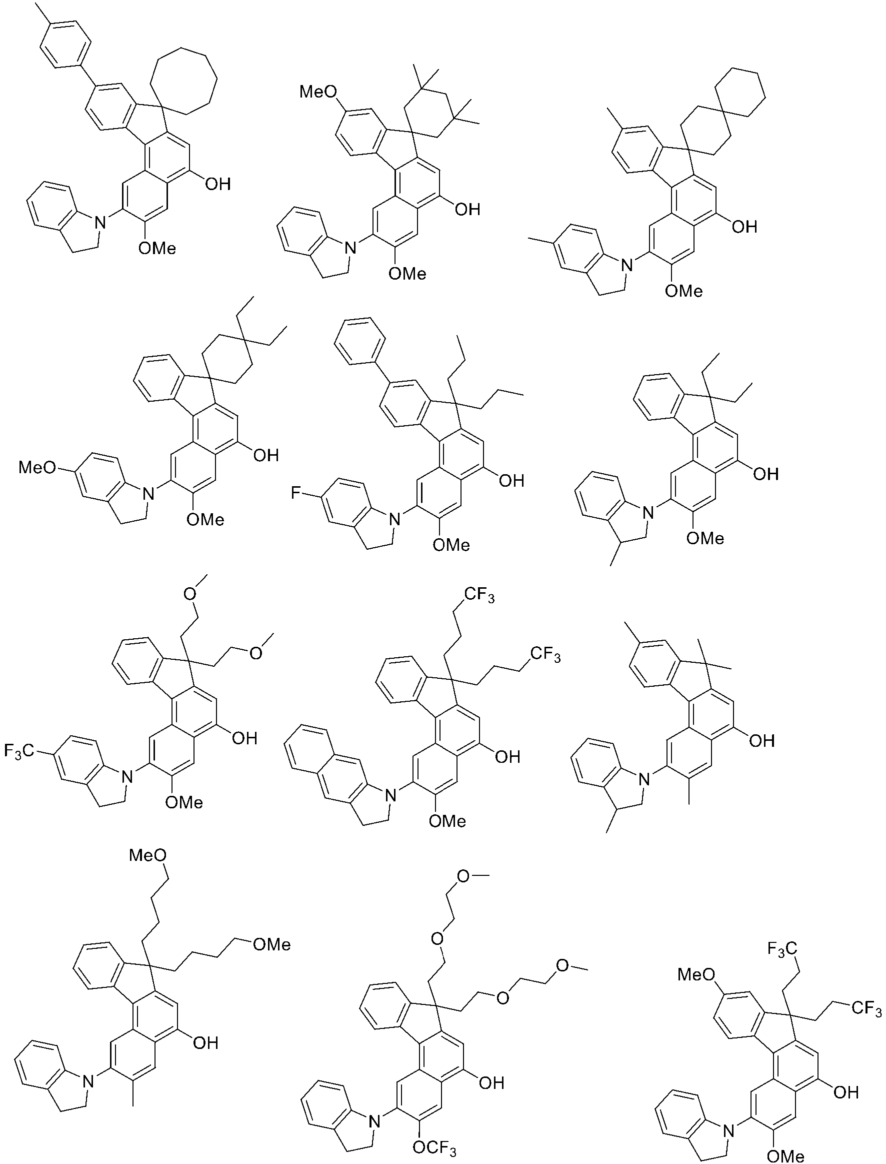

### [Method for Producing Photochromic Compound]

The photochromic compound according to the embodiment may be produced by any synthetic method. A representative example of the method for producing the photochromic compound will be explained, but the present invention is not limited to this method. Note that, in the following description, unless otherwise specified, the symbols in each formula have the same meanings as explained for the formulas described above.

The photochromic compound can be produced by a method of reacting a naphthol derivative represented by formula (6a) or formula (6Aa) above with a propargyl alcohol compound represented by formula (7) below in the presence of an acid catalyst.

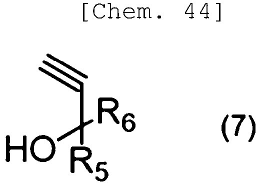

A reaction ratio of the naphthol compound and the propargyl alcohol compound is preferably selected from a range of 1:10 to 10:1 (molar ratio). As an acid catalyst, for example, sulfuric acid, benzenesulfonic acid, p-toluenesulfonic acid, acidic alumina and the like are used. The acid catalyst is used preferably in a range of 0.1 to 10 parts by weight per 100 parts by weight of the sum of the naphthol compound and the propargyl alcohol compound. The reaction temperature is preferably 0 to 200°C. As the solvent, it is preferable to use an aprotic organic solvent such as N-methylpyrrolidone, dimethylformamide, tetrahydrofuran, benzene, and toluene. A method for purifying a product obtained by this reaction is not particularly limited. For example, the product can be purified by a silica gel column purification followed by recrystallization.

### <Method for Synthesizing Naphthol Derivative>

The naphthol derivative can be synthesized e.g. based on reaction methods described in Non-Patent Document 1, Non-Patent Document 2, Patent Document 3, and other articles.

The method for synthesizing the naphthol compound represented by formula (6a) is not particularly limited, but, for example, when M is a carbon atom, the naphthol compound can be synthesized as follows.

First, a benzene compound represented by formula (8) below is reacted with an acid chloride compound represented by formula (9) below to obtain a benzophenone compound represented by formula (10) below. Note that, in formula (10), Z¹, R⁷, R⁸, b, and c are the same as those in formula (4).

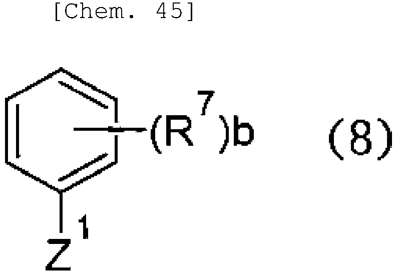

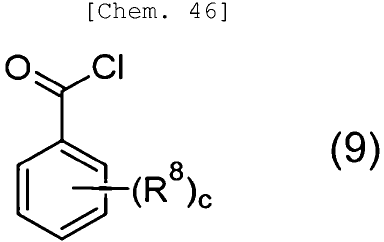

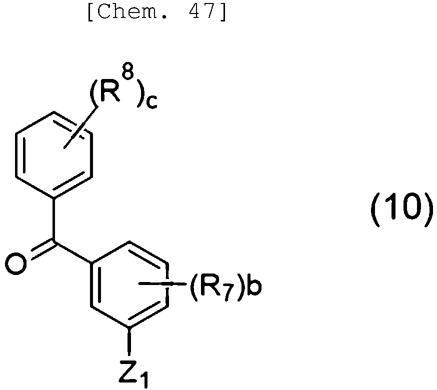

Note that, depending on the structure and substituents of the photochromic compound to be synthesized, the substituent of the acid chloride compound and the substituent of the benzene compound may be interchanged. That means, the acid chloride compound having Z¹ and R⁷ may be reacted with the benzene compound having R⁸.

Furthermore, the benzophenone compound (10) is subjected to a Stobbe reaction, a cyclization reaction, a hydrolysis reaction using an alkali or acid, benzyl protection, or a debenzylization by a hydrolysis reaction using an alkali or acid to obtain a carboxylic acid with a hydroxy group protected by benzyl (Bn), represented by formula (11) below. Subsequently, the benzyl-protected carboxylic acid represented by formula (11) is converted into an amine by Curtius rearrangement, Hofmann rearrangement, Lossen rearrangement, or the like to prepare a diazonium salt from this amine. This diazonium salt is converted into a halide such as a bromide or an iodide by a Sandmeyer reaction or the like to obtain a halide represented by formula (12) below (in the formula, Hal represents a halogen).

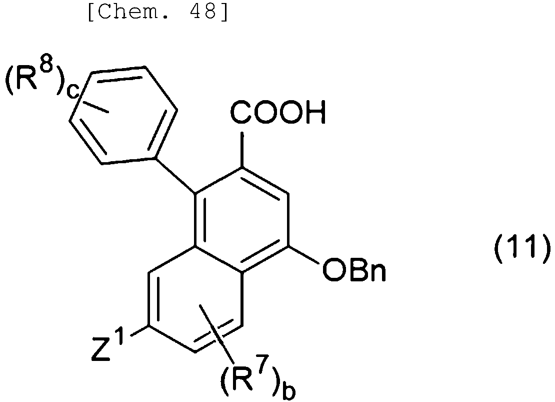

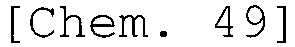

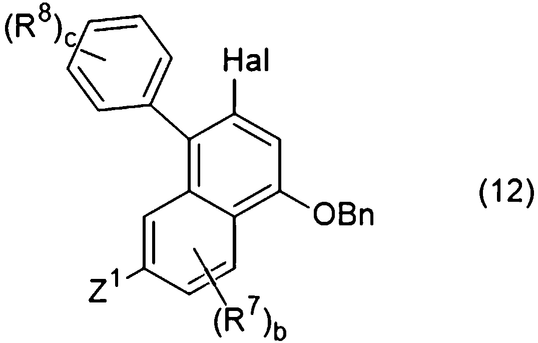

The resulting halide is reacted with magnesium, lithium, or the like to prepare an organometallic reagent. This organometallic reagent is reacted with a ketone represented by formula (13) below (in the formula, R³ and R⁴ are the same as those in formula (4)) in an organic solvent at -100 to 70°C to obtain a compound represented by formula (14) below. The resulting compound (14) is debenzylized and then reacted under a neutral to acidic conditions at 10 to 120°C for 10 minutes to 2 hours to spiroannulate the alcohol, so that a desired naphthol derivative represented by formula (6a) can be synthesized.

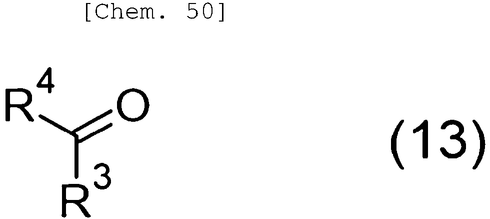

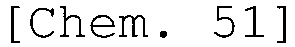

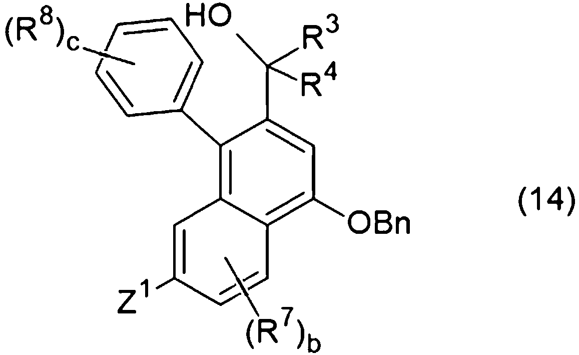

In this reaction, a reaction ratio between the organometallic reagent and the ketone represented by a formula (13) can be adopted from a wide range, but is preferably selected from a range of 1:10 to 10:1 (molar ratio). The reaction temperature is preferably -100 to 70°C. As the solvent, an aprotic organic solvent such as diethyl ether, tetrahydrofuran, benzene, and toluene is preferably used. The spiroannulation of the alcohol under a neutral to acidic condition is preferably performed in the presence of an acid catalyst. As the acid catalyst, for example, acetic acid, hydrochloric acid, sulfuric acid, benzenesulfonic acid, p-toluenesulfonic acid, and acidic alumina are used. Suitably, such an acid catalyst is used in a range of 0.1 to 10 parts by weight per 100 parts by weight of the alcohol. Preferably, the spiroannulation is performed in the presence of a solvent such as tetrahydrofuran, benzene, and toluene.

Although the above method is exemplified by a method using a raw material having the substituent Z¹, it is possible to adopt e.g. a method in which a precursor having a halogen atom is synthesized, to which the substituent Z¹ is introduced by Buchwald reaction using an amine having a Z¹-H structure and a Pd catalyst, or the like.

The reaction for introducing the substituent Z¹ is not particularly limited, and, for example, the substituent Z¹ can be introduced after synthesis of a photochromic compound having a halogen atom. An example of the method will be described below.

First, a reaction is performed in the same manner as in the above example except that a compound having a halogen atom instead of the substituent Z¹ is used. Then, using a benzophenone compound having a halogen atom instead of Z¹, a carboxylic acid compound represented by formula (15) below obtained by a Stobbe reaction, a cyclization reaction, or a hydrolysis reaction using alkali or acid is subjected to spiroannulation under a neutral to acidic condition and a reduction reaction using hydrogen, hydrazine, or the like to obtain a naphthol derivative represented by formula (16) below.

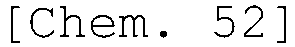

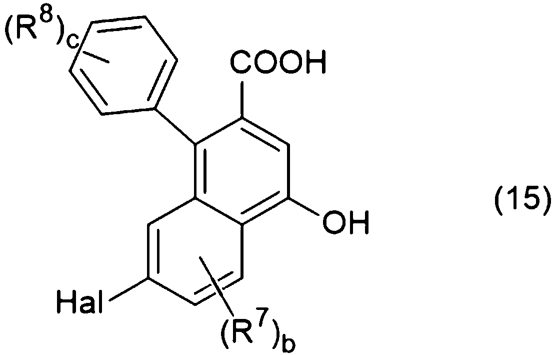

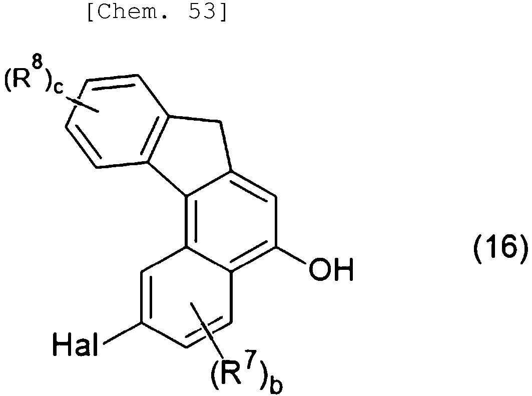

As a method other than the above method, the compound represented by formula (16) can be synthesized by subjecting the halogen compound represented by formula (15) to intramolecular cyclization in the presence of a palladium catalyst.

The resulting naphthol derivative is used to obtain a photochromic compound represented by formula (17) below.

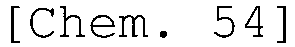

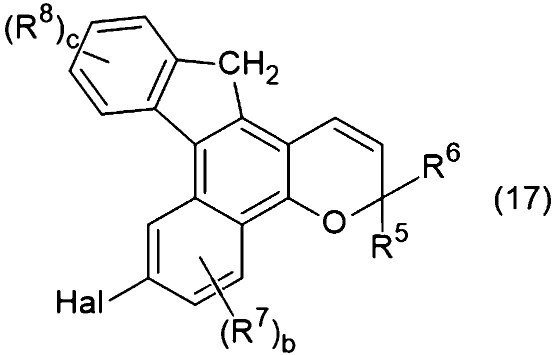

The resulting photochromic compound is reacted with the halide of R³ under a basic condition to obtain a photochromic compound precursor represented by formula (18) below.

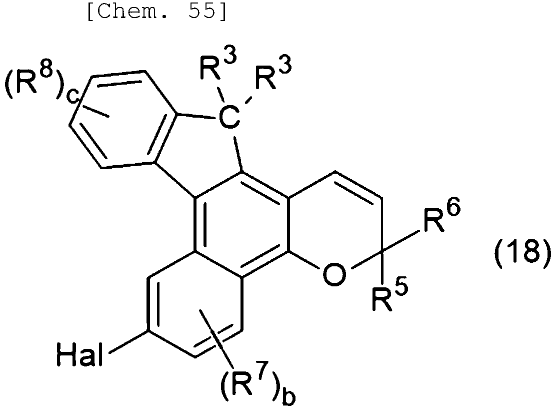

The photochromic compound according to the present invention can be obtained by Buchwald reaction between a halogen atom of the obtained precursor and an amine having the Z¹-H structure using a Pd catalyst, or the like.

The naphthol compound represented by formula (6Aa) may be obtained by a method below. First, a benzene compound represented by formula (8A) below is reacted with the acid chloride compound represented by formula (9) above to obtain a benzophenone compound represented by formula (10A) below. Except that this benzophenone compound is used, the naphthol compound represented by formula (6Aa) can be obtained in the same manner as the method for producing the naphthol compound represented by formula (6) above.

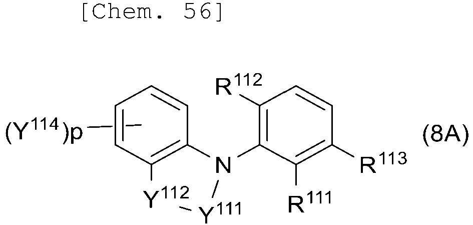

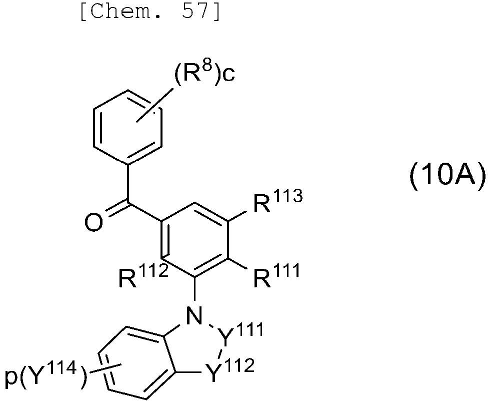

Depending on the structure and substituents of the photochromic compound to be synthesized, the substituent of the acid chloride compound and the substituent of the benzene compound may be interchanged. That means, the acid chloride compound having R¹¹¹ to R¹¹³ may be reacted with the benzene compound having R⁸.

### <Method for Synthesizing Naphthol Derivative Including Si and Ge>

An example of a method for producing the naphthol derivative represented by formula (6) with M being Si or Ge will be explained below.

First, the halide represented by formula (12) is reacted with magnesium, lithium, or the like to prepare an organometallic reagent. This organometallic reagent is reacted with a monohalide represented by formula (19) below (in the formula, R³ and R⁴ are the same as those in formula (4)) at - 100 to 70°C in an organic solvent to obtain a compound represented by formula (20) below.

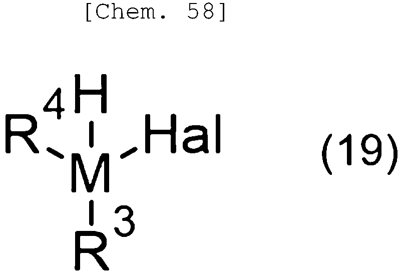

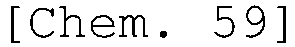

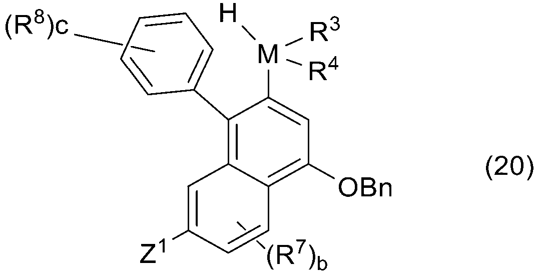

The resulting compound (20) is subjected to a reaction by referring to reaction methods described in Non-Patent Document 3, Non-Patent Document 4, Patent Document 4, and the like, to obtain a cyclized group represented by formula (21) below.

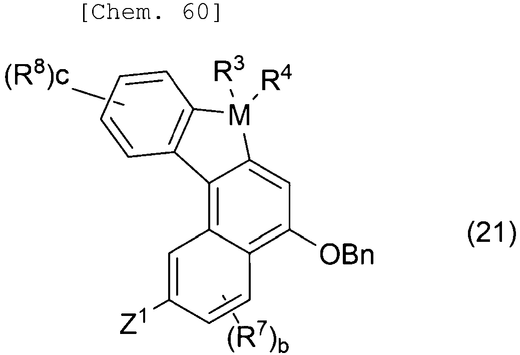

The resulting cyclized group can be debenzylized to obtain the naphthol derivative represented by formula (6a). The naphthol derivative represented by formula (6Aa) can be obtained in the same manner as the method for formula (6a) except that Z¹ was changed.

### <Identification of Photochromic Compound>

The photochromic compound according to the embodiment is, for example, a solid or viscous liquid at room temperature and atmospheric pressure. From this solid or liquid, a photochromic compound can be isolated by a separation operation such as a thin-layer chromatography, a silica gel column chromatography, a high-performance liquid chromatography, and a gas chromatography. Furthermore, it is confirmed that the isolate does not include by-products other than the photochromic compound, such as raw material compounds and coloring components.

In a proton nuclear magnetic resonance spectroscopy (¹H-NMR), the obtained photochromic compound showed peaks attributed to aromatic protons and protons of an alkene at around δ: 5.0 to 9.0 ppm, and peaks attributed to protons of an alkyl group and an alkylene group at around δ: 1.0 to 4.0 ppm. When the intensities of each spectrum are relatively compared, the number of protons corresponding to each bond group can be determined. This makes it possible to identify the skeleton, substituents, or the like of the photochromic compound.

When the photochromic compound is contained in a cured product such as a resin, this resin is dissolved and subjected to the above-described separation methods to isolate the photochromic compound.

### <Photochromic Composition>

The photochromic compound according to the embodiment is soluble in general organic solvents such as toluene, chloroform, and tetrahydrofuran. The photochromic compound having the skeleton represented by formula (1) is dissolved in such a solvent to obtain a colorless transparent solution. When this solution is exposed to sunlight or ultraviolet ray, it rapidly develops color, and when it is blocked from sunlight or the like, it reversibly and rapidly returns to its original colorless state and exhibits a good photochromic function.

Furthermore, the photochromic compound according to the embodiment can be used in combination with other photochromic compounds having other structures, depending on an intended purpose. For example, the photochromic compound can be used in combination with other photochromic compounds for the purpose of achieving various color tones required for photochromic lenses. The photochromic compounds to be used in combination can be any known compound with no limitation. Examples of the photochromic compounds to be used in combination include indenonaphthopyran, naphthopyran, spirooxazine, spiropyran, fulgide, fulgimide, diarylethene, and the like. Above all, an indenonaphthopyran compound is particularly preferable because it can maintain uniform color tones during color development/fading, suppress color shift during color development accompanying degradation of the photochromic properties, and further reduce the initial coloring. It is preferable to adjust the color tone by using plural kinds of photochromic compounds in combination, because the combination can achieve both a high color optical density and a high color fading speed particularly at a high temperature and in addition has an excellent durability.

When the photochromic composition includes the photochromic compound according to the embodiment and other photochromic compounds, the compounding ratios of each photochromic compound are determined as appropriate depending on a desired color tone.

### <Photochromic Curable Composition>

The curable composition according to the embodiment contains the photochromic compound according to the embodiment, and at least one selected from a group consisting of a radical polymerizable monomer, a cationic polymerizable monomer, a compound having a polymerizable group, and a (thio) urethane (urea) polymer. In this case, the (thio)urethane (urea) polymer includes at least one selected from a group consisting of a urethane polymer, a thiourethane polymer, a urethaneurea polymer, and a thiourethaneurea polymer.

Preferably, the photochromic compound and photochromic composition according to the embodiment are used as a photochromic curable composition in combination with a polymerizable compound.

The photochromic curable composition depends on a color development intensity of the photochromic compound, selected lens materials, and a lens thickness, and therefore it is preferable that, although it is not always the case, the photochromic compound (or photochromic composition) is used in an amount of 0.001 to 10 parts by mass based on 100 parts by mass of the polymerizable compound. An optimal compounding ratio of this photochromic compound varies depending on an intended purpose. For example, there is a difference as described below between a case that the photochromic curable composition is used as a thin-film optical article and a case that the photochromic curable composition is used as a thick-film optical article.

### (Use as Thin-Film Optical Article)

For example, when the photochromic curable composition is formed into a thin film having a thickness of 10 µm or more and less than 1000 µm, e.g. about 100 µm (polymer film with the polymerized photochromic curable composition), it is preferable that the color tone is adjusted by blending the photochromic compound (or photochromic composition) in an amount of 0.001 to 10 parts by mass based on 100 parts by mass of other polymerizable monomers.

### (Use as Thick-Film Optical Article)

When the photochromic curable composition is formed into a thick cured product (polymer formed product with the polymerized photochromic curable composition) having a thickness of e.g. 1 mm or more, it is preferable that the color tone is adjusted by blending the photochromic compound (or photochromic composition) according to the present invention in an amount of 0.001 to 1 part by mass based on 100 parts by mass of the thick cured product or other polymerizable monomers capable of providing a thick cured product.

### <Polymerizable Compound>

As described above, the photochromic compound is preferably used in combination with a polymerizable compound, as a photochromic curable composition. Examples of the polymerizable compound include a urethane or urea-based polymerizable compound capable of forming a urethane bond, a urea bond, or the like, a compound having a polymerizable group, a radical-polymerizable compound, an epoxy-based polymerizable compound, and the like. These polymerizable compounds are not particularly limited, but, for example, the polymerizable compound described in Patent Document 5 can be suitably used.

Above all, the following polymerizable compounds are particularly suitable for use.

### <Compound Having Polymerizable Group>

The compound having a polymerizable group is exemplified by a compound having an iso(thio)cyanate group. The iso(thio)cyanate compound is a compound having an isocyanate group or an isothiocyanate group, and may have both the isocyanate group and isothiocyanate group. Suitably, this compound is used in combination with a compound including active hydrogen described later. Examples of such an iso(thio)cyanate compound include, but are not limited to, the following compounds.

### (Polyiso(thio)cyanate)

Polyiso(thio)cyanates are compounds having at least two or more iso(thio)cyanate groups in one molecule. Examples of the polyiso(thio)cyanate include an aromatic polyiso(thio)cyanate having an aromatic ring, such as m-xylene diisocyanate and 4,4'-diphenylmethane diisocyanate, and an aliphatic polyiso(thio)cyanate such as norbornane diisocyanate and dicyclohexylmethane-4,4'-diisocyanate.

### (Compound Having Active Hydrogen)

The compound containing active hydrogen is preferably, but not limited to, a compound having a hydroxy group and/or a thiol group, particularly preferably a polyfunctional compound having two or more active hydrogen atoms in one molecule. Specific examples of the compound having active hydrogen include: a polyfunctional thiol compound such as pentaerythritol tetrakis(3-mercaptopropionate) and 4-mercaptomethyl-3,6-dithia-octanedithiol, and a polyfunctional alcohol such as trimethylolpropane and pentaerythritol.

### (Radical-Polymerizable Compound)

The radical-polymerizable compound includes polyfunctional radical-polymerizable compounds and monofunctional radical-polymerizable compounds. These compounds can be used alone or in combination with others. Examples of radical-polymerizable substituents include a group having an unsaturated double bond, i.e. a vinyl group (including a styryl group, a (meth)acrylic group, an allyl group, and the like).

The polyfunctional radical-polymerizable compound has two or more radical-polymerizable substituents in one molecule. This polyfunctional radical-polymerizable compound includes a first polyfunctional radical-polymerizable compound having 2 to 10 radical-polymerizable substituents and a second polyfunctional radical-polymerizable compound having more than 10 radical-polymerizable substituents.

The first polyfunctional radical-polymerizable compound is not particularly limited, but, more preferably, it has 2 to 6 radical-polymerizable substituents. Specific examples thereof are as follows.

(Polyfunctional (Meth)acrylic Acid Ester Compound) Ethylene glycol di(meth)acrylate, diethylene glycol di(meth)acrylate, triethylene glycol di(meth)acrylate, tetraethylene glycol di(meth)acrylate, ethylene glycol bisglycidyl(meth)acrylate, bisphenol A di(meth)acrylate, 2,2-bis(4-(meth)acryloyloxyethoxyphenyl)propane, 2,2-bis(3,5-dibromo-4-(meth)acryloyloxyethoxyphenyl)propane.

### (Polyfunctional Allyl Compound)

Diallyl phthalate, diallyl terephthalate, diallyl isophthalate, diallyl tartrate, diallyl epoxy succinate, diallyl fumarate, diallyl chlorendate, diallyl hexaphthalate, diallyl carbonate, allyl diglycol carbonate, trimethylol propane triallyl carbonate.

### (Polyfunctional Thio(meth)acrylic Acid Ester Compound)

1,2-bis(methacryloylthio)ethane, bis(2-acryloylthioethyl)ether, 1,4-bis(methacryloylthiomethyl)benzene.

### (Vinyl Compound)

Divinyl benzene.

Examples of the second polyfunctional radically-polymerizable compound having more than 10 radical-polymerizable substituents include relatively high-molecular-weight compounds such as a silsesquioxane compound having a radical-polymerizable substituent and a polyrotaxane compound having a radical-polymerizable substituent.

The monofunctional radical-polymerizable compound has one radical-polymerizable substituent in its molecule, and specific examples thereof include, but are not limited to, the following compounds.

### (Unsaturated Carboxylic Acid)

Acrylic acid, methacrylic acid, maleic anhydride.

### ((Meth)acrylic Acid Ester)

Methyl (meth)acrylate, benzyl methacrylate, phenyl methacrylate.

2-hydroxyethyl methacrylate, glycidyl (meth)acrylate, β-methylglycidyl (meth)acrylate, bisphenol A-monoglycidyl ether-methacrylate, 4-glycidyl oxymethacrylate, 3-(glycidyl-2-oxyethoxy)-2-hydroxy, propyl methacrylate, 3-(glycidyloxy-1-isopropyloxy)-2-hydroxypropyl acrylate, 3-glycidyloxy-2-hydroxypropyloxy)-2-hydroxypropyl acrylate.

### (Fumaric Acid Ester)

Diethyl fumarate, diphenyl fumarate.

### (Thio(meth)acrylic Acid)

Methyl thioacrylate, benzyl thioacrylate, benzyl thiomethacrylate.

### (Vinyl Compound)

Styrene, chlorostyrene, thylstyrene, vinyl naphthalene, α-methylstyrene dimer, bromostyrene.

The radical-polymerizable compound may be used alone or in combination of two or more types. In this case, based on 100 parts by mass of the total amount of the radical-polymerizable compounds, the amounts of the polyfunctional radical-polymerizable compound and the monofunctional radical-polymerizable compound are preferably 80 to 100 parts by mass and 0 to 20 parts by mass respectively, more preferably 90 to 100 parts by mass and 0 to 10 parts by mass respectively. Furthermore, based on 100 parts by mass of the total amount of the radical-polymerizable compound, the amounts of the first polyfunctional radical-polymerizable compound, the second polyfunctional radical-polymerizable compound, and the monofunctional radical polymerizable compound are preferably 80 to 100 parts by mass, 0 to 20 parts by mass, and 0 to 20 parts by mass respectively, more preferably 85 to 100 parts by mass, 0 to 10 parts by mass, and 0 to 10 parts by mass respectively.

### (Various Compounding Agents)

The curable composition may contain various known compounding agents unless the effects of the composition are impaired. Examples of compounding agents include various stabilizers such as release agents, ultraviolet absorbers, infrared absorbers, ultraviolet stabilizers, antioxidants, coloring inhibitors, antistatic agents, fluorescent dyes, dyes, pigments, and fragrances. Also, solvents and leveling agents may be blended. Thiols such as t-dodecylmercaptan may be blended as polymerization modifier.

Among the above compounding agents, ultraviolet stabilizers are suitable because they can improve the durability of the photochromic site. As such ultraviolet stabilizers, hindered amine light stabilizers, hindered phenol antioxidants, sulfur-based antioxidants, and the like are known. Particularly suitable ultraviolet stabilizers are as follows.

Bis(1,2,2,6,6-pentamethyl-4-piperidyl) sebacate; ADEKA Corporation's ADK STAB LA-52, LA-57, LA-62, LA-63, LA-67, LA-77, LA-82, LA-87; 2,6-di-tert-butyl-4-methylphenol, ethylene bis(oxyethylene) bis[3-(5-tert-butyl-4-hydroxy-m-tolyl) propionate]; BASF Japan's IRGANOX 1010, 1035, 1075, 1098, 1135, 1141, 1222, 1330, 1425, 1520, 259, 3114, 3790, 5057, 565. An amount used of such an ultraviolet stabilizer is not particularly limited unless the effects of the stabilizer are impaired, but is typically in a range of 0.001 to 10 parts by mass, particularly 0.01 to 1 part by mass based on 100 parts by mass of the photochromic curable composition.

In addition to the ultraviolet stabilizer, an ultraviolet absorber may also be used. As the ultraviolet absorber, a known ultraviolet absorber such as a benzophenone compound, a benzotriazole compound, s cyanoacrylate compound, a triazine compound, and a benzoate compound may be used. Above all, a cyanoacrylate compound and a benzophenone compound are preferable. Preferably, the above ultraviolet stabilizer is used in a range of 0.001 to 5 parts by mass based on 100 parts by mass of the photochromic curable composition containing a photochromic compound and a polymerizable compound.

### <Method for Using Photochromic Curable Composition; Optical Article>

A photochromic cured product is obtained by curing the photochromic curable composition. The polymerization curing for preparing the photochromic cured product is performed by irradiation with active energy rays such as ultraviolet ray, α-ray, β-ray, and γ-ray, heating, or a combination thereof, through a radical polymerization, a ring-opening polymerization, an anion polymerization, or a condensation polymerization. That means, an appropriate polymerization means should be adopted depending on the types of the polymerizable compound or polymerization curing promoter and the form of the photochromic cured product to be formed.

When thermally polymerizing a curable composition containing a polymerizable compound, the temperature affects the properties of the resulting photochromic cured product.

This temperature condition is affected by the type and amount of the thermal polymerization initiator and the type of the polymerizable compound, and therefore, although it cannot be generally limited, it is generally preferable to initiate the polymerization at a relatively low temperature and gradually increase the temperature. Since the polymerization time also varies depending on various factors like the temperature, it is preferable to previously determine the optimal time in accordance with these conditions. However, generally, it is desirable to select a polymerization condition such that the polymerization is completed within 2 to 48 hours. When obtaining a photochromic laminated sheet, it is preferable that the polymerization is performed at a temperature at which the reaction between polymerizable functional groups proceeds, and, at this time, the optimal temperature and time are determined such that the molecular weight is a desired weight.

Moreover, when photopolymerizing the curable composition, particularly the UV intensity among the polymerization condition affects the properties of the resulting photochromic cured product. This illuminance condition is affected by the type and amount of the photoinitiator and the type of the polymerizable monomer, and therefore, although it cannot be generally limited, it is generally preferable to select a condition such that UV light at a wavelength of 365 nm having an intensity of 50 to 500 mW/cm² is emitted for 0.5 to 5 minutes.

### [Optical Article]

The photochromic compound according to the embodiment can be widely used as a photochromic material, e.g. as various memory materials that are alternatives for silver halide photosensitive materials, copying materials, printing photoreceptors, cathode ray tube memory materials, laser photosensitive materials, and various memory materials such as holography photosensitive materials. Photochromic materials can also be used as photochromic lens materials, optical filter materials, display materials, as well as materials for actinometers, decoration, fabric, string, and the like.

The photochromic compound according to the embodiment is suitable particularly for photochromic lens applications. Photochromic lenses are suitable for eyeglasses lenses such as sunglass lenses. As the method for producing the photochromic lens, any known method can be adopted as long as a uniform dimming performance can be achieved.

When photochromic properties are exhibited by a kneading method, the above-described curable composition is injected into between glass molds held by an elastomer gasket or spacer, and then cast-polymerized by heating in an air furnace or irradiation with an active energy ray such as ultraviolet ray depending on the types of the polymerizable compound and polymerization curing promoter, resulting in a photochromic cured product formed into an optical material such as a lens.

When exhibiting photochromic properties by a lamination method, the curable composition is dissolved in an appropriate organic solvent to prepare a coating liquid, which is then applied on a surface of an optical substrate such as a lens substrate by spin coating, dipping, or the like. The coating liquid is dried to remove the organic solvent, and then cured by polymerization with UV irradiation, heating, or the like in an inert gas such as nitrogen, to form a photochromic layer consisting of the photochromic cured product on the surface of the optical substrate (coating method).

Alternatively, an optical substrate such as a lens substrate is arranged so as to face a glass mold such that predetermined voids are formed, and a curable composition is injected into these voids. In this state, a photochromic layer consisting of a photochromic cured product can be formed on the surface of the optical substrate also by a cast polymerization using an inner mold in which polymerization curing is performed by UV irradiation, heating, or the like (cast polymerization method).

When forming a photochromic layer on a surface of an optical substrate using the lamination method as described above (coating method and cast polymerization method), the adhesiveness between the photochromic layer and the optical substrate can be improved by previously subjecting a surface of the optical substrate to a chemical treatment using an alkaline solution, an acidic solution, or the like, or a physical treatment using corona discharge, plasma discharge, polishing, or the like. Needless to say, it is also possible to provide a transparent adhesive resin layer on the surface of the optical substrate.

Furthermore, when exhibiting the photochromic properties by a binder method, a photochromic sheet is prepared by sheet forming using a curable composition, this sheet is inserted into between two transparent sheets (optical sheets) and then cured by polymerization as described above, to obtain a photochromic laminate having a photochromic layer as an adhesive layer.

In this case, for the preparation of the photochromic sheet, it is also possible to adopt a method in which a curable composition is dissolved in an organic solvent to prepare a coating liquid, and this coating liquid is used for coating. An adhesive layer may be provided between the photochromic sheet and the optical sheet.

For example, the photochromic laminate prepared in this manner is placed in a mold, onto which a thermoplastic resin (e.g. polycarbonate) for an optical substrate such as lens is injection-formed, to obtain an optical substrate such as lens in a predetermined shape having photochromic properties.

Moreover, this photochromic laminate can be bonded to the surface of the optical substrate using an adhesive or the like to obtain a photochromic lens.

Note that, when preparing the photochromic laminate as described above, particularly in terms of high adhesiveness with the optical substrate, it is preferable that a urethane or urea-based polymerizable compound, particularly a urethane-based polymerizable compound is used as a polymerizable compound, and adjusted so as to form a polyurethane.

The above-described curable composition can exhibit a photochromic property with excellent color optical density at a high temperature.

In addition, the photochromic layer or photochromic cured product prepared from the curable composition can be subjected to post-processings e.g. dying using dyes such as dispersant dyes, formation of a hard coat film using a silane coupling agent, or a hard coating agent predominantly containing a sol of silicon, zirconium, antimony, aluminum, tin, tungsten, etc., formation of a thin film by vapor deposition of metal oxides such as SiO₂, TiO₂, and ZrO₂, antireflection treatment using a thin film formed by application of organic polymers, and anti-static treatment.

### EXAMPLES

The present invention will be explained in more detail with reference to Examples below. These Examples are intended to merely explain the present invention, and the spirit and scope of the present invention are not limited to these Examples.

### (Example 1)

### First Step

By referring to the method described in Patent Document 6, 28.9 g (62.4 mmol) of compound represented by formula (22) below synthesized from 3-bromo-4-methoxybenzophenone, 550 mL of toluene, 9.36 g (87.4 mmol) of N-methylaniline, and 24.0 g (249.6 mmol) of t-butoxy sodium were mixed and stirred under reduced pressure, from which dissolved oxygen was removed. Subsequently, 0.57 g (0.6 mmol) of Pd₂(dba)₃ and 1.19 g (2.5 mmol) of X-phos were added to the reaction liquid, which was heated to 80°C.

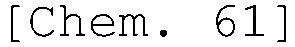

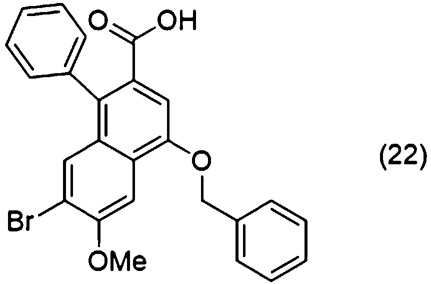

The heating was continued until the raw materials became invisible, and after completion of the reaction, the reaction liquid was cooled to room temperature and then filtrated. To the resulting filtrate, 500 mL of tetrahydrofuran and 10% hydrochloric acid was added, which was neutralized and then subjected to liquid separation. The resulting organic layer was concentrated and then purified by reslurry with 100 mL of methanol to obtain a carboxylic acid compound represented by formula (23) below with a yield of 90%.

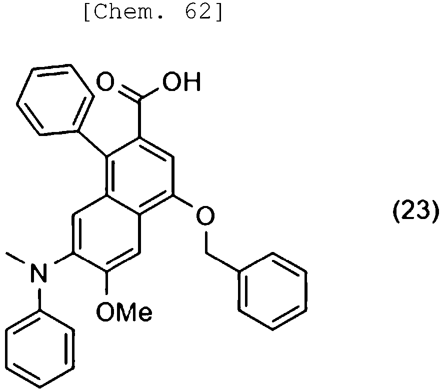

### Second Step

By referring to the method described in Patent Document 6 except that the carboxylic acid compound obtained in the first step was used, an iodine compound represented by formula (24) below was obtained with a yield of 82%.

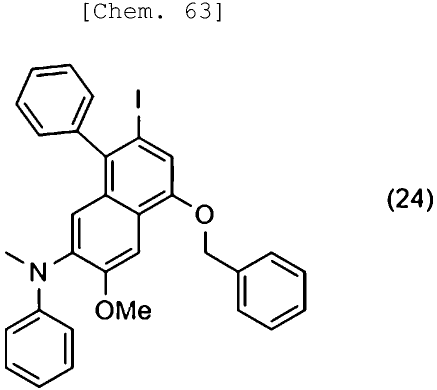

### Third Step

To 26.3 g (46.1 mmol) of the compound represented by formula (24) above obtained in the second step, 400 mL of toluene was added, and subjected to azeotropic dehydration until the water content in toluene decreased to 100 ppm or less. After the azeotropic dehydration, the mixture was slowly cooled to - 20°C, into which 35 mL of n-BuLi (1.6 mol/L hexane solution) was slowly dripped while maintaining the temperature at -15 to -20°C. After confirming that the raw materials had been consumed, 3.5 g of dehydrated acetone was dripped into the mixture. After the dripping, the temperature was slowly raised to room temperature. After the temperature rising, 200 mL of water was added to the mixture, which was subjected to liquid separation. The mixture was repeatedly washed with water until a pH of a water layer reached 7 to 8. From the obtained organic layer, the solvent was removed, and the organic layer was purified by a chromatography on silica gel to obtain a compound represented by formula (25) below with a yield of 83%.

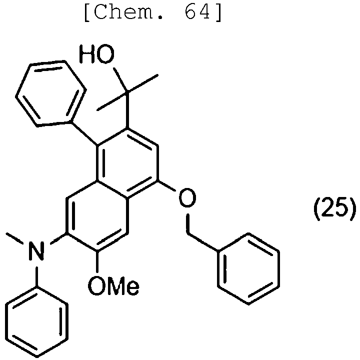

### Fourth Step

To 28.4 g (150.0 mmol) of p-toluenesulfonic acid monohydrate, 750 mL of toluene was added, which was subjected to azeotropic dehydration until the water content in toluene decreased to 300 ppm or less. Then, into this mixture, a toluene solution with 19.3 g (38.3 mmol) of the compound represented by formula (25) above dissolved in 100 mL of toluene was slowly dripped while maintaining the temperature at 85 to 100°C, and after the dripping, the mixture was refluxed. After confirming that the raw materials had been consumed, the mixture was cooled to room temperature, to which 500 mL of water was added, which was subjected to liquid separation. This operation was repeated three times, the solvent was removed from the obtained organic layer, and the organic layer was purified by a chromatography on silica gel to obtain a naphthol derivative represented by formula (26) below with a yield of 74%.

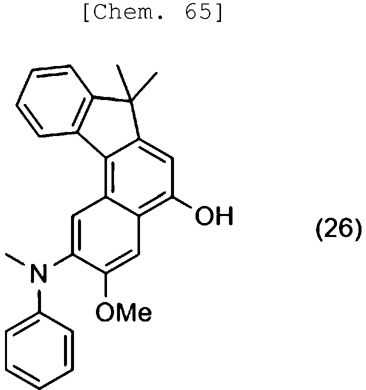

### Fifth Step

Into 40 mL of toluene, 1.98 g (5.0 mmol) of the naphthol derivative represented by formula (26) above and 1.60 g (6.0 mmol) of propargyl alcohol represented by formula (27) below were dissolved, additionally 0.12 g (0.5 mmol) of pyridinium p-toluenesulfonate was added, and the mixture was stirred at 85°C for 1 hour.

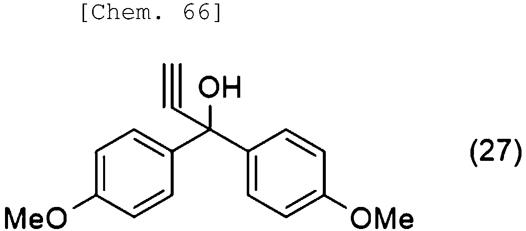

After consuming the naphthol derivative as the raw material, the mixture was cooled to room temperature, and 40 mL of water was added to the mixture, which was subjected to liquid separation. From the obtained organic layer, the solvent was removed, and the organic layer was purified by a chromatography on silica gel to obtain a photochromic compound represented by formula (28) below with a yield of 83%.

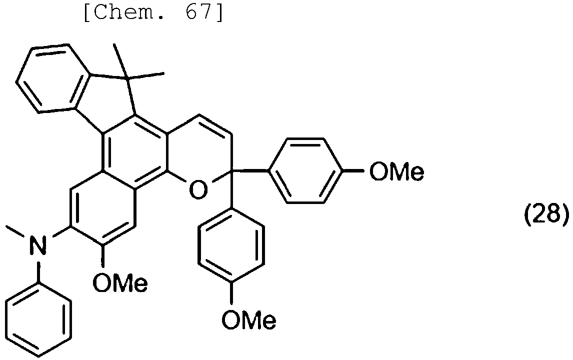

The photochromic compound represented by formula (28) above had elemental analysis values of C: 81.86%, H: 6.04%, and N: 2.14%, which were considerably consistent with the C₄₄H₄₉NO₄ calculated values of C: 81.83%, H: 6.09%, and N: 2.17%.

In a proton nuclear magnetic resonance spectroscopy, the photochromic compound represented by formula (28) showed a 9H peak attributed to a methyl group at around 50.5 to 3.0 ppm, a 9H peak attributed to a methoxy group at around δ3.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (28) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 2)

The same reaction as in the first step of Example 1 was performed except that diphenylamine was used instead of N-methylaniline, to synthesize a naphthol derivative represented by formula (29) below, and further to obtain a photochromic compound represented by formula (30) below with a yield of 75%.

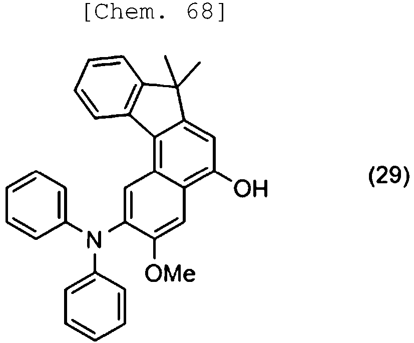

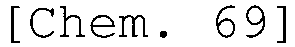

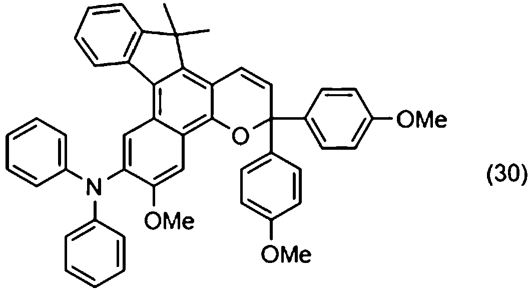

The photochromic compound represented by formula (30) above had elemental analysis values of C: 83.17%, H: 5.86%, and N: 1.96%, which were considerably consistent with the C₄₉H₄₁NO₄ calculated values of C: 83.14%, H: 5.84%, and N: 1.98%.

In a proton nuclear magnetic resonance spectroscopy, the photochromic compound represented by formula (30) showed a 6H peak attributed to a methyl group at around δ0.5 to 3.0 ppm, a 9H peak attributed to a methoxy group at around δ3.0 to 5.0 ppm, and 26H peaks attributed to aromatic protons and protons of an alkene at around δ5.0 to 9.0 ppm.

Furthermore, in a ¹³C-nuclear magnetic resonance spectroscopy, the photochromic compound represented by formula (30) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 3)

The same reaction as in the third step of Example 1 was performed except that 4-heptanone was used instead of acetone, to synthesize a naphthol derivative represented by formula (31) below, and further to obtain a photochromic compound represented by formula (32) below with a yield of 80%.

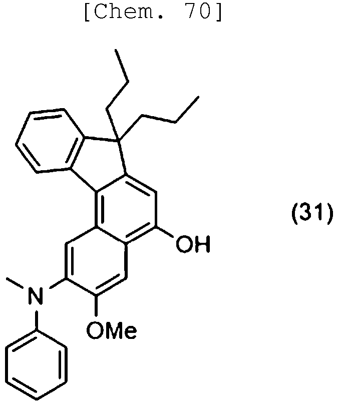

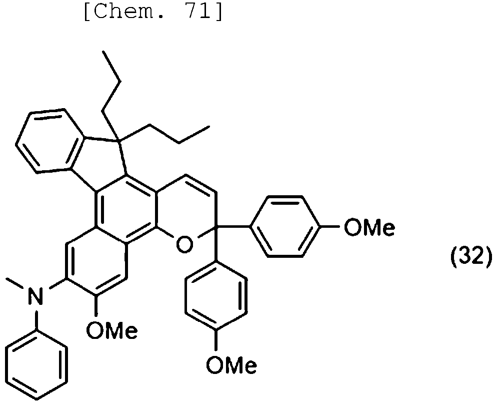

The photochromic compound represented by formula (32) above had elemental analysis values of C: 82.11%, H: 6.78%, and N: 1.99%, which were considerably consistent with the C₄₈H₄₇NO₄ calculated values of C: 82.14%, H: 6.75%, and N: 2.00%. In a proton nuclear magnetic resonance spectroscopy, the photochromic compound represented by formula (32) showed 17H peaks attributed to a methyl group and a propyl group at around δ0.5 to 3.0 ppm, a 9H peak attributed to a methoxy group at around 63.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (32) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 4)

The same reaction as in Example 3 was performed except that N-methyl-(4-fluorophenyl)amine was used instead of N-methylaniline, to synthesize a naphthol derivative represented by formula (33) below, and further to obtain a photochromic compound represented by formula (34) with a yield of 79%.

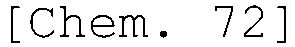

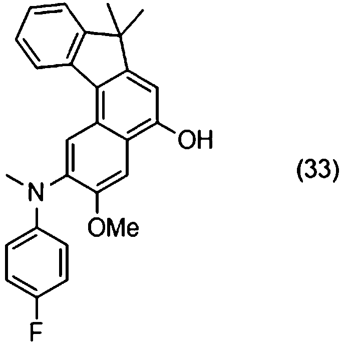

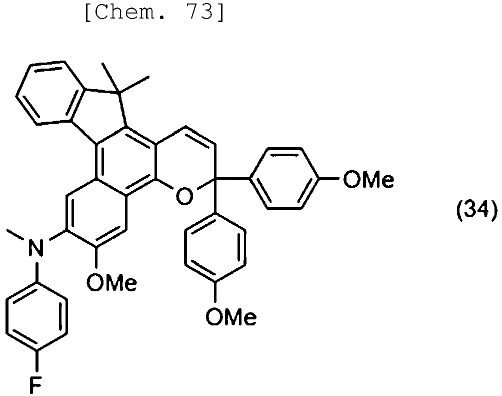

The photochromic compound represented by formula (34) above had elemental analysis values of C: 79.58%, H: 5.79%, and N: 2.14%, which were considerably consistent with the C₄₄H₃₈FNO₄ calculated values of C: 79.62%, H: 5.77%, and N: 2.11%. In a proton nuclear magnetic resonance spectroscopy, the photochromic compound represented by formula (34) showed a 9H peak attributed to a methyl group at around δ0.5 to 3.0 ppm, a 9H peak attributed to a methoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (34) showed a peak attributed to carbon of an aromatic ring at around 5110 to 160 ppm, a peak attributed to carbon of an alkene at around δ80 to 140 ppm, and a peak attributed to carbon of an alkyl at δ20 to 60 ppm.

### (Example 5)

### First Step

By referring to the method described in Patent Document 6, 95.4 g (500 mmol) of p-toluenesulfonic acid monohydrate and 1000 mL of toluene were added to 35.7 g (100 mmol) of carboxylic acid represented by formula (35) below synthesized from 3-bromo-4-methylbenzophenone, and reacted while performing azeotropic dehydration. After confirming that the raw materials had been consumed, the mixture was cooled to room temperature, and then the resulting solid was filtrated to obtain a carbonyl compound represented by formula (36) below with a yield of 90%.

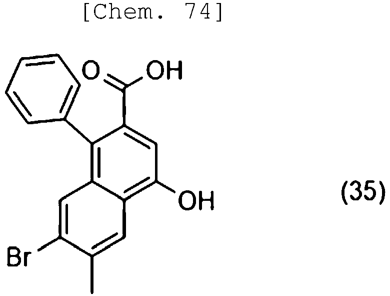

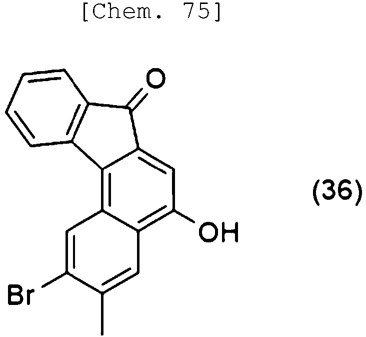

### Second Step

By referring to the method described in Patent Document 7, a reaction was performed using the carbonyl compound represented by formula (36) above to obtain a naphthol compound represented by formula (37) below with a yield of 87%.

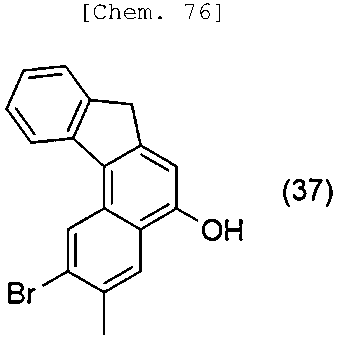

### Third Step

Into 250 ml of DMF, 25.5 g (78.3 mmol) of naphthol compound represented by formula (37) above and 12.4 g (97.6 mmol) of benzyl chloride were dissolved. To this solution, 19.4 g (140.6 mmol) of potassium carbonate was added, which was stirred at 80°C. After raw material consumption, the mixture was cooled to room temperature, to which water was added, and the solution was washed until the solution became neutral. From the obtained organic layer, the solvent was removed, and the organic layer was purified by a chromatography on silica gel to obtain a benzyl compound represented by formula (38) below with a yield of 97%.

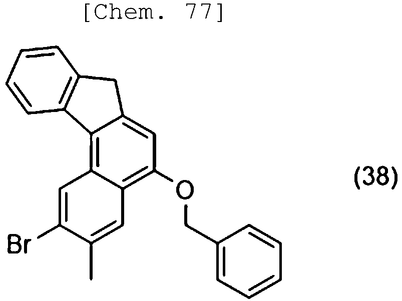

### Fourth Step

First, 4.2 g (10.0 mmol) of benzyl compound represented by formula (38) above, 8.4 g (50.0 mmol) of 1-bromo-4-methoxybutane, and 60 mL of THF were mixed, and cooled with ice. To the mixture, 3.4 g (30.0 mmol) of tBuOK was added in four batches while maintaining the temperature at 0 to 5°C. After raw materials consumption, the mixture was neutralized with 10% hydrochloric acid, to which 30 mL of toluene was added, and the mixture was subjected to liquid separation. From the obtained organic layer, the solvent was removed, and then the organic layer was purified by a chromatography on silica gel to obtain a benzyl body represented by formula (39) below with a yield of 83%.

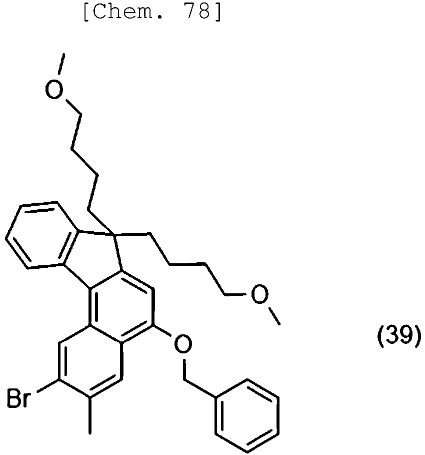

### Fifth Step

The reaction was performed in the same manner as in the first step of Example 1 to obtain a benzyl body represented by formula (40) below. To the obtained benzyl body, 40 mL of THF and 1.0 g of 5% Pd/C (water content: 50%) were added, and the mixture was stirred under a pressurized hydrogen condition for 12 hours. After completion of the reaction, the mixture was filtrated, and, from the resulting filtrate, THF was removed. Then, the mixture was purified by a chromatography on silica gel to obtain a naphthol derivative represented by formula (41) below with a yield of 88%.

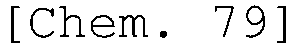

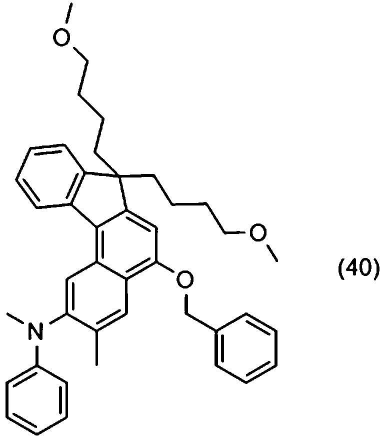

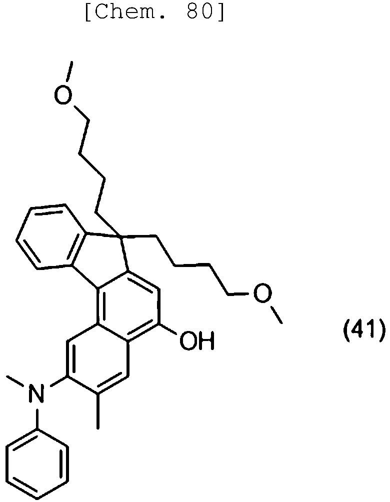

### Sixth Step

The same reaction as in the fifth step of Example 1 was performed except that the naphthol derivative represented by formula (41) above was used instead of the naphthol derivative represented by formula (26) above, and a propargyl alcohol represented by formula (42) below was used instead of the propargyl alcohol represented by formula (27) above, to obtain a photochromic compound represented by formula (43) below with a yield of 76%.

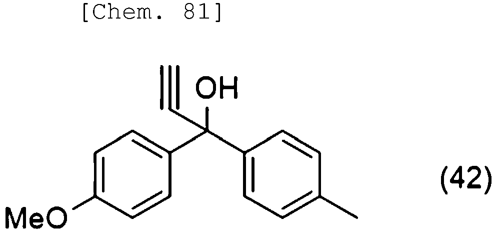

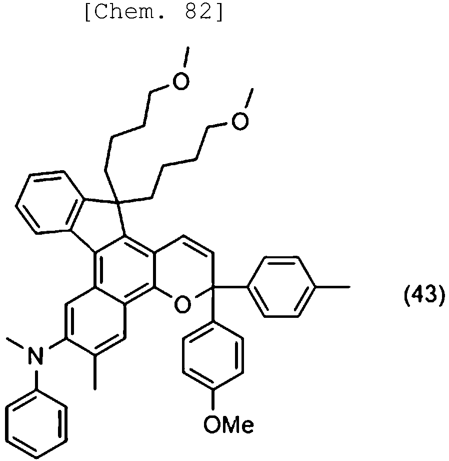

The photochromic compound represented by formula (43) above had elemental analysis values of C: 82.43%, H: 7.27%, and N: 1.84%, which were considerably consistent with the C₅₂H₅₅NO₄ calculated values of C: 82.40%, H: 7.31%, and N: 1.85%. In a proton nuclear magnetic resonance spectroscopy, the photochromic compound represented by formula (43) showed 21H peaks attributed to a methyl group and a butylene group at around δ0.5 to 3.0 ppm, 13H peaks attributed to a methoxy group and a butyleneoxy group at around δ3.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (43) showed a peak attributed to carbon of an aromatic ring at around 5110 to 160 ppm, a peak attributed to carbon of an alkene at around δ80 to 140 ppm, and a peak attributed to carbon of an alkyl at δ20 to 60 ppm.

### (Example 6)

### First Step

The same reaction as in the first step of Example 1 was performed except that N-(2-methoxy)ethylaniline was used instead of N-methylaniline, to obtain an iodine compound represented by formula (44) below with a yield of 70%.

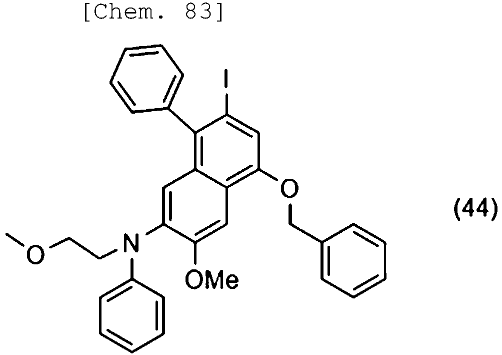

### Second Step

The same reaction as in the third step of Example 1 was performed except that cyclooctanone was used instead of acetone, to obtain a naphthol derivative represented by formula (45) below with a yield of 43%.

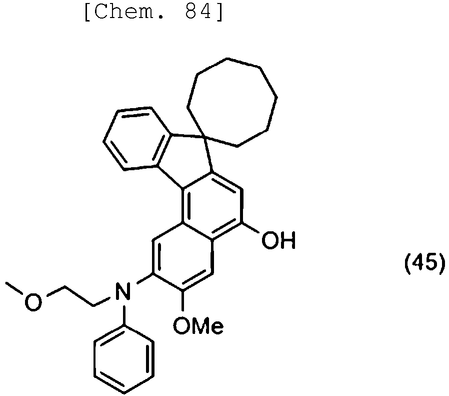

### Third Step

The same reaction as in the fifth step of Example 1 was performed except that the naphthol derivative represented by formula (45) above was used instead of the naphthol derivative represented by formula (26) above and a propargyl alcohol represented by formula (46) below was used instead of the propargyl alcohol represented by formula (27) above, to obtain a photochromic compound represented by formula (47) below with a yield of 71%.

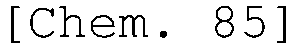

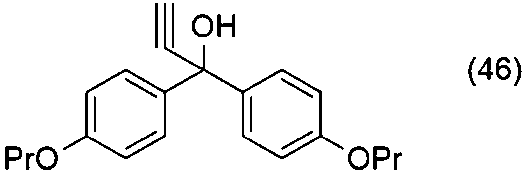

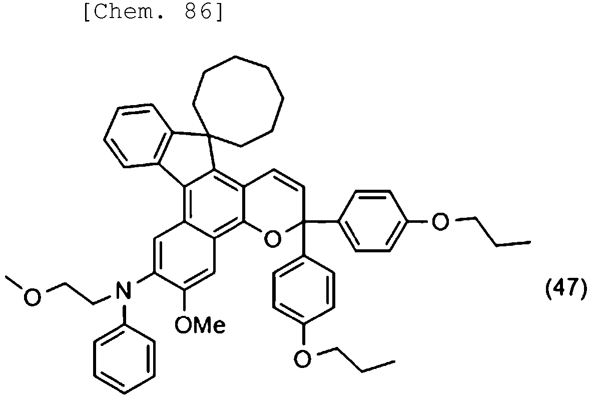

The photochromic compound represented by formula (47) above had elemental analysis values of C: 81.19%, H: 7.34%, and N: 1.69%, which were considerably consistent with the C₅₂H₅₉NO₅ calculated values of C: 81.15%, H: 7.31%, and N: 1.72%.

In a proton nuclear magnetic resonance spectroscopy, the photochromic compound represented by formula (47) showed 26H peaks attributed to an ethylene group, a cyclooctylene group, and a propoxy group at around δ0.5 to 3.0 ppm, 12H peaks attributed to a methoxy group, an ethyleneoxy group, and a propoxy group at around δ3.0 to 5.0 ppm, and 21H peaks attributed to aromatic protons and protons of an alkene at around δ5.0 to 9.0 ppm.

Furthermore, in a ¹³C-nuclear magnetic resonance spectroscopy, the photochromic compound represented by formula (47) showed a peak attributed to carbon of an aromatic ring at around 5110 to 160 ppm, a peak attributed to carbon of an alkene at around δ80 to 140 ppm, and a peak attributed to carbon of an alkyl at δ20 to 60 ppm.

### (Example 7)

### First Step

The same reaction as in the first step of Example 3 was performed except that diphenylamine was used instead of the N-methylamine, to synthesize a naphthol derivative represented by formula (48) below. The same reaction as in the first step of Example 3 was performed except that a propargyl alcohol represented by formula (49) below was used, to obtain a photochromic compound represented by formula (50) below with a yield of 77%.

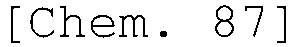

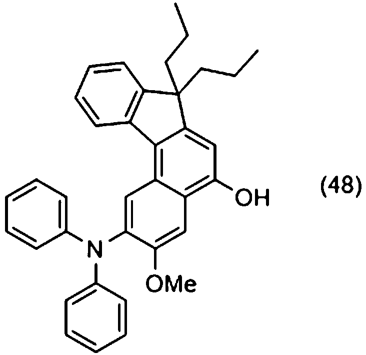

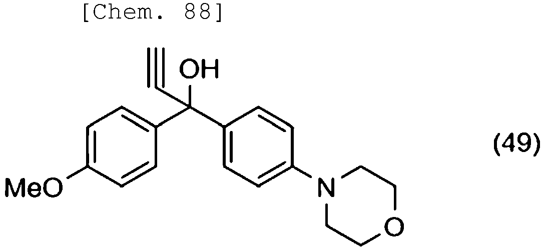

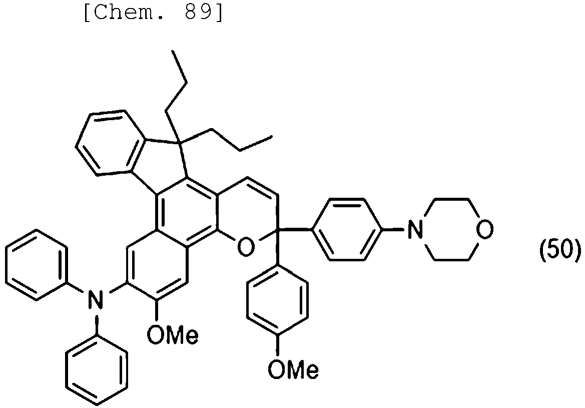

The photochromic compound represented by formula (50) above had elemental analysis values of C: 82.09%, H: 6.68%, and N: 3.39%, which were considerably consistent with the C₅₆H₅₄N₂O₄ calculated values of C: 82.12%, H: 6.65%, and N: 3.42%.

In a proton nuclear magnetic resonance spectroscopy, the photochromic compound represented by formula (50) showed 18H peaks attributed to a propyl group and a morpholino group at around δ0.5 to 3.0 ppm, 10H peaks attributed to a methoxy group and a morpholino group at around δ3.0 to 5.0 ppm, and 26H peaks attributed to aromatic protons and protons of an alkene at around 65.0 to 9.0 ppm.

Furthermore, in a ¹³C-nuclear magnetic resonance spectroscopy, the photochromic compound represented by formula (50) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 8)

### First Step

The same reaction as in the first Example 5 was performed except that 3-bromo-4-methox-4'-methylbenzophenone was used instead of 3-bromo-4-methylbenzophenone, to obtain a benzyl compound represented by formula (51) below with a yield of 83%.

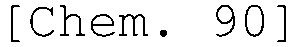

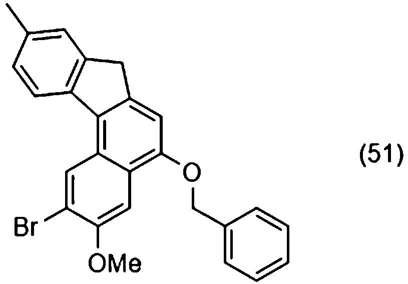

### Second Step

The same reaction as in the fifth step of Example 5 was performed except that 1,2,3,4-tetrahydroquinoline was used instead of N-methylaniline, to obtain a benzyl compound represented by formula (52) below.

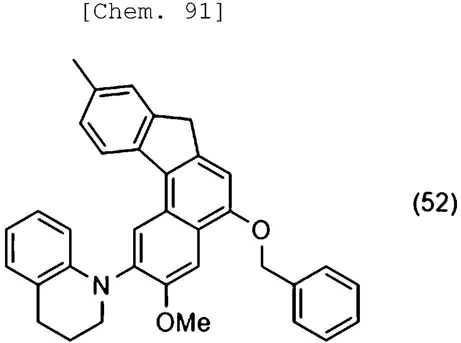

### Third Step

The same reaction as in the fourth step of Example 5 was performed except that 4-bromo-1,1,1-trifluorobutane was used instead of 1-bromo-4-methoxybutane, to synthesize a naphthol derivative represented by formula (53) below.

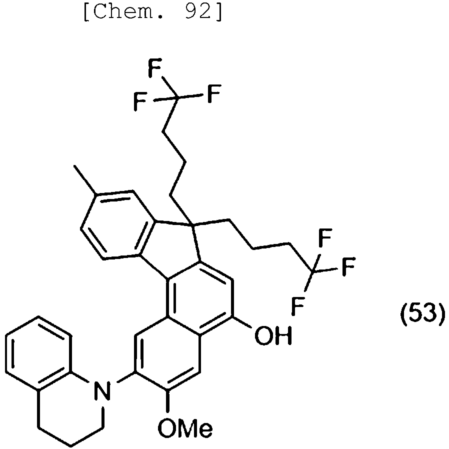

### Fourth Step

The same reaction as in the first step of Example 7 was performed except that the naphthol derivative represented by formula (53) above was used instead of the naphthol derivative represented by formula (48) above, to obtain a photochromic compound represented by formula (54) below with a yield of 73%.

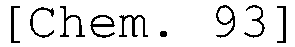

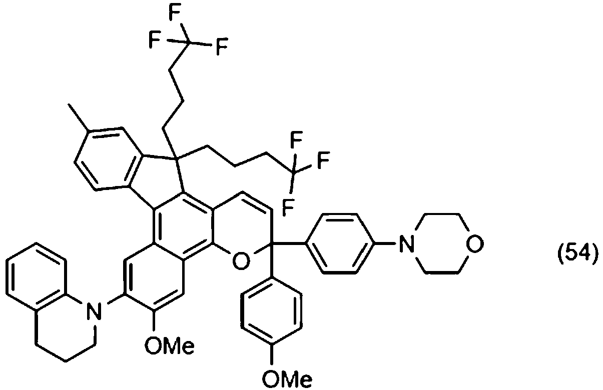

The photochromic compound represented by formula (54) above had elemental analysis values of C: 72.06%, H: 5.82%, and N: 3.04%, which were considerably consistent with the C₅₆H₅₄F₆N₂O₄ calculated values of C: 72.09%, H: 5.83%, and N: 3.00%. In a proton nuclear magnetic resonance spectroscopy, the photochromic compound represented by formula (54) showed 25H peaks attributed to a methyl group, a trifluoropropyl group, a morpholino group, and a tetrahydroquinoline ring at around 50.5 to 3.0 ppm, 10H peaks attributed to a methoxy group and a morpholino group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (54) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 9)

### First Step

The same reaction as in the first step of Example 6 was performed except that N-methyl-4-(trifluoromethyl)benzeneamine was used, to obtain an iodine compound represented by formula (55) below with a yield of 75%.

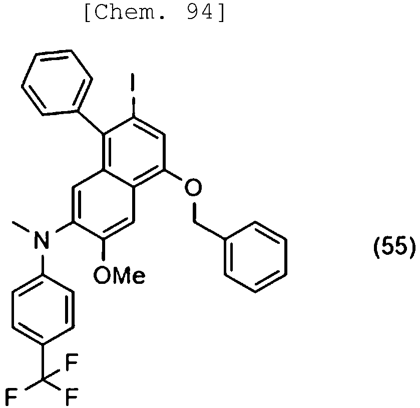

### Second Step

The same reaction as in the second step of Example 6 was performed except that 4,4-diethylcyclohexanone was used instead of cyclooctanone, to obtain a naphthol derivative represented by formula (56) below with a yield of 83%.

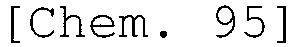

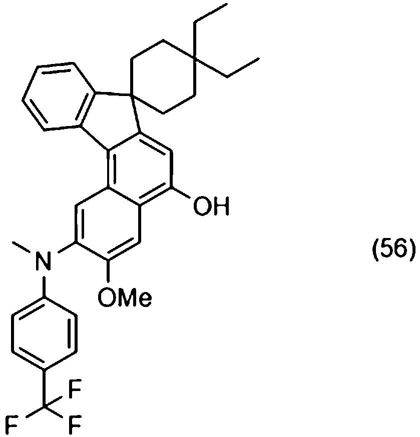

### Third Step

The same reaction as in the sixth step of Example 5 was performed except that the naphthol derivative represented by formula (56) above was used instead of the naphthol derivative represented by formula (41) above, to obtain a photochromic compound represented by formula (57) below with a yield of 76%.

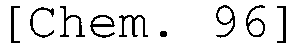

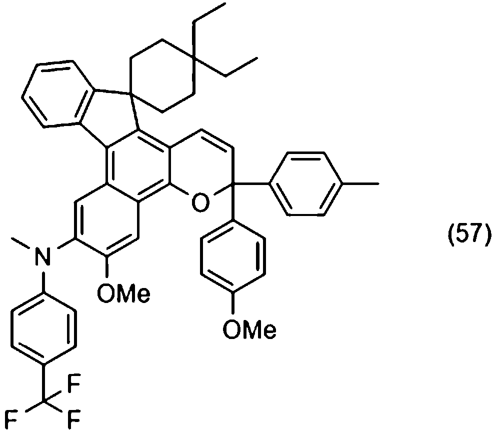

The photochromic compound represented by formula (57) above had elemental analysis values of C: 78.70%, H: 6.33%, and N: 1.75%, which were considerably consistent with the C₅₂H₅₀F₃NO₃ calculated values of C: 78.66%, H: 6.35%, and N: 1.76%.

In a proton nuclear magnetic resonance spectroscopy, the photochromic compound represented by formula (57) showed 24H peaks attributed to a methyl group and a diethylcyclohexyl group at around 50.5 to 3.0 ppm, a 6H peak attributed to a methoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (57) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 10)

### First Step

The same reaction as in the first step of Example 3 was performed except that 3,4-dihydro-2H-1,4-benzoxazine was used instead of N-methylaniline, to synthesize a naphthol derivative represented by formula (58) below, and further to obtain a photochromic compound represented by formula (59) below with a yield of 83%.

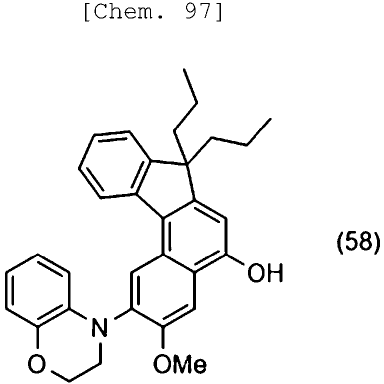

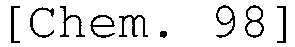

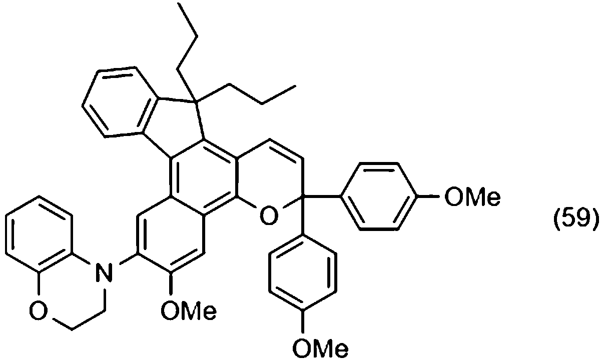

The photochromic compound represented by formula (59) above had elemental analysis values of C: 80.66%, H: 6.49%, and N: 1.90%, which were considerably consistent with the C₄₉H₄₇NO₅ calculated values of C: 80.63%, H: 6.49%, and N: 1.92%.

In a proton nuclear magnetic resonance spectroscopy, the photochromic compound represented by formula (59) showed 16H peaks attributed to a propyl group and a benzoxazoline ring at around 50.5 to 3.0 ppm, 11H peaks attributed to a methoxy group and a benzoxazoline ring at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (59) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 11)

### First Step

The same reaction as in Example 5 was performed except that 3-bromo-4-methoxybenzophenone was used instead of 3-bromo-4-methylbenzophenone, to obtain a benzyl body represented by formula (60) below with a yield of 79%.

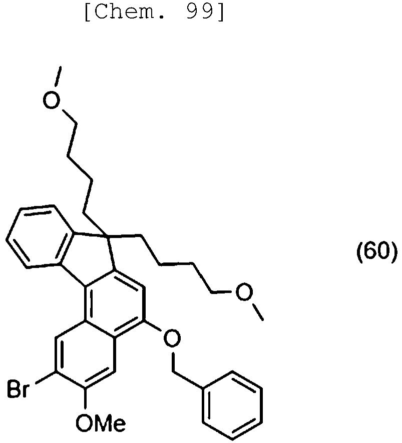

### Second Step

The same reaction as in the first step of Example 1 was performed except that bis(4-trifluoromethylphenyl)amine was used instead of N-methylaniline, to obtain a naphthol derivative represented by formula (61) below with a yield of 86%.

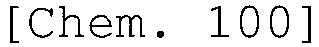

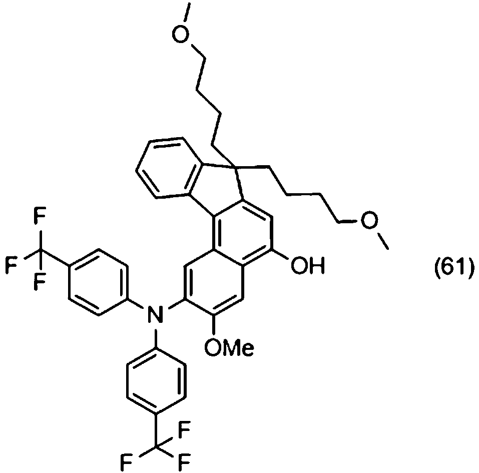

### Third Step

The same reaction as in the fifth step of Example 1 was performed except that the naphthol derivative represented by formula (61) above was used instead of the naphthol derivative represented by formula (26) above and a propargyl alcohol represented by formula (62) below was used instead of the propargyl alcohol represented by formula (27) above, to obtain a photochromic compound represented by formula (63) below with a yield of 82%.

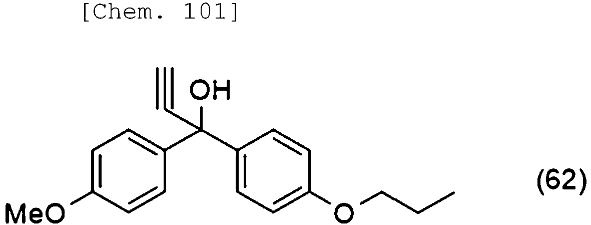

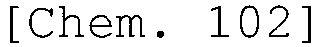

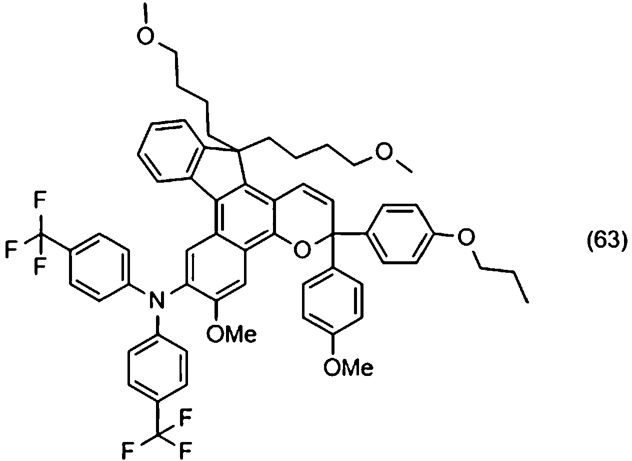

The photochromic compound represented by formula (63) above had elemental analysis values of C: 72.06%, H: 5.87%, and N: 1.41%, which were considerably consistent with the C₆₁H₅₉F₆NO₆ calculated values of C: 72.10%, H: 5.85%, and N: 1.38%.

In a proton nuclear magnetic resonance spectroscopy, the photochromic compound represented by formula (63) showed 17H peaks attributed to a butylene group and a propyl group at around 50.5 to 3.0 ppm, 18H peaks attributed to a methoxy group, a butyleneoxy group, and a propyleneoxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (63) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 12)

### First Step

The same reaction as in the first step of Example 1 was performed except that bis(4-fluorophenyl)amine was used instead of N-methylaniline, to obtain an iodine compound represented by formula (64) below with a yield of 76%.

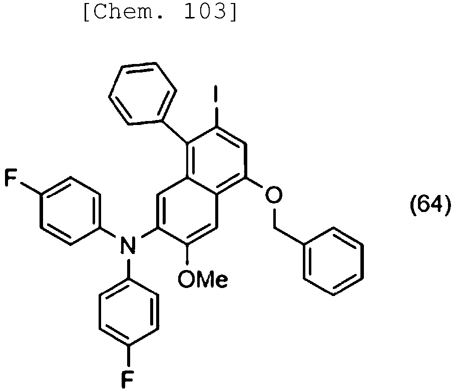

### Second Step

The same reaction as in the third step of Example 1 was performed except that dibutylketone was used instead of acetone, to obtain a naphthol derivative represented by formula (65) below with a yield of 80%.

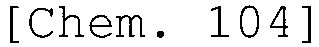

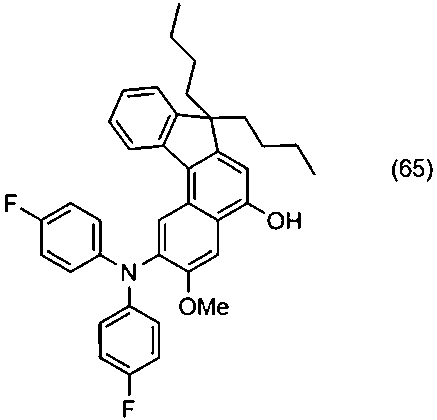

### Third Step

The same reaction as in the fifth step of Example 1 was performed except that the naphthol derivative represented by formula (65) above was used instead of the naphthol derivative represented by formula (26) above, to obtain a photochromic compound represented by formula (66) below with a yield of 87%.

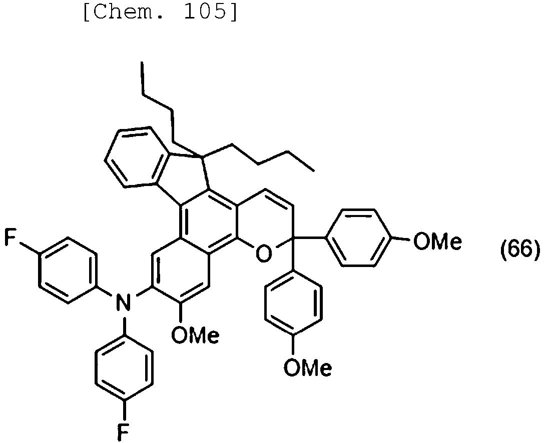

The photochromic compound represented by formula (66) above had elemental analysis values of C: 79.76%, H: 6.21%, and N: 1.70%, which were considerably consistent with the C₅₅H₅₁F₂NO₄ calculated values of C: 79.78%, H: 6.21%, and N: 1.69%.

In a proton nuclear magnetic resonance spectroscopy, the photochromic compound represented by formula (66) showed a 18H peak attributed to a butyl group at around δ0.5 to 3.0 ppm, a 9H peak attributed to a methoxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (66) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 13)

### First Step

The same reaction as in Example 7 was performed except that (4-methylphenyl)phenylamine was used instead of diphenylamine, to obtain a naphthol derivative represented by formula (67) below with a yield of 87%.

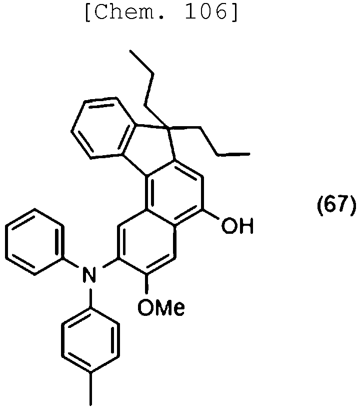

### Second Step

The same reaction as in the fifth step of Example 1 was performed except that the naphthol derivative represented by formula (67) above was used instead of the naphthol derivative represented by formula (26) above, to obtain a photochromic compound represented by formula (68) below with a yield of 89%.

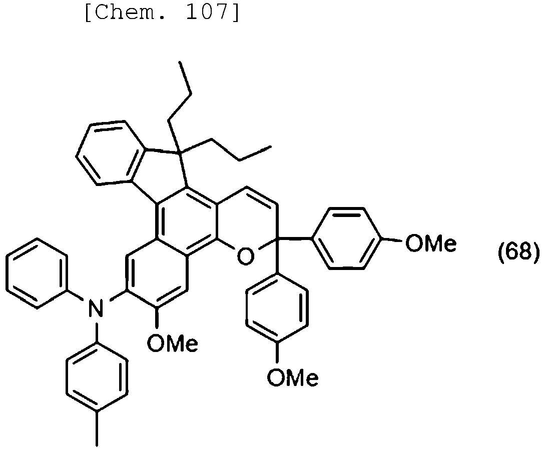

The photochromic compound represented by formula (68) above had elemental analysis values of C: 83.39%, H: 6.64%, and N: 1.81%, which were considerably consistent with the C₅₄H₅₁NO₄ calculated values of C: 83.37%, H: 6.61%, and N: 1.80%.

In a proton nuclear magnetic resonance spectroscopy, the photochromic compound represented by formula (68) showed 17H peaks attributed to a methyl group and a propyl group at around δ0.5 to 3.0 ppm, a 9H peak attributed to a methoxy group at around 63.0 to 5.0 ppm, and 25H peaks attributed to aromatic protons and protons of an alkene at around δ5.0 to 9.0 ppm.

Furthermore, in a ¹³C-nuclear magnetic resonance spectroscopy, the photochromic compound represented by formula (68) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 14)

### First Step

The same reaction as in the first step of Example was performed except that 1-iodo-4-(methoxymethoxy)butane was used instead of 1-bromo-4-methoxybutane, to obtain a benzyl body represented by formula (69) below, and then the same reaction as in the second step of Example 11 was performed except that bis(4-methylphenyl)amine was used instead of bis(4-trifluoromethylphenyl)amine, to obtain a naphthol derivative represented by formula (70) below with a yield of 80%.

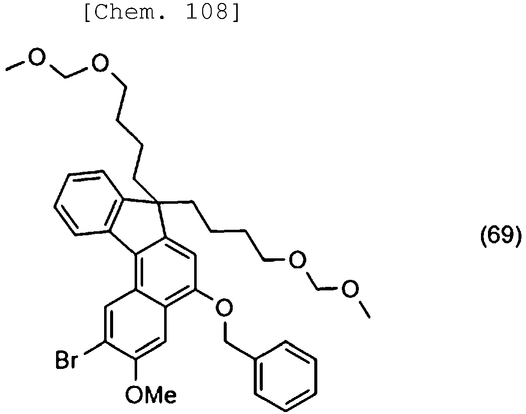

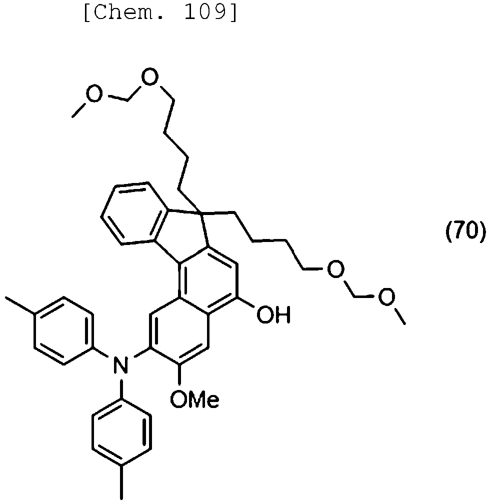

### Second Step

The same reaction as in the third step of Example 6 was performed except that the naphthol derivative represented by formula (70) above was used instead of the naphthol derivative represented by formula (45) above, to obtain a photochromic compound represented by formula (71) below with a yield of 80%.

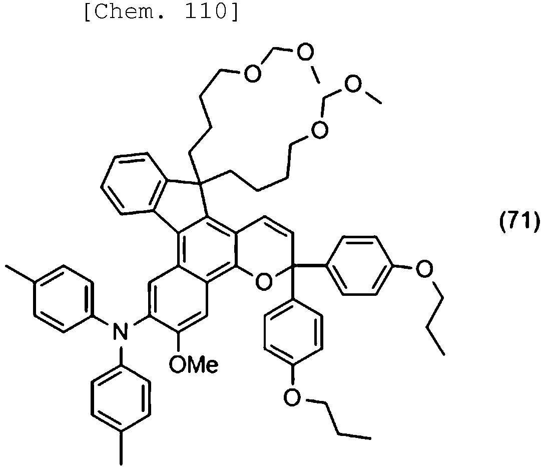

The photochromic compound represented by formula (71) above had elemental analysis values of C: 78.39%, H: 7.41%, and N: 1.39%, which were considerably consistent with the C₆₆H₇₃NO₈ calculated values of C: 78.36%, H: 7.39%, and N: 1.41%.

In a proton nuclear magnetic resonance spectroscopy, the photochromic compound represented by formula (71) showed 28H peaks attributed to a butyleneoxy group, a propoxy group, and a methyl group at around 50.5 to 3.0 ppm, 21H peaks attributed to a methoxy group, a propoxy group, and a butyleneoxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (71) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 15)

### First Step

The same reaction as in the first step of Example 1 was performed except that (4-methylphenyl) (4-methoxyphenyl)amine was used instead of N-methylaniline, to obtain an iodine compound represented by formula (72) below. The same reaction as in the third step of Example 1 was performed except that the obtained iodine compound was reacted with 3,3,5,5-tetramethylcyclohexanone, to obtain a naphthol derivative represented by formula (73) below with a yield of 87%.

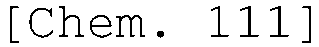

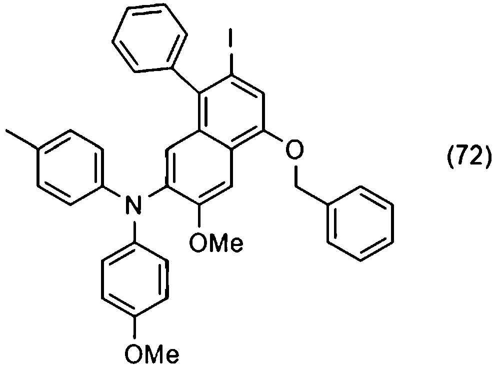

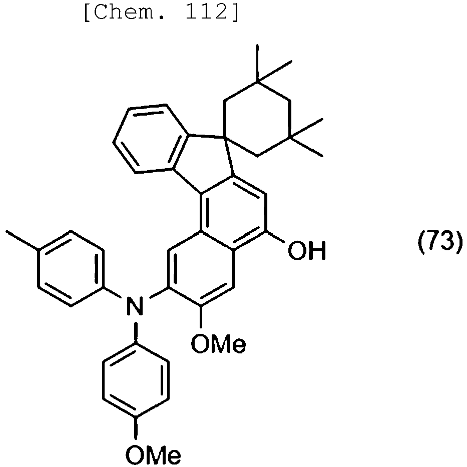

### Second Step

The same reaction as in the fifth step of Example 1 was performed except that the naphthol derivative represented by formula (73) above was used instead of the naphthol derivative represented by formula (26) above and a propargyl alcohol represented by formula (74) below was used instead of the propargyl alcohol represented by formula (27) above, to obtain a photochromic compound represented by formula (75) below with a yield of 82%.

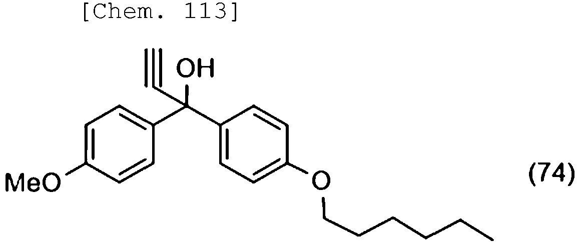

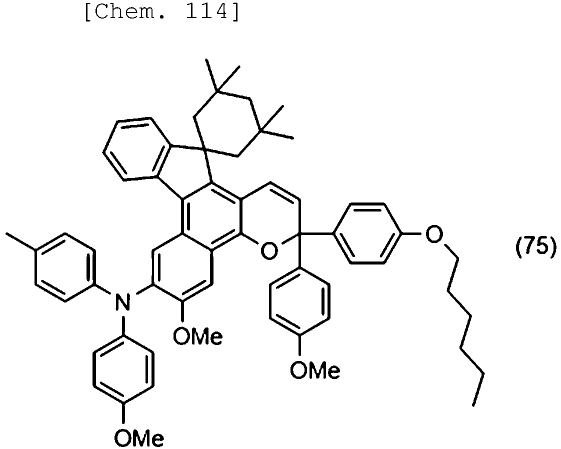

The photochromic compound represented by formula (75) above had elemental analysis values of C: 82.44%, H: 7.34%, and N: 1.52%, which were considerably consistent with the C₆₃H₆₇NO₅ calculated values of C: 82.41%, H: 7.35%, and N: 1.53%.

In a proton nuclear magnetic resonance spectroscopy, the photochromic compound represented by formula (75) showed 32H peaks attributed to a methyl group, a 3,3,5,5-tetramethylcyclohexylene group, and a hexyloxy group at around 50.5 to 3.0 ppm, 11H peaks attributed to a methoxy group and a hexyloxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (75) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 16)

### First Step

The same reaction as in the first step of Example 11 was performed except that N-butyl (4-fluorophenyl)amine was used instead of bis(4-trifluoromethylphenyl)amine, to obtain a naphthol derivative represented by formula (76) below with a yield of 76%.

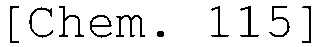

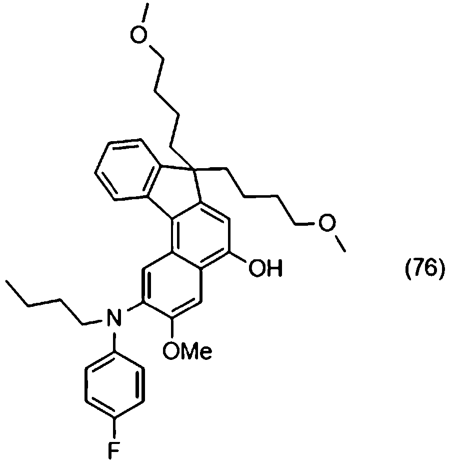

### Second Step

The same reaction as in the fifth step of Example 1 was performed except that the naphthol derivative represented by formula (76) above was used instead of the naphthol derivative represented by formula (26) above, to obtain a photochromic compound represented by formula (77) below with a yield of 84%.

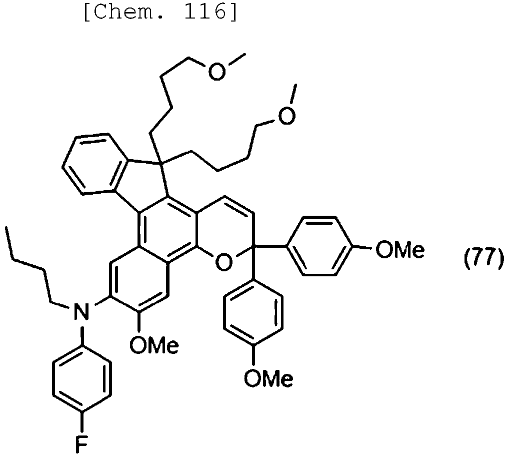

The photochromic compound represented by formula (77) above had elemental analysis values of C: 77.75%, H: 7.08%, and N: 1.68%, which were considerably consistent with the C₅₅H₆₀FNO₆ calculated values of C: 77.71%, H: 7.11%, and N: 1.65%.

In a proton nuclear magnetic resonance spectroscopy, the photochromic compound represented by formula (77) showed 21H peaks attributed to a butyl group and a butyleneoxy group at around δ0.5 to 3.0 ppm, 19H peaks attributed to a methoxy group and a butyleneoxy group at around 63.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (77) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 17)

### First Step

The same reaction as in the fifth step of Example 5 was performed except that 3-bromo-4-methyl-4'-methylbenzophenone was used instead of 3-bromo-4-methylbenzophenone, to obtain a naphthol derivative represented by formula (78) below with a yield of 76%.

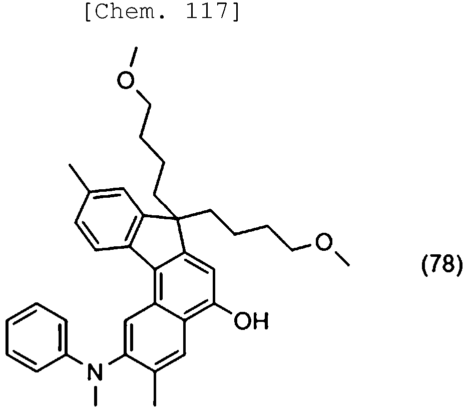

### Second Step

The same reaction as in the second step of Example 11 was performed except that the naphthol derivative represented by formula (78) above was used instead of the naphthol derivative represented by formula (61) above, to obtain a photochromic compound represented by formula (79) below with a yield of 79%.

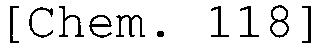

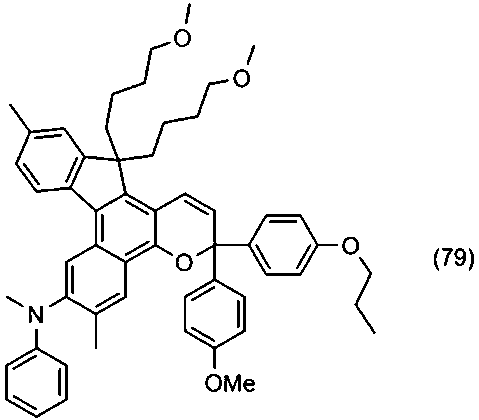

The photochromic compound represented by formula (79) above had elemental analysis values of C: 80.94%, H: 7.52%, and N: 1.73%, which were considerably consistent with the C₅₅H₆₁NO₅ calculated values of C: 80.95%, H: 7.53%, and N: 1.72%. In a proton nuclear magnetic resonance spectroscopy, the photochromic compound represented by formula (79) showed 26H peaks attributed to a methyl group, a butyleneoxy group, and a propoxy group at around δ0.5 to 3.0 ppm, 15H peaks attributed to a methoxy group, a butyleneoxy group, and a propoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (79) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 18)

The same reaction as in the second step of Example 8 was performed except that N-methylaniline was used instead of 1,2,3,4-tetrahydroquinoline, and the same reaction as in the third step of Example 8 was performed except that iodopropane was used instead of 4-bromo-1,1,1-trifluorobutane, to obtain a naphthol derivative represented by formula (80) below with a yield of 81%.

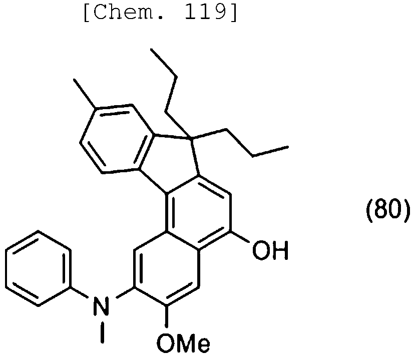

### Second Step

The same reaction as in the fifth step of Example 1 was performed except that the naphthol derivative represented by formula (80) above was used instead of the naphthol derivative represented by formula (26) above, to obtain a photochromic compound represented by formula (81) below with a yield of 73%.

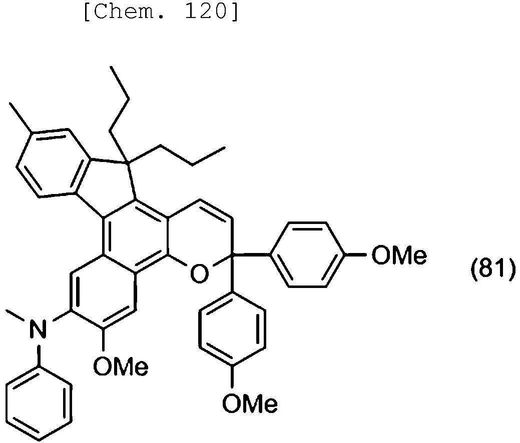

The photochromic compound represented by formula (81) above had elemental analysis values of C: 82.23%, H: 6.89%, and N: 1.96%, which were considerably consistent with the C₄₉H₄₉NO₄ calculated values of C: 82.21%, H: 6.90%, and N: 1.96%.

In a proton nuclear magnetic resonance spectroscopy, the photochromic compound represented by formula (81) showed 20H peaks attributed to a methyl group and a propyl group at around δ0.5 to 3.0 ppm, a 9H peak attributed to a methoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (81) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 19)

### First Step

The same reaction as in the first step of Example 1 was performed except that 3,4-dihydro-2H-1,4-benzoxazine was used instead of N-methylaniline, to synthesize a naphthol derivative represented by formula (82) below, and further to obtain a photochromic compound represented by formula (83) with a yield of 78%.

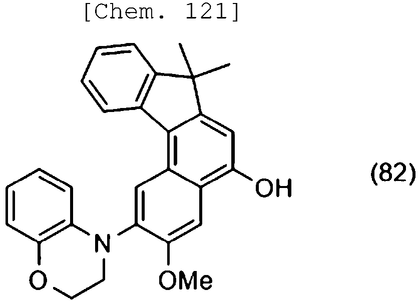

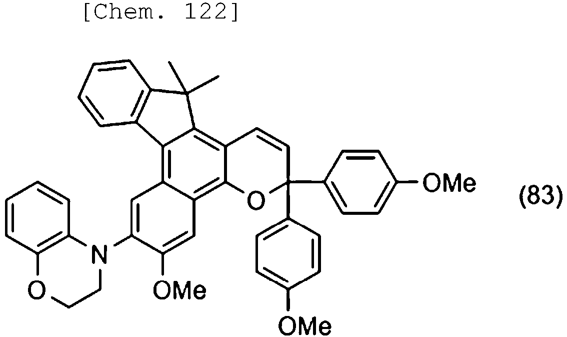

The photochromic compound represented by formula (83) above had elemental analysis values of C: 80.24%, H: 5.80%, and N: 2.10%, which were considerably consistent with the C₄₅H₃₉NO₅ calculated values of C: 80.21%, H: 5.83%, and N: 2.08%.

In a proton nuclear magnetic resonance spectroscopy, the photochromic compound represented by formula (83) showed 8H peaks attributed to a methyl group and a benzoxazoline ring at around δ0.5 to 3.0 ppm, 11H peaks attributed to a methoxy group and a benzoxazoline ring at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (83) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 20)

### First Step

The same reaction as in the first step of Example 16 was performed except that N-methyl-(4-methylphenyl)amine was used instead of N-butyl(4-fluorophenyl)amine, to obtain a naphthol derivative represented by formula (84) below with a yield of 79%.

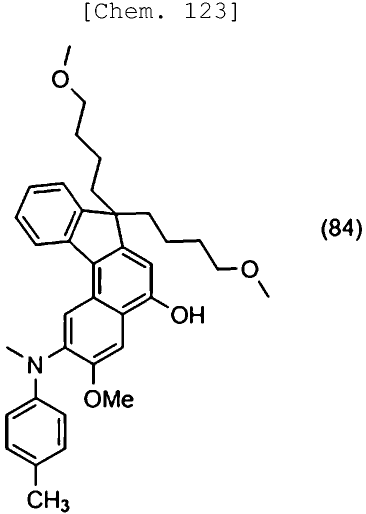

### Second Step

The same reaction as in the third step of Example 6 was performed except that the naphthol derivative represented by formula (84) above was used instead of the naphthol derivative represented by formula (45) above, to obtain a photochromic compound represented by formula (85) below with a yield of 84%.

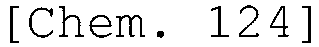

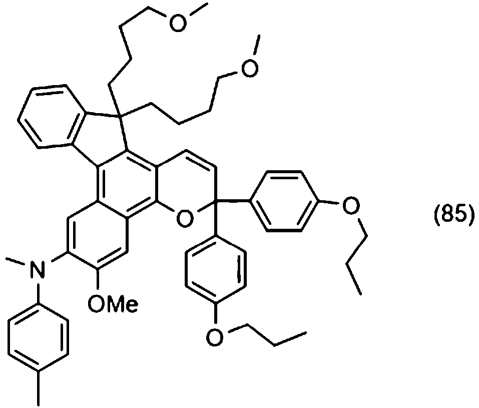

The photochromic compound represented by formula (85) above had elemental analysis values of C: 79.61%, H: 7.58%, and N: 1.61%, which were considerably consistent with the C₅₇H₆₅NO₆ calculated values of C: 79.59%, H: 7.62%, and N: 1.63%.

In a proton nuclear magnetic resonance spectroscopy, the photochromic compound represented by formula (85) showed 28H peaks attributed to a methyl group, a butyleneoxy group, and a propoxy group at around δ0.5 to 3.0 ppm, 17H peaks attributed to a methoxy group and a butyleneoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (85) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 21)

### First Step

The same reaction as in the first step of Example 3 was performed except that a propargyl alcohol represented by formula (86) below was used instead of the propargyl alcohol represented by formula (27) above, to obtain a photochromic compound represented by formula (87) below with a yield of 82%.

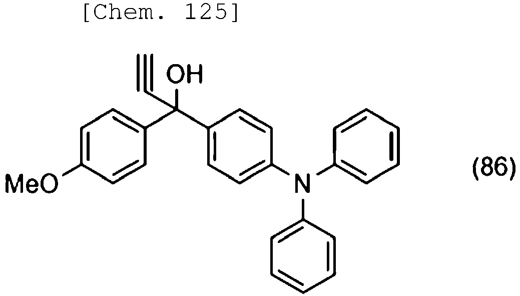

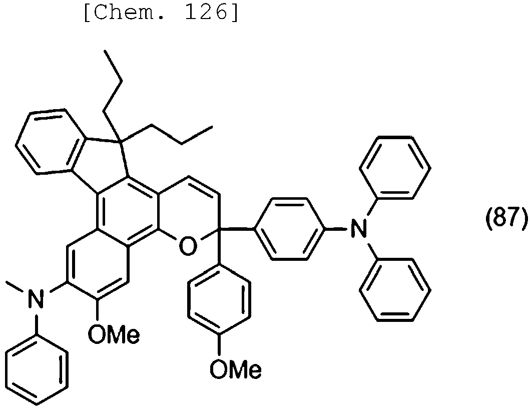

The photochromic compound represented by formula (87) above had elemental analysis values of C: 84.47%, H: 6.50%, and N: 3.33%, which were considerably consistent with the C₅₉H₅₄N₂O₃ calculated values of C: 84.45%, H: 6.49%, and N: 3.34%.

In a proton nuclear magnetic resonance spectroscopy, the photochromic compound represented by formula (87) showed 17H peaks attributed to a methyl group and a propyl group at around δ0.5 to 3.0 ppm, a 6H peak attributed to a methoxy group at around 63.0 to 5.0 ppm, and 31H peaks attributed to aromatic protons and protons of an alkene at around δ5.0 to 9.0 ppm.

Furthermore, in a ¹³C-nuclear magnetic resonance spectroscopy, the photochromic compound represented by formula (87) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 22)

### First Step

The same reaction as in the first step of Example 7 was performed except that a propargyl alcohol represented by formula (88) below was used instead of the propargyl alcohol represented by formula (49) above, to obtain a photochromic compound represented by formula (89) below with a yield of 86%.

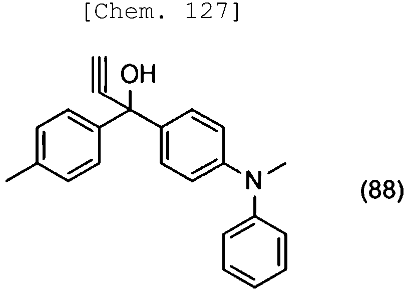

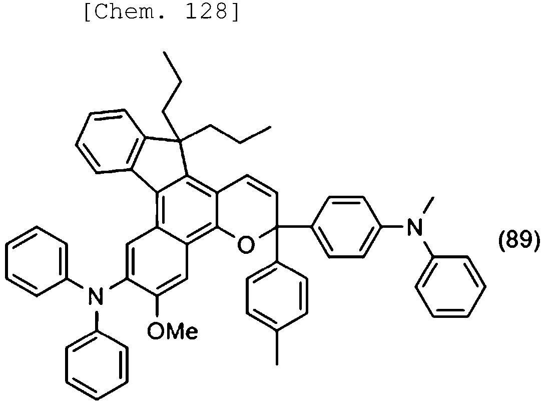

The photochromic compound represented by formula (89) above had elemental analysis values of C: 86.08%, H: 6.61%, and N: 3.41%, which were considerably consistent with the C₅₉H₅₄N₂O₂ calculated values of C: 86.10%, H: 6.61%, and N: 3.40%.

In a proton nuclear magnetic resonance spectroscopy, the photochromic compound represented by formula (89) showed 20H peaks attributed to a methyl group and a propyl group at around δ0.5 to 3.0 ppm, a 3H peak attributed to a methoxy group at around 63.0 to 5.0 ppm, and 31H peaks attributed to aromatic protons and protons of an alkene at around δ5.0 to 9.0 ppm.

Furthermore, in a ¹³C-nuclear magnetic resonance spectroscopy, the photochromic compound represented by formula (89) showed a peak attributed to carbon of an aromatic ring at around δ110 to 160 ppm, a peak attributed to carbon of an alkene at around 580 to 140 ppm, and a peak attributed to carbon of an alkyl at δ20 to 60 ppm.

### (Analysis Result of Naphthol Derivative)

The analysis results of the naphthol derivatives used in Examples 1 to 20 are summarized in Table 1.

### [Table 1]

**[table 1]**

| | Compound No. | Calculated value | | | Measured value | | | 1H-NMR |
|---|---|---|---|---|---|---|---|---|
| | | C | H | N | C | H | N | |
| Example 1 | Formula (28) | 82.00 | 6.37 | 3.54 | 81.96 | 6.39 | 3.55 | δ0.5-5.0ppm 12H |
| | | | | | | | | 65.0-9.0ppm 13H |
| Example 2 | Formula (30) | 84.00 | 5.95 | 3.06 | 84.02 | 5.96 | 3.05 | 50.5-5.0ppm 9H |
| | | | | | | | | 55.0-9.0ppm 18H |
| Example 3 | Formula (32) | 82.45 | 7.37 | 3.10 | 82.43 | 7.38 | 3.08 | δ0.5-5.0ppm 20H |
| | | | | | | | | 65.0-9.0ppm 13H |
| Example 4 | Formula (34) | 78.43 | 5.85 | 3.39 | 78.46 | 5.81 | 3.42 | δ0.5-5.0ppm 12H |
| | | | | | | | | 65.0-9.0ppm 12H |
| Example 5 | Formula (43) | 80.27 | 7.89 | 2.67 | 80.25 | 7.91 | 2.70 | δ0.5-5.0ppm 28H |
| | | | | | | | | δ5.0-9.0ppm 13H |
| Example 6 | Formula (47) | 80.44 | 7.35 | 2.67 | 80.47 | 7.36 | 2.64 | δ0.5-5.0ppm 24H |
| | | | | | | | | δ5.0-9.0ppm 13H |
| Example 7 | Formula (50) | 84.18 | 6.87 | 2.73 | 84.22 | 6.85 | 2.70 | δ0.5-5.0ppm 17H |
| | | | | | | | | 65.0-9.0ppm 18H |
| Example 8 | Formula (54) | 68.89 | 5.62 | 2.23 | 68.91 | 5.60 | 2.25 | δ0.5-5.0ppm 24H |
| | | | | | | | | δ5.0-9.0ppm 11H |
| Example 9 | Formula (57) | 75.11 | 6.48 | 2.50 | 75.13 | 6.45 | 2.49 | δ0.5-5.0ppm 24H |
| | | | | | | | | 65.0-9.0ppm 12H |
| Example 10 | Formula (59) | 80.14 | 6.94 | 2.92 | 80.17 | 6.93 | 2.90 | δ0.5-5.0ppm 21H |
| | | | | | | | | 55.0-9.0ppm 12H |
| Example 11 | Formula (63) | 68.37 | 5.60 | 1.90 | 68.33 | 5.64 | 1.93 | δ0.5-5.0ppm 25H |
| | | | | | | | | 55.0-9.0ppm 16H |
| Example 12 | Formula (66) | 79.00 | 6.46 | 2.42 | 79.02 | 6.48 | 2.44 | δ0.5-5.0ppm 21H |
| | | | | | | | | 55.0-9.0ppm 16H |
| Example 13 | Formula (68) | 84.21 | 7.07 | 2.65 | 84.24 | 7.05 | 2.65 | δ0.5-5.0ppm 20H |
| | | | | | | | | 65.0-9.0ppm 17H |
| Example 14 | Formula (71) | 76.60 | 7.45 | 2.03 | 76.63 | 7.44 | 2.00 | δ0.5-5.0ppm 35H |
| | | | | | | | | 65.0-9.0ppm 16H |
| Example 15 | Formula (75) | 82.38 | 7.25 | 2.34 | 82.41 | 7.27 | 2.31 | δ0.5-5.0ppm 27H |
| | | | | | | | | 65.0-9.0ppm 16H |
| Example 16 | Formula (77) | 76.10 | 7.73 | 2.34 | 76.12 | 7.71 | 2.36 | δ0.5-5.0ppm 34H |
| | | | | | | | | δ5.0-9.0ppm 12H |
| Example 17 | Formula (79) | 80.41 | 8.06 | 2.60 | 80.39 | 8.09 | 2.58 | δ0.5-5.0ppm 31H |
| | | | | | | | | δ5.0-9.0ppm 12H |
| Example 18 | Formula (81) | 82.54 | 7.58 | 3.01 | 82.51 | 7.60 | 3.00 | δ0.5-5.0ppm 23H |
| | | | | | | | | δ5.0-9.0ppm 12H |
| Example 19 | Formula (83) | 79.41 | 5.95 | 3.31 | 79.43 | 5.92 | 3.30 | δ0.5-5.0ppm 13H |
| | | | | | | | | 65.0-9.0ppm 12H |
| Example 20 | Formula (85) | 78.09 | 7.83 | 2.53 | 78.11 | 7.82 | 2.55 | δ0.5-5.0ppm 31H |
| | | | | | | | | 65.0-9.0ppm 12H |

### (Physical Property Evaluation for Photochromic Plastic Lens Prepared by Coating Method)

### (Example 23)

### (Preparation of Curable Composition)

First, the photochromic compound obtained in Example 1, a photoinitiator, and a polymerizable compound were mixed to obtain a curable composition.

As the polymerizable compound, a combination of the following radical polymerizable monomers was used.
Polyethylene glycol dimethacrylate (average molecular weight: 736): 42 parts by mass
Polyethylene glycol dimethacrylate (average molecular weight: 536): 12 parts by mass
Trimethylolpropane trimethacrylate: 38 parts by mass γ-methacryloyloxypropyl trimethoxysilane: 2 parts by mass Glycidyl methacrylate: 1 part by mass

Note that the photochromic compound was added to the curable composition so that the amount of the photochromic compound was 0.25 mmol based on 100 g of the total amount of the radical-polymerizable monomers.

The following additives were used.
Phenylbis(2,4,6-trimethylbenzoyl)phosphine oxide (photoinitiator: Omnirad 819): 0.3 parts by mass Ethylene bis(oxyethylene) bis[3-(5-tert-butyl-4-hydroxy-m-tolyl)propionate] (stabilizer, Irganox 245): 1 part by mass Bis(1,2,2,6,6-pentamethyl-4-piperidyl) sebacate: 3 parts by mass
Leveling agent (L7001) manufactured by Dow Corning Toray Co., Ltd.: 0.1 part by mass

Note that each compounding ratio of the above additives is based on 100 parts by mass of the total amount of the radical-polymerizable monomers.

### (Production of Optical Article)

This curable composition was subjected to a lamination method using a polymerization to obtain a photochromic laminate, as described below.

First, a thiourethane-based plastic lens having a central thickness of 2 mm and a refractive index of 1.60 was prepared as the optical substrate. Note that this thiourethane-based plastic lens was pre-treated by alkaline etching with a 10% sodium hydroxide aqueous solution at 50°C for 5 minutes, followed by sufficient washing with distilled water.

A surface of the above-described plastic lens was coated with a moisture-curable primer (product name: TR-SC-P, manufactured by Tokuyama Corporation) at a rotation frequency of 70 rpm for 15 seconds, followed by 1000 rpm for 10 seconds, using a spin coater (1H-DX2, manufactured by MIKASA CO., LTD), Subsequently, about 2 g of the photochromic curable composition obtained above was spin-coated at a rotation frequency of 60 rpm for 40 seconds, followed by 600 rpm for 10 to 20 seconds, to form a photochromic coating layer with a film thickness of 40 µm.

The lens with the surface coated with the photochromic curable composition (photochromic coating layer) was irradiated with light using a metal halide lamp with an output power of 200 mW/cm² in a nitrogen gas atmosphere for 90 seconds to cure the coating film. Subsequently, the lens was further heated at 110°C for 1 hour to prepare a photochromic laminate having a photochromic layer.

### (Examples 24 to 44)

In the same manner as in Example 23, photochromic laminates were prepared using the photochromic compounds obtained in Examples 2 to 22.

### (Comparative Examples 1 to 8)

In the same manner as in Example 23, each photochromic laminate was obtained using each of the photochromic compounds represented by formulas (A) to (H) below.

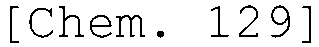

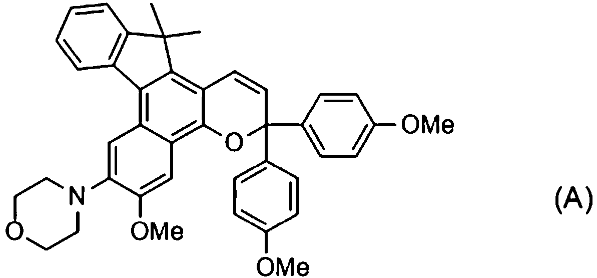

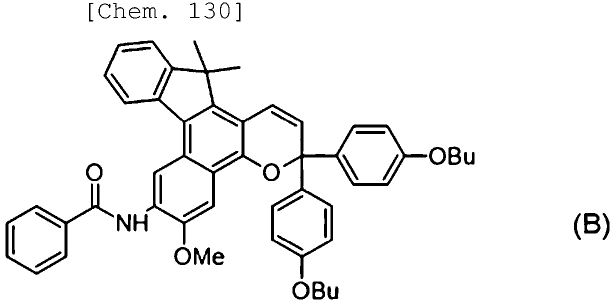

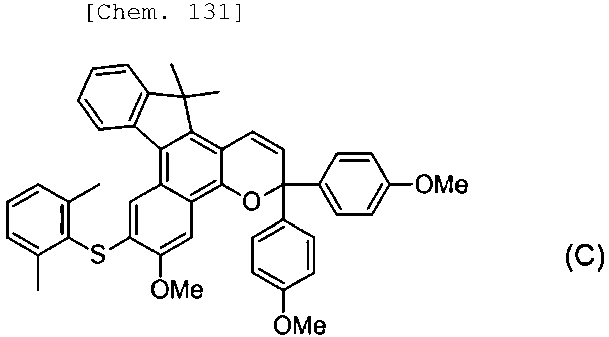

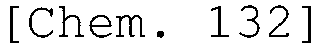

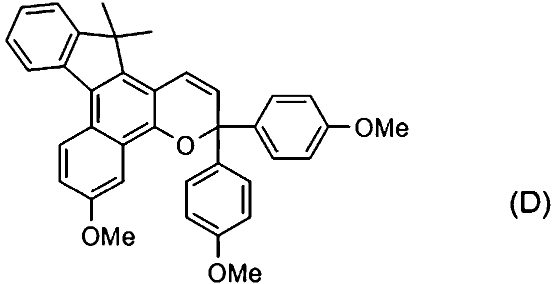

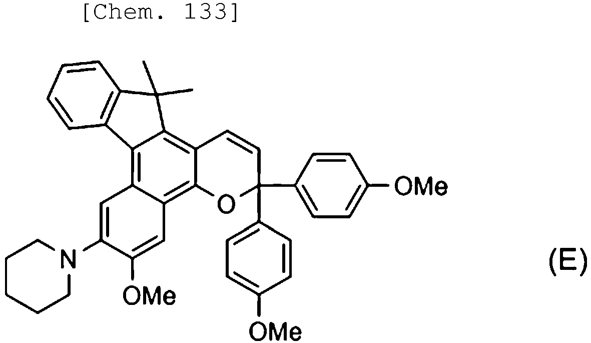

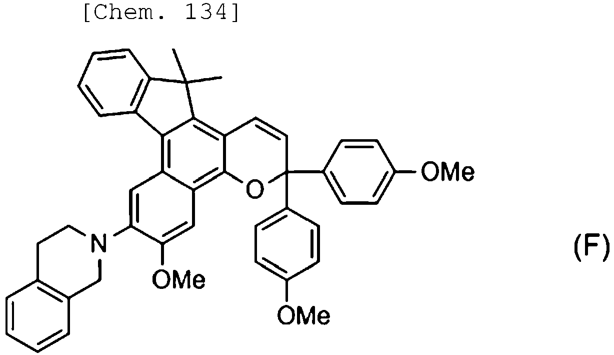

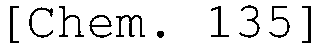

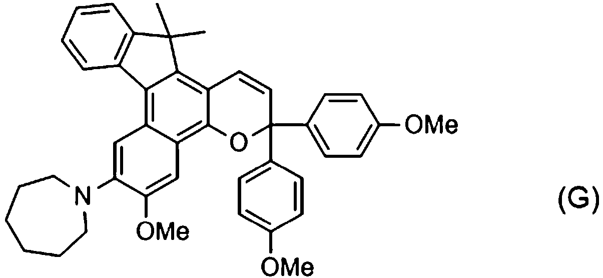

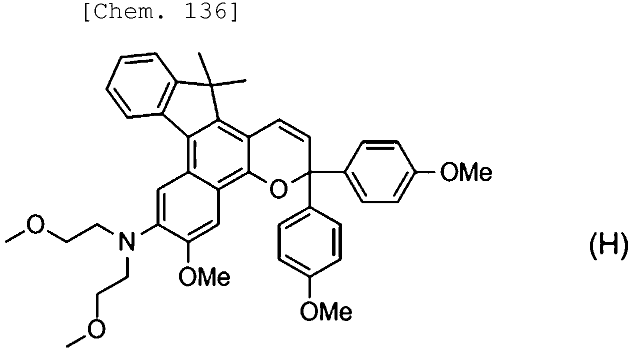

### (Synthesis of Compound E)

The same reaction as in Example 11 was performed except that iodomethane was used instead of 1-bromo-4-methoxybutane, to obtain a benzyl body represented by formula (90) below with a yield of 86%.

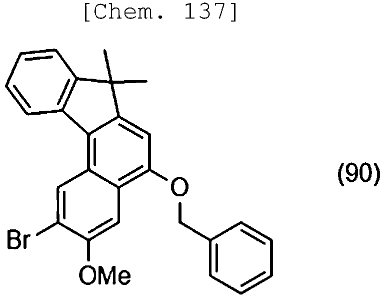

The same reaction as in Example 11 was performed except that the benzyl body represented by formula (90) above was used instead of the benzyl body represented by formula (60) above, piperidine was used instead of bis(4-trifluoromethylphenyl)amine, and the compound represented by formula (27) above was used instead of the compound represented by formula (62) above, to synthesize a compound (E) .

### (Synthesis of Compound F)

The same reaction as in the synthesis of the compound E was performed except that 1,2,3,4-tetrahydroisoquinoline was used instead of piperidine, to synthesize a compound (F).

### (Synthesis of Compound G)

The same reaction as in the synthesis of the compound E was performed except that hexamethyleneimine was used instead of piperidine, to synthesize a compound (G).

### (Synthesis of Compound H)

The same reaction as in the synthesis of the compound E was performed except that bis(2-methoxyethyl)amine was used instead of piperidine, to synthesize a compound (G).

### <Evaluation Method>

The obtained photochromic laminates were evaluated by a method described below.

### (1) Photochromic Properties

[1] Maximum Absorption Wavelength (λmax):
   The maximum absorption wavelength after color development, measured using a spectrophotometer (instantaneous multichannel photodetector MCPD3000) manufactured by Otsuka Electronics Co., Ltd. was used as an indicator for the color tone during the color development.
[2] 23°C Color Optical Density (A₂₃):
   A difference between an absorbancy measured after light irradiation at 23°C for 240 seconds {ε(240)} and an absorbancy without light irradiation ε(0) at the maximum absorption wavelength was used as an indicator for the color optical density. It is considered that, the higher this value, the superior the photochromic property.
[3] 35°C Color Optical Density (A₃₅):
   A difference between an absorbancy measured after light irradiation at 35°C for 240 seconds {ε(240)} and an absorbancy without light irradiation ε(0) at the first and second absorption wavelengths was used as an indicator for the color optical density. It is considered that, the higher this value, the superior the photochromic property.
[4] High-Temperature Color Development Rate (A₃₅/A₂₃×100): 35°C color optical density (A₃₅) /23°C color optical density (A₂₃)×100 (%)
   It is considered that, the higher this value, the smaller the difference between the color optical densities at high and low temperatures, indicating that a temperature dependency is low.
[5] 23°C color fading half-life [τ1/2 (sec.)]:
   A time required for the absorbancy of the sample at the maximum absorption wavelength to decrease to 1/2 of {ε(240)-ε(0)} when the light irradiation is terminated after the light irradiation at 23°C for 240 seconds, was used as an indicator for the color fading speed. The shorter this time, the higher the color fading speed.
[6] Residual rate (A₉₀/A₀×100): The obtained photochromic plastic lens was subjected to accelerated deterioration using a xenon weather meter X25 manufactured by Suga Test Instruments Co., Ltd. for 90 hours. Subsequently, the color optical density was evaluated before and after the test, then a color optical density before the test (A₀) and a color optical density after the test (A₉₀) were measured, and a ratio (A₉₀/A₀) was defined as a residual rate. The residual rate was used as an indicator for a color developing durability. The higher the residual rate, the higher the color developing durability.

The results of Examples 23 to 33 are summarized in Table 2, the results of Examples 34 to 44 are summarized in Table 3, and the results of Comparative Examples 1 to 8 are summarized in Table 4.

### [Table 2]

**[table 2]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 23 | Example 1 Formula (28) | 501 | 1.15 | 0.79 | 69% | 90 | 83% |
| Example 24 | Example 2 Formula (30) | 510 | 1.49 | 1.05 | 70% | 96 | 82% |
| Example 25 | Example 3 Formula (32) | 498 | 1.0897 | 0.74 | 68% | 67 | 84% |
| Example 26 | Example 4 Formula (34) | 496 | 1.18 | 0.78 | 66% | 90 | 80% |
| Example 27 | Example 5 Formula (43) | 442 | 1.19 | 0.79 | 66% | 90 | 73% |
| Example 28 | Example 6 Formula (47) | 499 | 1.34 | 0.95 | 71% | 116 | 80% |
| Example 29 | Example 7 Formula (50) | 522 | 1.19 | 0.74 | 62% | 44 | 77% |
| | | 615 | 1.14 | 0.71 | | 44 | 76% |
| Example 30 | Example 8 Formula (54) | 545 | 1.18 | 0.75 | 64% | 54 | 75% |
| Example 31 | Example 9 Formula (57) | 451 | 0.73 | 0.47 | 64% | 75 | 83% |
| | | 566 | 0.86 | 0.55 | | 75 | 84% |
| Example 32 | Example 10 Formula (59) | 510 | 1.3 | 0.86 | 66% | 70 | 82% |
| Example 33 | Example 11 Formula (63) | 462 | 0.75 | 0.46 | 61% | 40 | 83% |
| | | 576 | 0.66 | 0.41 | | 40 | 83% |

### [Table 3]

**[table 3]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 34 | Example 12 Formula (66) | 501 | 1.38 | 0.92 | 67% | 76 | 81% |
| Example 35 | Example 13 Formula (68) | 512 | 1.57 | 1.08 | 69% | 79 | 83% |
| Example 36 | Example 14 Formula (71) | 521 | 1.89 | 1.33 | 70% | 105 | 82% |
| Example 37 | Example 15 Formula (75) | 523 | 1.20 | 0.74 | 62% | 42 | 75% |
| Example 38 | Example 16 Formula (77) | 498 | 1.25 | 0.84 | 67% | 72 | 80% |
| Example 39 | Example 17 Formula (79) | 558 | 1.06 | 0.71 | 67% | 65 | 78% |
| Example 40 | Example 18 Formula (81) | 493 | 1.25 | 0.88 | 70% | 98 | 78% |
| Example 41 | Example 19 Formula (83) | 515 | 1.34 | 0.90 | 67% | 97 | 77% |
| Example 42 | Example 20 Formula (85) | 503 | 1.22 | 0.85 | 70% | 81 | 81% |
| Example 43 | Example 21 Formula (87) | 524 | 1.26 | 0.87 | 69% | 97 | 80% |
| Example 44 | Example 22 Formula (89) | 530 | 1.51 | 1.03 | 68% | 75 | 76% |

### [Table 4]

**[table 4]**

| | Compound No. | Maximum absorption wavelength (nm) | 23°C color optical density (-) | 35°C color optical density (-) | Temperature change rate (%) | 23°C color fading half-life (sec) | Residual rate (%) |
|---|---|---|---|---|---|---|---|
| Comparative Example 1 | Formula (A) | 474 | 1.65 | 1.14 | 69% | 170 | 53% |
| | | 567 | 0.83 | 0.57 | | 172 | 57% |
| Comparative Example 2 | Formula (B) | 463 | 1.30 | 0.88 | 68% | 142 | 68% |
| | | 573 | 0.90 | 0.62 | | 145 | 68% |
| Comparative Example 3 | Formula (C) | 469 | 1.20 | 0.78 | 65% | 115 | 73% |
| | | 570 | 0.77 | 0.50 | | 117 | 73% |
| Comparative Example 4 | Formula (D) | 449 | 0.69 | 0.44 | 63% | 95 | 74% |
| | | 569 | 0.87 | 0.55 | | 95 | 73% |
| Comparative Example 5 | Formula (E) | 481 | 1.81 | 1.28 | 71% | 194 | 51% |
| Comparative Example 6 | Formula (F) | 481 | 1.81 | 1.28 | 71% | 191 | 50% |
| Comparative Example 7 | Formula (G) | 493 | 2.24 | 1.89 | 84% | 345 | 38% |
| Comparative Example 8 | Formula (H) | 497 | 2.21 | 1.72 | 78% | 262 | 45% |

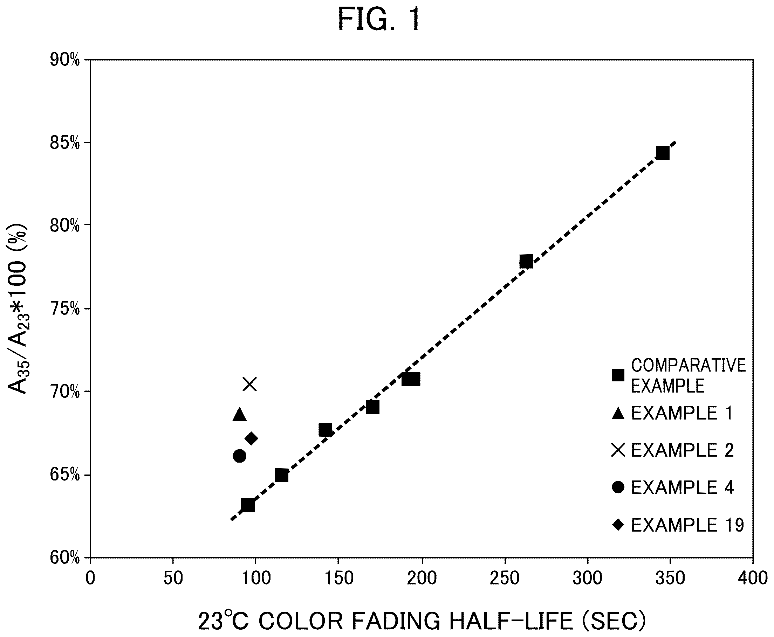

FIG. 1 is a graph presenting an example of a relationship between a color fading half-life at 23°C and a high-temperature color development rate of each photochromic laminate according to Examples and Comparative Examples. FIG. 1 is based on data from Example 23 using the photochromic compound in Example 1, Example 24 using the photochromic compound in Example 2, Example 26 using the photochromic compound in Example 4, Example 41 using the photochromic compound in Example 19, and Comparative Examples 1 to 8. In FIG. 1, the plots of the Comparative Examples correspond to, from the left, Comparative Example 4, Comparative Example 3, Comparative Example 2, Comparative Example 1, Comparative Example 6, Comparative Example 5, Comparative Example 8, and Comparative Example 7 respectively.

### (Example 45)

### First Step

By referring to the method described in Patent Document 6, the same reaction as in the first step of Example 5 was performed except that a benzophenone compound represented by formula (91) below, synthesized by reacting 3-bromoanisole with 4'-(1,1-dimethylethyl) [1,1'-biphenyl]-4-carbonyl chloride, was used instead of 3-bromo-4-methylbenzophenone, and the same reaction as in the fourth step of Example 5 was performed except that iodoethane was used instead of 1-bromo-4-methoxybutane, to obtain a naphthol compound represented by formula (92) below with a yield of 79%.

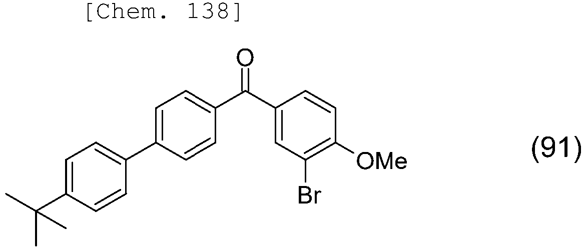

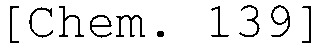

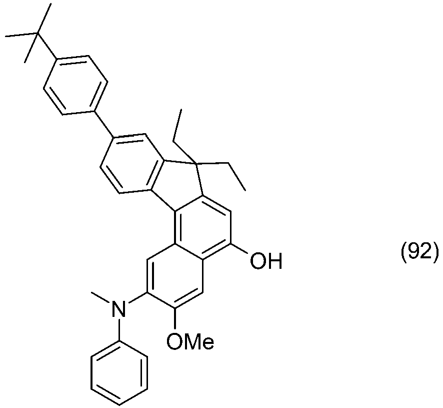

### Second Step

The same reaction as in the fifth step of Example 1 was performed except that the naphthol compound represented by formula (92) above was used instead of the naphthol compound represented by formula (26) above and a propargyl alcohol represented by formula (93) below was used instead of the propargyl alcohol represented by formula (27) above, to obtain a photochromic compound represented by formula (94) below with a yield of 69%.

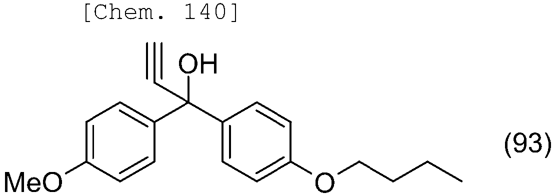

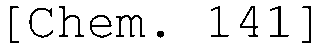

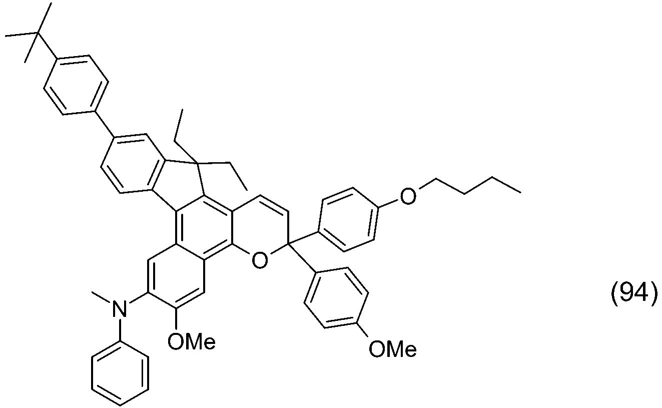

The photochromic compound represented by formula (94) above had elemental analysis values of C: 83.57%, H: 7.22%, and N: 1.63%, which were considerably consistent with the C₅₉H₆₁NO₄ calculated values of C: 83.55%, H: 7.25%, and N: 1.65%.

In a proton nuclear magnetic resonance spectroscopy, the photochromic compound represented by formula (94) showed 29H peaks attributed to a t-butyl group, a butyloxy group, an ethyl group, and a methyl group at around 50.5 to 3.0 ppm, 8H peaks attributed to a methoxy group and a butyloxy group at around δ3.0 to 5.0 ppm, and 24H peaks attributed to aromatic protons and protons of an alkene at around δ5.0 to 9.0 ppm.

Furthermore, in a ¹³C-nuclear magnetic resonance spectroscopy, the photochromic compound represented by formula (94) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 46)

### First Step

The same reaction as in the first step of Example 5 was performed except that a benzophenone represented by formula (95) below was used instead of 3-bromo-4-methylbenzophenone and iodobutane was used instead of 1-bromo-4-methoxybutane, to obtain a benzyl compound represented by formula (96) below with a yield of 93%.

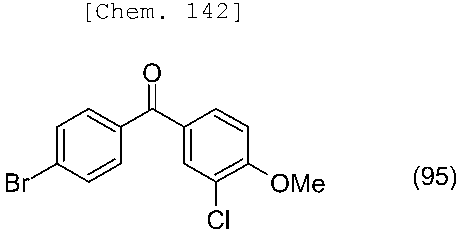

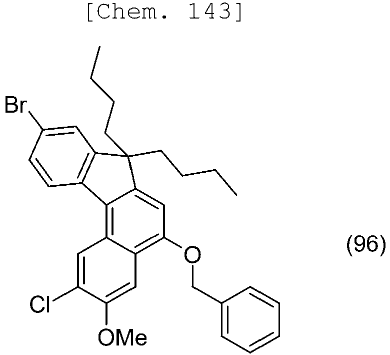

### Second Step

Into 230 mL of THF, 13.3 g (23.0 mmol) of the benzyl compound represented by formula (96) above was dissolved, and the mixture was cooled to -78°C, into which 15.8 mL of n-BuLi (1.6 mol/L hexane solution) was slowly dripped while maintaining the temperature at -70°C or lower. After confirming that the raw materials had been consumed, 4.57 g (30.3 mmol) of t-butyl dimethylchlorosilane dissolved in 18 mL of THF was slowly dripped into the mixture while maintaining the temperature at -70°C or lower. After the dripping, the temperature was slowly raised to room temperature. After completion of the reaction, 200 mL of water and 200 mL of toluene were added to the mixture, which was subjected to liquid separation. From the obtained organic layer, the solvent was removed, and then the organic layer was purified by a chromatography on silica gel to obtain a benzyl compound represented by formula (97) below with a yield of 54%.

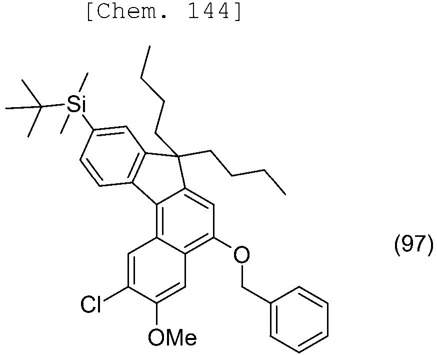

### Third Step

The same reaction as in the first step of Example 1 was performed except that the compound represented by formula (97) above was used instead of the compound represented by formula (22) above and N-ethylaniline was used instead of N-methylaniline, to obtain a naphthol compound represented by formula (98) below with a yield of 47%.

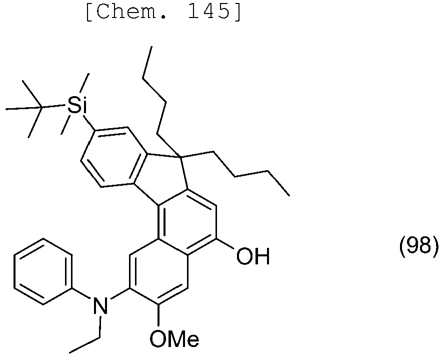

### Fourth Step

The same reaction as in the third step of Example 11 was performed except that the naphthol compound represented by formula (98) above was used instead of the naphthol compound represented by formula (61) above, to obtain a photochromic compound represented by formula (99) below with a yield of 73%.

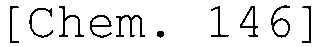

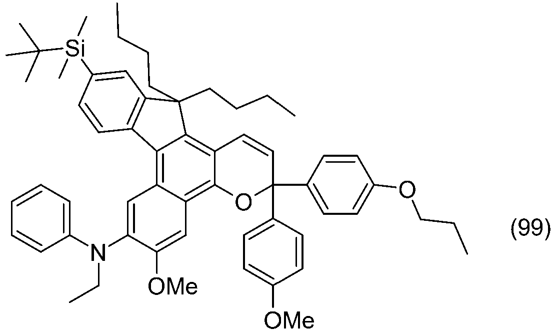

The photochromic compound represented by formula (99) above had elemental analysis values of C: 79.96%, H: 8.05%, and N: 1.60%, which were considerably consistent with the C₅₉H₇₁NO₄Si calculated values of C: 79.96%, H: 8.07%, and N: 1.58%.

In a proton nuclear magnetic resonance spectroscopy, the photochromic compound represented by formula (99) showed 43H peaks attributed to a t-butyl group, a butyl group, an ethyl group, a methyl group, and a propyloxy group at around δ0.5 to 3.0 ppm, 8H peaks attributed to a methoxy group and a propyloxy group at around 63.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (99) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 47)

### First Step

The same reaction as in the first step of Example 5 was performed except that 3-bromo-4-methyl-4'-methoxybenzophenone was used instead of 3-bromo-4-methylbenzophenone, to obtain a benzyl compound represented by formula (100) below with a yield of 86%.

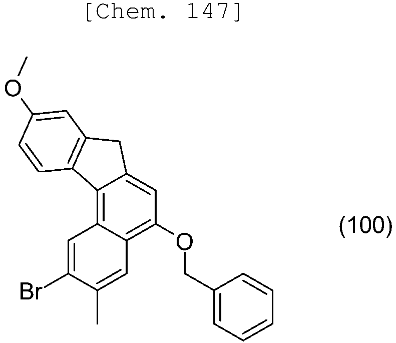

### Second Step

The same reaction as in the second step of Example 8 was performed except that 3,4-dihydro-2H-1,4-benzoxazine was used instead of 1,2,3,4-tetrahydroquinoline, and the same reaction as in the third step of Example 8 was performed except that 1-fluoro-4-iodobutane was used instead of 4-bromo-1,1,1-trifluorobutane, to obtain a naphthol compound represented by formula (101) below with a yield of 68%.

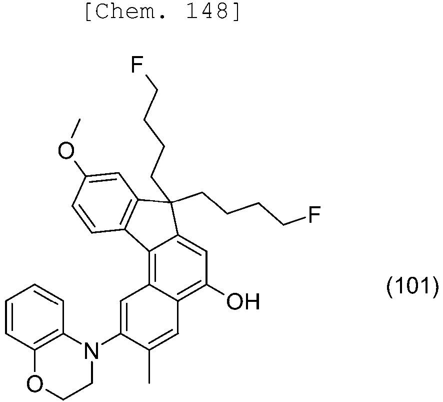

### Third Step

The same reaction as in the sixth step of Example 7 was performed except that the naphthol compound represented by formula (101) above was used instead of the naphthol compound represented by formula (48) above, to obtain a photochromic compound represented by formula (102) below with a yield of 69%.

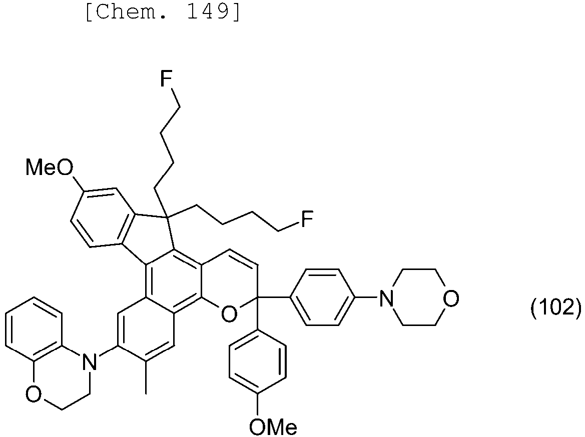

The photochromic compound represented by formula (102) above had elemental analysis values of C: 76.52%, H: 6.51%, and N: 3.23%, which were considerably consistent with the C₅₅H₅₆F₂N₂O₅ calculated values of C: 76.54%, H: 6.54%, and N: 3.25%.

In a proton nuclear magnetic resonance spectroscopy, the photochromic compound represented by formula (102) showed 25H peaks attributed to a methyl group, a fluorobutyl group, a morpholino group, and a benzoxazoline ring at around δ0.5 to 3.0 ppm, 12H peaks attributed to a methoxy group, a morpholino group, and a benzoxazoline ring at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (102) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 48)

### First Step

The same reaction as in the first step of Example 46 was performed except that 1-bromo-2-methoxyethane was used instead of iodobutane, to obtain a benzyl compound represented by formula (103) below.

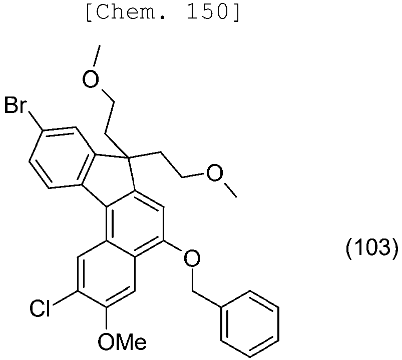

### Second Step

To 5.8 g (10.0 mmol) of the carboxylic acid compound represented by formula (103) above, 2.1 g (10.9 mmol) of p-(trifluoromethyl)phenylboronic acid, 4.5 g (21.7 mmol) of sodium carbonate, 20.5 mL of water, 30.5 mL of 1,2-dimethoxyethane, and 2.2 mL of ethanol were added, and stirred while carrying out nitrogen bubbling. The nitrogen bubbling was continued for about 20 minutes, then 28.5 mg (0.02 mmol) of Pd(PPh₃)₄ was added to the mixture, which was reacted at 75°C for 2 hours. After the reaction, the mixture was cooled to room temperature, to which 130 mL of THF was added, and the mixture was cooled to 0 to 5°C, then adjusted to pH1 by adding concentrated hydrochloric acid, and subjected to liquid separation. The mixture was washed twice with 100 mL of water to remove the solvent. The mixture was purified by reslurry with 100 mL of methanol to obtain a benzyl compound represented by formula (104) below with a yield of 87%.

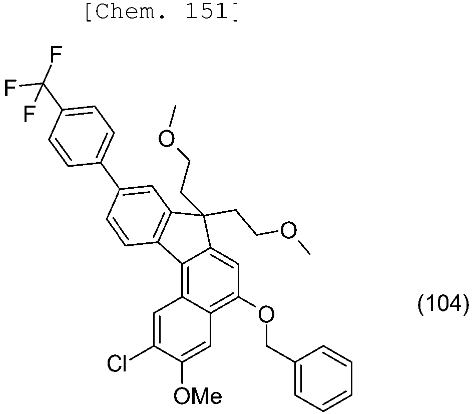

### Third Step

The same reaction as in the third step of Example 46 was performed except that the compound represented by formula (104) above was used instead of the compound represented by formula (97) above and 7-methyl-3,4-dihydro-2H-1,4-benzoxazine was used instead of N-ethylaniline, to obtain a naphthol compound represented by formula (105) below with a yield of 59%.

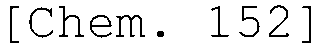

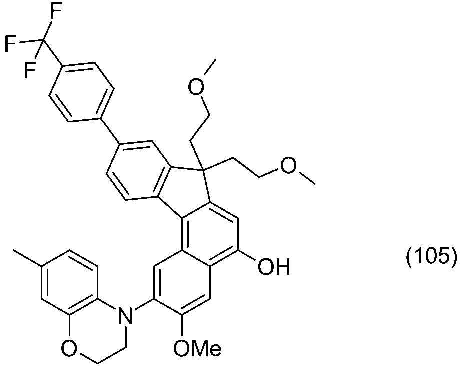

### Fourth Step

The same reaction as in the third step of Example 6 was performed except that the naphthol compound represented by formula (105) above was used instead of the naphthol compound represented by formula (45) above to obtain a photochromic compound represented by formula (106) below with a yield of 72%.

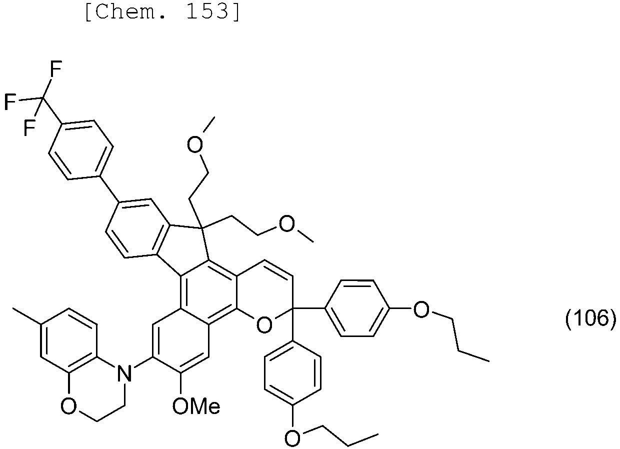

The photochromic compound represented by formula (106) above had elemental analysis values of C: 75.03%, H: 6.18%, and N: 1.42%, which were considerably consistent with the C₆₁H₆₀F₃NO₇ calculated values of C: 75.06%, H: 6.20%, and N: 1.43%.

In a proton nuclear magnetic resonance spectroscopy, the photochromic compound represented by formula (106) showed 19H peaks attributed to a methyl group, a propyloxy group, an ethylmethoxy group, and a benzoxazoline ring at around δ0.5 to 3.0 ppm, 19H peaks attributed to a methoxy group, a propyloxy group, an ethylmethoxy group, and a benzoxazoline ring at around δ3.0 to 5.0 ppm, and 22H peaks attributed to aromatic protons and protons of an alkene at around δ5.0 to 9.0 ppm.

Furthermore, in a ¹³C-nuclear magnetic resonance spectroscopy, the photochromic compound represented by formula (106) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 49)

### First Step

The same reaction as in the first step of Example 1 was performed except that 7-fluoro-3,4-dihydro-2H-1,4-benzoxazine was used instead of N-methylaniline, and the same reaction as in the third step of Example 1 was performed except that 4,4-diethylcyclohexanone was used instead of acetone, to obtain a naphthol compound represented by formula (107) below with a yield of 72%.

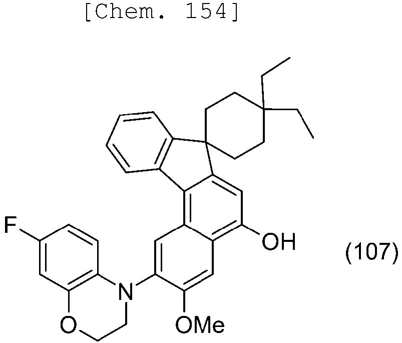

### Second Step

The same reaction as in the fifth step of Example 1 was performed except that the naphthol compound represented by formula (107) above was used instead of the naphthol compound represented by formula (26) above and a propargyl alcohol represented by formula (108) below was used instead of the propargyl alcohol represented by formula (27) above, to obtain a photochromic compound represented by formula (109) below with a yield of 65%.

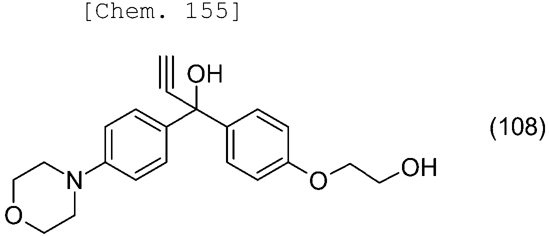

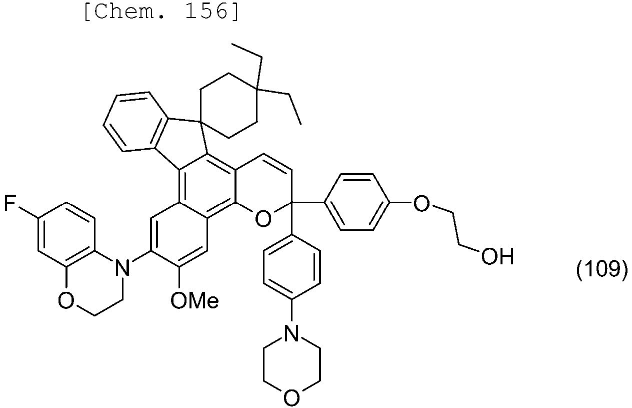

### Third Step

By referring to the method described in Patent Document 9, a compound represented by formula (110) below was reacted with the photochromic compound represented by formula (109) above, to obtain a photochromic compound represented by formula (111) below with a yield of 73%.

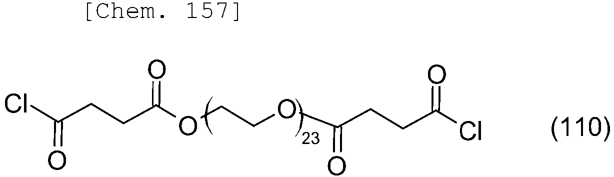

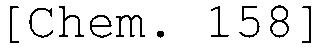

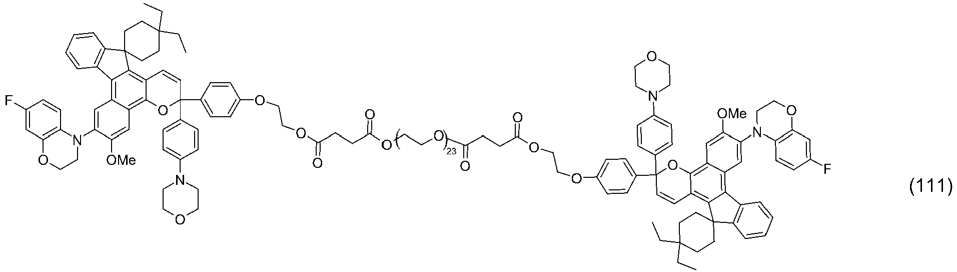

In a proton nuclear magnetic resonance spectroscopy, the photochromic compound represented by formula (111) above showed 56H peaks attributed to an ethyl group, a succinic acid moiety, a morpholino group, and a benzoxazoline ring at around δ0.5 to 3.0 ppm, about 118H peaks attributed to a methoxy group, an ethyleneoxy group, a polyethylene glycol chain, a morpholino group, and a benzoxazoline ring at around δ3.0 to 5.0 ppm, and 38H peaks attributed to aromatic protons and protons of an alkene at around δ5.0 to 9.0 ppm.

### (Example 50)

### First Step

By referring to the method described in Patent Document 6, the same reaction as in the first step of Example 1 was performed except that 3-bromo-4-methoxy-4'-phenylbenzophenone obtained by reacting 3-bromoanisole and 4-phenylbenzoyl chloride was used instead of 3-bromo-4-methoxybenzophenone and bis(4-trifluoromethylphenyl)amine was used instead of N-methylaniline, and the same reaction as in the third step of Example 1 was performed except that spiro[5,5]undecan-3-one was used instead of acetone, to obtain a naphthol compound represented by formula (112) below with a yield of 67%.

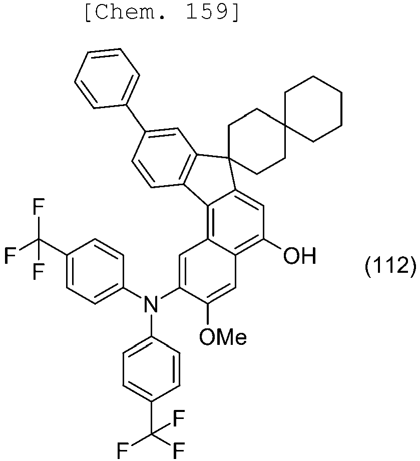

### Second Step

The same reaction as in the fifth step of Example 1 was performed except that the naphthol compound represented by formula (112) above was used instead of the naphthol compound represented by formula (26) above and a propargyl alcohol represented by formula (113) below was used instead of the propargyl alcohol represented by formula (27) above, to obtain a photochromic compound represented by formula (114) below with a yield of 68%.

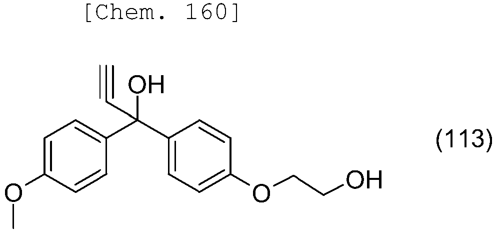

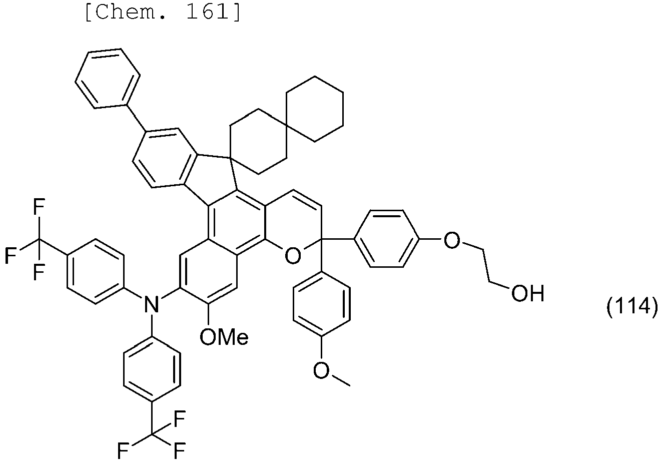

### Third Step

The same reaction as in the third step of Example 49 was performed except that a polypropylene glycol monobutyl ether having a number-average molecular weight of 1000 was used instead of the polyethylene glycol having a number-average molecular weight of 1000, to obtain a photochromic compound represented by formula (115) below with a yield of 73%.

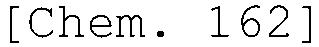

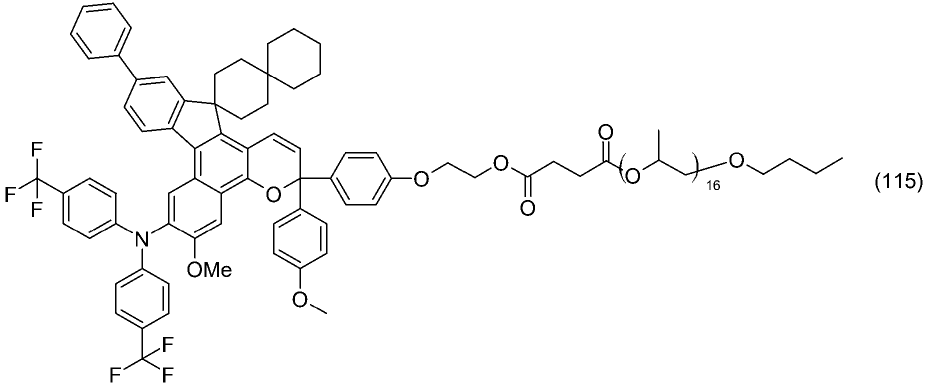

In a proton nuclear magnetic resonance spectroscopy, the photochromic compound represented by formula (115) above showed about 77H peaks attributed to a spiro[5,5]undecane ring, a succinic acid moiety, a polypropylene glycol chain, and a butoxy group at around δ0.5 to 3.0 ppm, about 60H peaks attributed to a methoxy group, an ethyleneoxy group, a polypropylene glycol chain, and a butoxy group at around δ3.0 to 5.0 ppm, and 28H peaks attributed to aromatic protons and protons of an alkene at around 65.0 to 9.0 ppm.

### (Example 51)

### First Step

The same reaction as in the first step of Example 46 was performed except that 4-trifluoromethoxybenzoyl chloride was used instead of 4'-(1,1-dimethylethyl) [1,1'-biphenyl]-4-carbonyl chloride and N-butylaniline was used instead of N-methylaniline, to obtain a naphthol compound represented by formula (116) below with a yield of 70%.

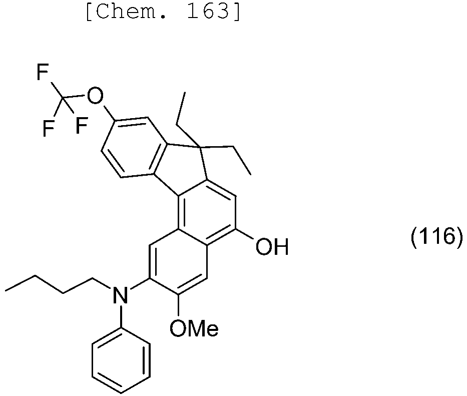

### Second Step

The same reaction as in the third step of Example 11 was performed except that the naphthol compound represented by formula (116) above was used instead of the naphthol compound represented by formula (61) above, to obtain a photochromic compound represented by formula (117) below with a yield of 73%.

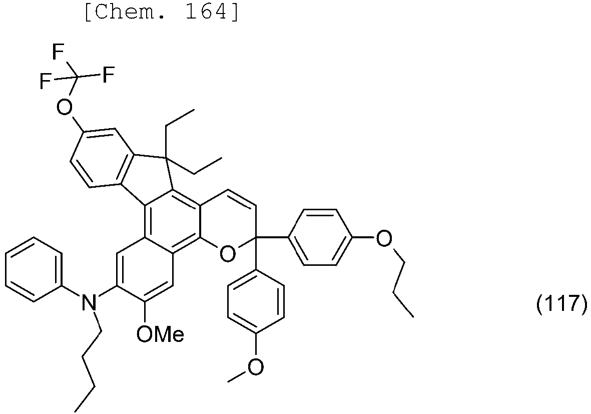

The photochromic compound represented by formula (117) above had elemental analysis values of C: 75.41%, H: 6.30%, and N: 1.68%, which were considerably consistent with the C₅₂H₅₂F₃NO₅ calculated values of C: 75.43%, H: 6.33%, and N: 1.69%.

In a proton nuclear magnetic resonance spectroscopy, the photochromic compound represented by formula (117) showed 24H peaks attributed to an ethyl group, a butyl group, and a propyloxy group at around δ0.5 to 3.0 ppm, 8H peaks attributed to a methoxy group and a propyloxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (117) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 52)

### First Step

The same reaction as in the first step of Example 51 was performed except that 4-phenoxybenzoyl chloride was used instead of 4-trifluoromethoxybenzoyl chloride, (4-methylphenyl)phenylamine was used instead of N-butylaniline, and iodohexane was used instead of iodoethane, to obtain a naphthol compound represented by formula (118) below with a yield of 64%.

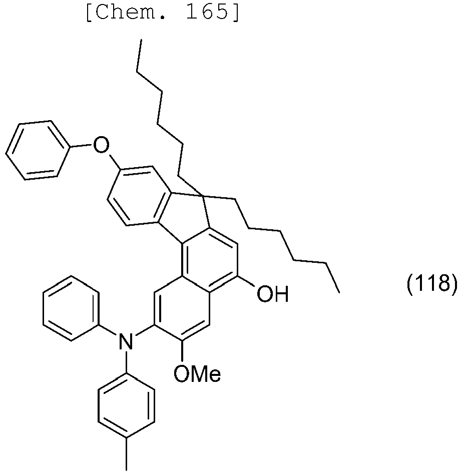

### Second Step

The same reaction as in the second step of Example 45 was performed except that the naphthol compound represented by formula (118) above was used instead of the naphthol compound represented by formula (92) above, to obtain a photochromic compound represented by formula (119) below with a yield of 71%.

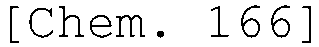

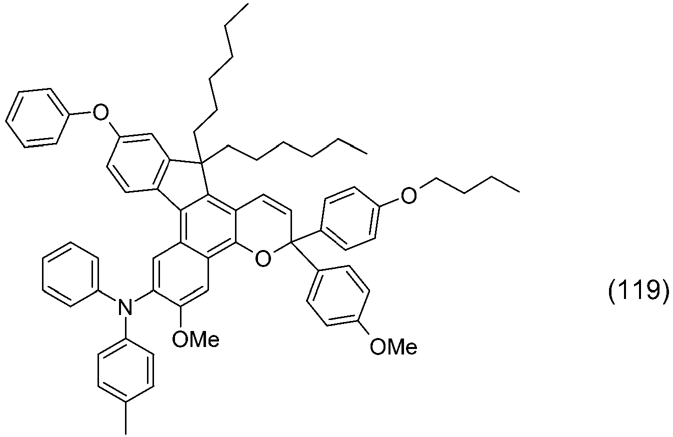

The photochromic compound represented by formula (119) above had elemental analysis values of C: 83.19%, H: 7.39%, and N: 1.43%, which were considerably consistent with the C₆₉H₇₃NO₃ calculated values of C: 83.18%, H: 7.39%, and N: 1.41%.

In a proton nuclear magnetic resonance spectroscopy, the photochromic compound represented by formula (119) showed 36H peaks attributed to a methyl group, a hexyl group, and a butyloxy group at around δ0.5 to 3.0 ppm, 8H peaks attributed to a methoxy group and a butyloxy group at around δ3.0 to 5.0 ppm, and 29H peaks attributed to aromatic protons and protons of an alkene at around δ5.0 to 9.0 ppm.

Furthermore, in a ¹³C-nuclear magnetic resonance spectroscopy, the photochromic compound represented by formula (119) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 53)

### First Step

The same reaction as in the first step of Example 48 was performed except that 2-[2-(2-methoxyethoxy)ethoxy]ethyl tosylate was used instead of 1-bromo-2-methoxyethane, the same reaction as in the second step of Example 48 was performed except that p-isopropoxyphenylboronic acid was used instead of p-trifluoromethylphenylboronic acid, and the same reaction as in the third step of Example 48 was performed except that N-methyl-4-(trifluoromethyl)benzeneamine was used instead of 7-methyl-3,4-dihydro-2H-1,4-benzoxazine, to obtain a naphthol compound represented by formula (120) below with a yield of 54%.

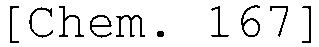

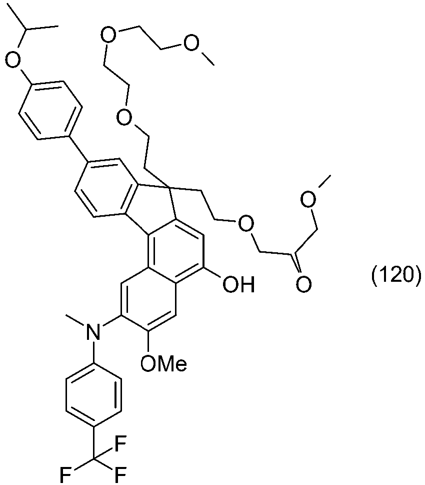

### Second Step

The same reaction as in the fifth step of Example 1 was performed except that the naphthol compound represented by formula (120) above was used instead of the naphthol compound represented by formula (26) above, to obtain a photochromic compound represented by formula (121) below with a yield of 76%.

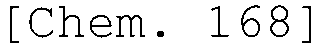

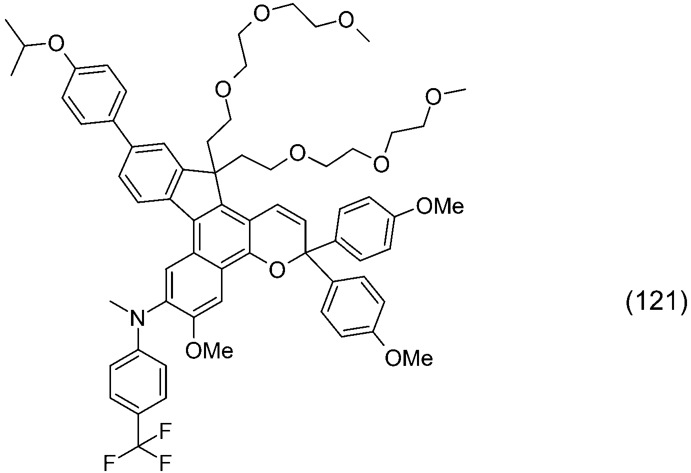

The photochromic compound represented by formula (121) above had elemental analysis values of C: 71.25%, H: 6.49%, and N: 1.24%, which were considerably consistent with the C₆₆H₇₂F₃NO₁₁ calculated values of C: 71.27%, H: 6.52%, and N: 1.26%.

In a proton nuclear magnetic resonance spectroscopy, the photochromic compound represented by formula (121) showed 13H peaks attributed to a methyl group, an isopropoxy group, and a 2-[2-(2-methoxyethoxy)ethoxy]ethyl group at around 50.5 to 3.0 ppm, 36H peaks attributed to a methoxy group, an isopropoxy group, and a 2-[2-(2-methoxyethoxy)ethoxy]ethyl group at around δ3.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (121) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 54)

### First Step

The same reaction as in the first step of Example 1 was performed except that 3-bromo-4-methoxy-4'-methoxybenzophenone was used instead of 3-bromo-4-methoxybenzophenone, N-butyl-4-(trifluoromethyl)benzeneamine was used instead of N-methylaniline, and cyclododecanone was used instead of acetone, to obtain a naphthol compound represented by formula (122) below with a yield of 48%.

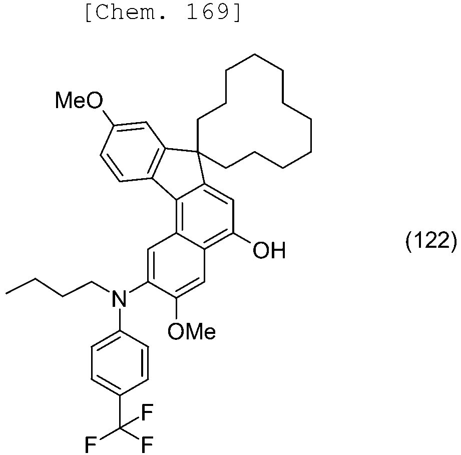

### Second Step

The same reaction as in the sixth step of Example 5 was performed except that the naphthol compound represented by formula (122) above was used instead of the naphthol compound represented by formula (41) above, to obtain a photochromic compound represented by formula (123) below with a yield of 71%.

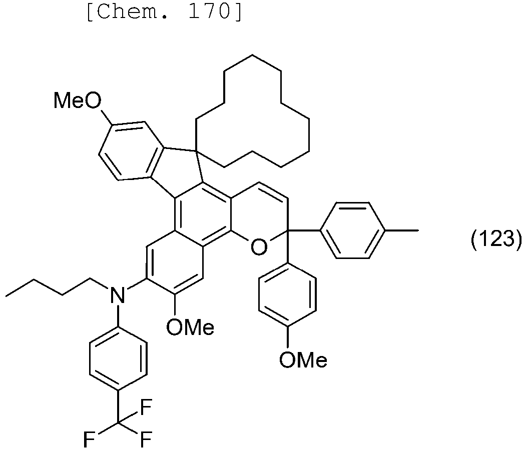

The photochromic compound represented by formula (123) above had elemental analysis values of C: 77.90%, H: 6.97%, and N: 1.56%, which were considerably consistent with the C₅₈H₆₂F₃NO₄ calculated values of C: 77.91%, H: 6.99%, and N: 1.57%.

In a proton nuclear magnetic resonance spectroscopy, the photochromic compound represented by formula (123) showed 34H peaks attributed to a methyl group, a butyl group, and a cyclododecane ring at around δ0.5 to 3.0 ppm, a 9H peak attributed to a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (123) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 55)

### First Step

The same reaction as in the second step of Example 8 was performed except that N-methylaniline was used instead of 1,2,3,4-tetrahydroquinoline, and the same reaction as in the third step of Example 8 was performed except that 1-bromo-3-methylthiopropane was used instead of 4-bromo-1,1,1-trifluorobutane, to obtain a naphthol compound represented by formula (124) below with a yield of 56%.

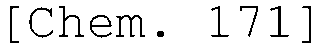

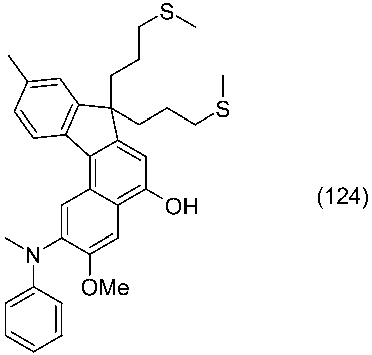

### Second Step

The same reaction as in the second step of Example 49 was performed except that the naphthol compound represented by formula (124) above was used instead of the naphthol compound represented by formula (109) above, and the same reaction as in the third step of Example 49 was performed except that a compound represented by formula (125) below was used instead of the compound represented by formula (110) above, to obtain a photochromic compound represented by formula (126) below with a yield of 65%.

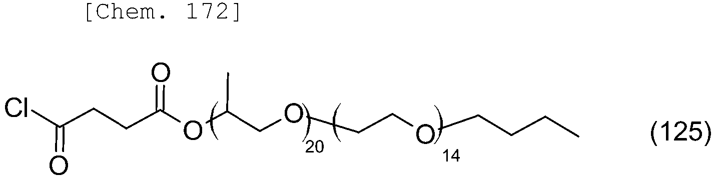

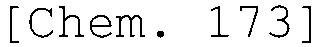

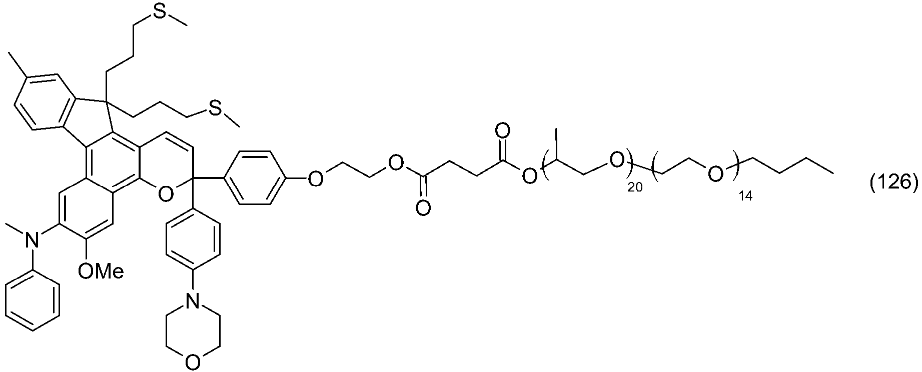

In a proton nuclear magnetic resonance spectroscopy, the photochromic compound represented by formula (126) above showed about 99H peaks attributed to a methyl group, a 3-mercaptomethylpropyl group, a morpholino group, a succinic acid moiety, a polypropylene glycol chain, and a butyloxy group at around δ0.5 to 3.0 ppm, about 129H peaks attributed to a methoxy group, an ethyleneoxy group, a polypropylene glycol chain, a polyethylene glycol chain, and a butoxy group at around δ3.0 to 5.0 ppm, and 20H peaks attributed to aromatic protons and protons of an alkene at around δ5.0 to 9.0 ppm.

### (Example 56)

### First Step

The same reaction as in the first step of Example 46 was performed except that iodooctane was used instead of iodobutane, and the same reaction as in the second step of Example 46 was performed except that triphenylchlorosilane was used instead of t-butyldimethylchlorosilane, and the same reaction as in the third step of Example 46 was performed except that diphenylamine was used instead of N-ethylaniline, to obtain a naphthol compound represented by formula (127) below with a yield of 40%.

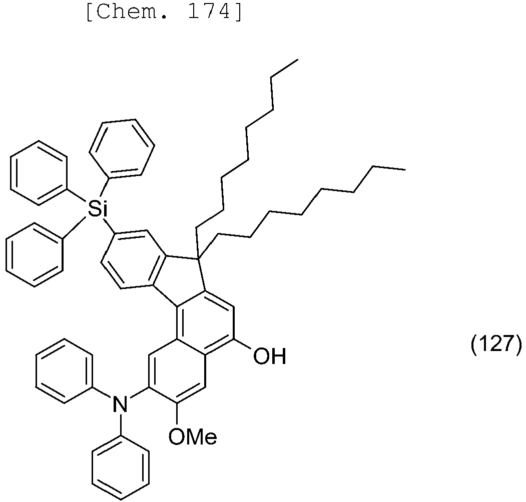

### Second Step

The same reaction as in the third step of Example 6 was performed except that the naphthol compound represented by formula (127) above was used instead of the naphthol compound represented by formula (45) above, to obtain a photochromic compound represented by formula (128) below with a yield of 63%.

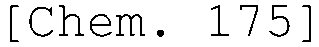

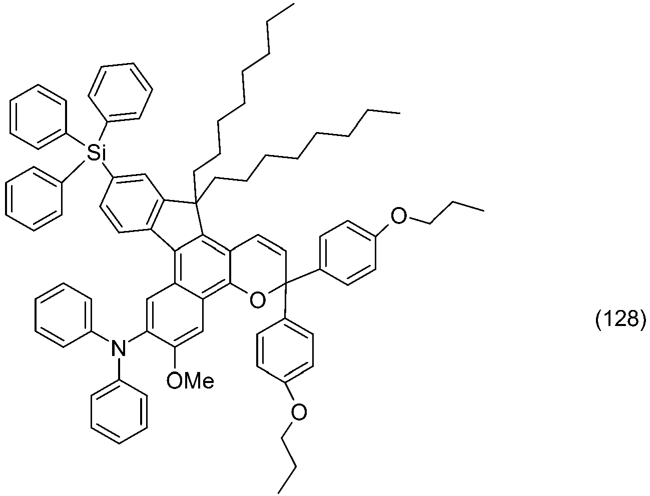

The photochromic compound represented by formula (128) above had elemental analysis values of C: 83.76%, H: 7.54%, and N: 1.16%, which were considerably consistent with the C₈₅H₉₁NO₄Si calculated values of C: 83.77%, H: 7.53%, and N: 1.15%.

In a proton nuclear magnetic resonance spectroscopy, the photochromic compound represented by formula (128) showed 44H peaks attributed to an octyl group and a propyloxy group at around δ0.5 to 3.0 ppm, 7H peaks attributed to a methoxy group and a propyloxy group at around δ3.0 to 5.0 ppm, and 40H peaks attributed to aromatic protons and protons of an alkene at around δ5.0 to 9.0 ppm.

Furthermore, in a ¹³C-nuclear magnetic resonance spectroscopy, the photochromic compound represented by formula (128) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 57)

### First Step

The same reaction as in the first step of Example 46 was performed except that 1-bromo-3-methoxypropane was used instead of iodobutane, to obtain a benzyl compound represented by formula (129) below with a yield of 86%.

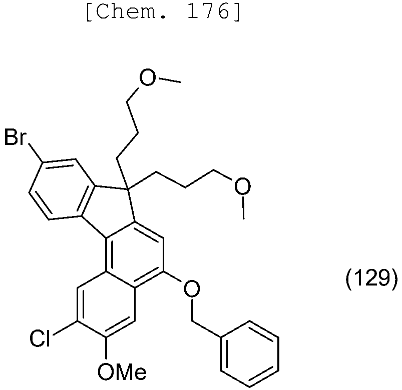

### Second Step

With a reference to the conditions described in Patent Document 3, the benzyl compound represented by formula (129) above was reacted with 2,6-dimethylbenzenethiol to obtain a benzyl compound represented by formula (130) below with a yield of 89%.

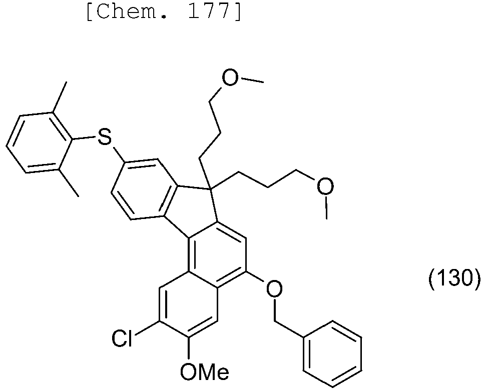

### Third Step

The same reaction as in the third step of Example 46 was performed except that the compound represented by formula (130) above was used instead of the compound represented by formula (97) above and N-(2-methoxyethyl)- (4-trifluoromethyl)benzeneamine was used instead of N-ethylaniline, to obtain a naphthol compound represented by formula (131) below with a yield of 69%.

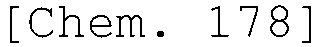

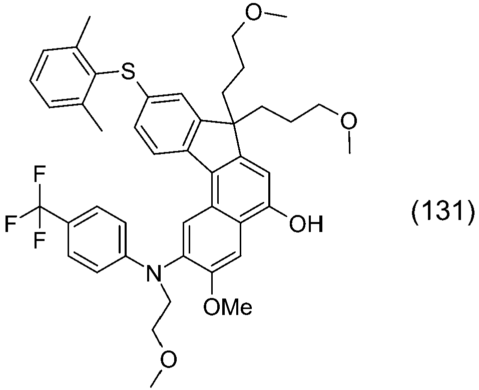

### Fourth Step

The same reaction as in the fifth step of Example 1 was performed except that the naphthol compound represented by formula (131) above was used instead of the naphthol compound represented by formula (26) above, to obtain a photochromic compound represented by formula (132) below with a yield of 71%.

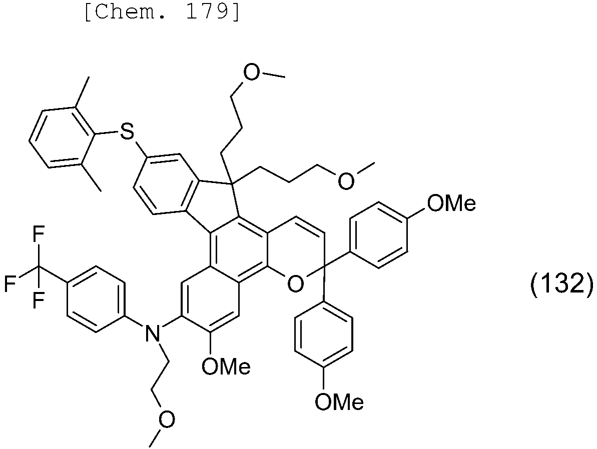

The photochromic compound represented by formula (132) above had elemental analysis values of C: 72.50%, H: 6.18%, N: 1.41%, and S: 3.16% which were considerably consistent with the C₆₁H₆₂F₃NO₇S calculated values of C: 72.52%, H: 6.19%, N: 1.39%, and S: 3.17%.

In a proton nuclear magnetic resonance spectroscopy, the photochromic compound represented by formula (132) showed 16H peaks attributed to a 3-methoxypropyl group, a 2-methoxyethyl group, and a methyl group at around δ0.5 to 3.0 ppm, 24H peaks attributed to a methoxy group, a 3-methoxypropyl group, and a 2-methoxyethyl group at around δ3.0 to 5.0 ppm, and 22H peaks attributed to aromatic protons and protons of an alkene at around δ5.0 to 9.0 ppm.

Furthermore, in a ¹³C-nuclear magnetic resonance spectroscopy, the photochromic compound represented by formula (132) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

The analysis results of the naphthol derivatives used in Examples 45 to 57 are summarized in Table 5.

### [Table 5]

**[table 5]**

| | Compound No. | Calculated value | | | | Measured value | | | | 1H-NMR |
|---|---|---|---|---|---|---|---|---|---|---|
| | | C | H | N | S | C | H | N | S | |
| Example 45 | Formula (94) | 84.29 | 7.44 | 2.52 | 0.00 | 84.26 | 7.46 | 2.54 | 0.00 | δ0.5-5.0ppm 25H |
| | | | | | | | | | | 55.0-9.0ppm 16H |
| Example 46 | Formula (99) | 79.03 | 8.79 | 2.30 | 0.00 | 79.01 | 8.80 | 2.31 | 0.00 | δ0.5-5.0ppm 41H |
| | | | | | | | | | | 55.0-9.0ppm 12H |
| Example 47 | Formula (102) | 75.38 | 6.69 | 2.51 | 0.00 | 75.35 | 6.67 | 2.49 | 0.00 | δ0.5-5.0ppm 26H |
| | | | | | | | | | | 55.0-9.0ppm 11H |
| Example 48 | Formula (106) | 71.73 | 5.72 | 2.09 | 0.00 | 71.71 | 5.71 | 2.07 | 0.00 | δ0.5-5.0ppm 24H |
| | | | | | | | | | | 55.0-9.0ppm 14H |
| Example 49 | Formula (111) | 78.19 | 6.75 | 2.61 | 0.00 | 78.20 | 6.74 | 2.63 | 0.00 | δ0.5-5.0ppm 25H |
| | | | | | | | | | | 55.0-9.0ppm 11H |
| Example 50 | Formula (115) | 74.12 | 5.31 | 1.80 | 0.00 | 74.09 | 5.30 | 1.77 | 0.00 | δ0.5-5.0ppm 21H |
| | | | | | | | | | | 55.0-9.0ppm 20H |
| Example 51 | Formula (117) | 72.11 | 6.24 | 2.55 | 0.00 | 72.09 | 6.21 | 2.54 | 0.00 | δ0.5-5.0ppm 22H |
| | | | | | | | | | | 55.0-9.0ppm 12H |
| Example 52 | Formula (119) | 83.60 | 7.59 | 1.99 | 0.00 | 83.61 | 7.61 | 1.98 | 0.00 | δ0.5-5.0ppm 32H |
| | | | | | | | | | | 55.0-9.0ppm 21H |
| Example 53 | Formula (121) | 68.28 | 6.78 | 1.62 | 0.00 | 68.27 | 6.75 | 1.60 | 0.00 | δ0.5-5.0ppm 43H |
| | | | | | | | | | | 55.0-9.0ppm 16H |
| Example 54 | Formula (123) | 74.63 | 7.33 | 2.12 | 0.00 | 74.61 | 7.31 | 2.10 | 0.00 | 50.5-5.0ppm 37H |
| | | | | | | | | | | 55.0-9.0ppm 11H |
| Example 55 | Formula (126) | 73.21 | 7.05 | 2.51 | 11.50 | 73.23 | 7.04 | 2.52 | 11.50 | δ0.5-5.0ppm 27H |
| | | | | | | | | | | 55.0-9.0ppm 12H |
| Example 56 | Formula (128) | 84.26 | 7.62 | 1.54 | 0.00 | 84.27 | 7.60 | 1.52 | 0.00 | 50.5-5.0ppm 37H |
| | | | | | | | | | | 55.0-9.0ppm 32H |
| Example 57 | Formula (132) | 69.54 | 6.37 | 1.84 | 4.22 | 69.52 | 6.38 | 1.82 | 4.23 | δ0.5-5.0ppm 34H |
| | | | | | | | | | | 55.0-9.0ppm 14H |

### (Examples 58 to 70)

In the same manner as in Example 23, photochromic laminates were prepared using the photochromic compounds obtained in Examples 45 to 57. The evaluation results are summarized in Table 6.

### [Table 6]

**[table 6]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 58 | Example 45 Formula (94) | 506 | 1.21 | 0.86 | 71% | 62 | 80% |
| Example 59 | Example 46 Formula (99) | 504 | 1.29 | 0.90 | 70% | 76 | 78% |
| Example 60 | Example 47 Formula (102) | 521 | 1.25 | 0.83 | 66% | 58 | 73% |
| Example 61 | Example 48 Formula (106) | 513 | 1.36 | 0.88 | 65% | 33 | 77% |
| Example 62 | Example 49 Formula (111) | 533 | 0.84 | 0.51 | 60% | 38 | 73% |
| Example 63 | Example 50 Formula (115) | 468 | 0.68 | 0.42 | 62% | 31 | 74% |
| | | 573 | 0.76 | 0.47 | 62% | 31 | 74% |
| Example 64 | Example 51 Formula (117) | 501 | 0.98 | 0.61 | 62% | 38 | 75% |
| Example 65 | Example 52 Formula (119) | 518 | 1.82 | 1.34 | 74% | 110 | 79% |
| Example 66 | Example 53 Formula (121) | 457 | 0.75 | 0.49 | 66% | 33 | 76% |
| | | 592 | 0.82 | 0.54 | 66% | 33 | 76% |
| Example 67 | Example 54 Formula (123) | 453 | 0.82 | 0.60 | 74% | 175 | 78% |
| | | 583 | 1.08 | 0.80 | 74% | 175 | 78% |
| Example 68 | Example 55 Formula (126) | 509 | 1.04 | 0.67 | 65% | 46 | 79% |
| | | 604 | 1.00 | 0.65 | 65% | 47 | 79% |
| Example 69 | Example 56 Formula (128) | 508 | 1.80 | 1.24 | 69% | 88 | 81% |
| Example 70 | Example 57 Formula (132) | 468 | 0.66 | 0.44 | 67% | 47 | 77% |
| | | 597 | 0.71 | 0.48 | 68% | 47 | 77% |

### <Physical Property Evaluation for Photochromic Layer Prepared by Binder Method>

### (Example 71)

A binder sheet was prepared using the following method. The binder sheet consisted of a first optical sheet, a first adhesive layer, a photochromic layer, a second adhesive layer, and a second optical sheet, which are laminated in this order. The first optical sheet and the second optical sheet were made of a polycarbonate sheet with a thickness of 400 µm.

### (Preparation of Photochromic Layer Forming Composition)

A 2L four-necked flask equipped with agitation blades, a cooling tube, a thermometer, and a nitrogen gas introducing tube was charged with 315 parts by mass of polycarbonate diol having a number-average molecular weight of 1000, 100 parts by mass of isophorone diisocyanate, and 72 parts by mass of toluene, and the mixture was reacted under nitrogen atmosphere at 100°C for 7 hours to synthesize a urethane prepolymer having an isocyanate group on its terminal. After completion of the urethane prepolymer reaction, the reaction liquid was cooled to approximately 0°C, then dissolved in 205 parts by mass of t-butyl alcohol and 382 parts by mass of diethyl ketone, and then the liquid temperature was maintained at 0°C. Subsequently, into this reaction liquid, a mixture of 21.3 parts by mass of bis-(4-aminocyclohexyl)methane as a chain extender and 20 parts by mass of diethyl ketone was dripped over within 30 minutes, and the mixture was reacted at 0°C for 1 hour. Then, into the mixture, 8.1 parts by mass of 1,2,2,6,6-pentamethyl-4-aminopiperidine was dripped, and the mixture was reacted at 0°C for 1 hour to obtain a diethyl ketone solution of a terminal non-reactive urethaneurea resin.

To 100 parts by mass of the obtained terminal non-reactive urethaneurea resin solution, the photochromic compound in Example 1 (represented by formula (28) above), 6.3 parts by mass of 4,4'-methylenebis(cyclohexyl isocyanate) isomer mixture (polyisocyanate compound), furthermore 0.4 part by mass of ethylene bis(oxyethylene)bis[3-(5-tert-butyl-4-hydroxy-m-tolyl)propionate] as an antioxidant, and 0.06 part by mass of DOW CORNING TORAY L-7001 as a surfactant were added, and the mixture was stirred and mixed at room temperature to obtain a photochromic layer forming composition.

Note that the addition amount of the photochromic compound was 0.25 mmol based on 100 parts by mass of the terminal non-reactive urethane urea resin.

### (Preparation of Adhesive Layer Composition)

A 5L separable flask (four-necked) equipped with agitation blades, a cooling tube, a thermometer, and a nitrogen gas introducing tube was charged with 400 parts by mass of polycarbonate diol having a number-average molecular weight of 1000, 175 parts by mass of isophorone diisocyanate, and 120 parts by mass of toluene, and the mixture was reacted under nitrogen atmosphere at 110°C for 7 hours to synthesize a urethane prepolymer having an isocyanate group on its terminal. After completion of the urethane prepolymer reaction, the reaction liquid was cooled to approximately 20°C, then dissolved in 2500 parts by mass of propylene glycol monomethyl ether, and then the liquid temperature was maintained at 20°C. Subsequently, into this reaction liquid, 60 parts by mass of isophorone diamine as a chain extender was dripped, and the mixture was reacted at 20°C for 1 hour. Then, into this mixture, 3 parts by mass of n-butylamine was dripped, and the mixture was reacted at 20°C for 1 hour to obtain a propylene glycol-monomethyl ether solution of a terminal non-reactive urethaneurea resin.

To 500 parts by mass of the obtained terminal non-reactive urethaneurea resin solution, 0.2 part by mass of DOW CORNING TORAY L-7001 manufactured as a surfactant was added, and the mixture was stirred and mixed at room temperature to obtain an adhesive layer composition.

### (Production of Binder Sheet)

Using a coater (manufactured by TESTER SANGYO CO., LTD.), the adhesive layer composition was applied onto one main face of the first optical sheet at a coating speed of 0.5 m/min, and dried at a drying temperature of 110°C for 3 minutes to obtain a first optical sheet having a 5 µm-thick first coating film. In the same manner as above, the adhesive layer composition was coated onto one main face of the second optical sheet to obtain a second optical sheet having a second coating film.

Subsequently, using a coater (manufactured by TESTER SANGYO CO., LTD.), a photochromic layer forming composition was applied onto a 50 µm-thick OPP film (stretched polypropylene film) at a coating speed of 0.3 m/min, and dried at a drying temperature of 100°C for 5 minutes. Thereby, a third coating film was obtained. Subsequently, the third coating film and the first optical sheet were bonded to each other so that the third coating film was in contact with the first coating film. From this structure, the OPP film was peeled off, and the second optical sheet and the third coating film were bonded to each other so that the exposed the other main face of the third coating film was in contact with the second coating film. Subsequently, the resulting laminate was allowed to stand under vacuum at 40°C for 24 hours, then heated at 110°C for 60 minutes, then humidified at 60°C and 100% RH for 24 hours, and finally allowed to stand under vacuum at 40°C for 24 hours to obtain a binder sheet. The obtained binder sheet was evaluated in the same manner as in Example 23. The results are presented in Table 7.

### (Examples 71 to 84, and Comparative Examples 9 to 13)

Binder sheets were prepared by the same method as in Example 70 using the photochromic compounds presented in Tables 7 and 8.

The results of Examples 70 to 73, and Comparative Examples 9 to 13 are summarized in Table 7, and the results of Examples 74 to 84 are summarized in Table 8.

### [Table 7]

**[table 7]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 70 | Example 1 Formula (28) | 501 | 1.17 | 0.80 | 69% | 98 | 95% |
| Example 71 | Example 2 Formula (30) | 510 | 1.52 | 1.07 | 70% | 104 | 94% |
| Example 72 | Example 4 Formula (34) | 496 | 1.20 | 0.79 | 66% | 97 | 93% |
| Example 73 | Example 19 Formula (83) | 515 | 1.36 | 0.91 | 67% | 105 | 91% |
| Comparative Example 9 | Formula (A) | 474 | 1.69 | 1.16 | 69% | 189 | 70% |
| | | 566 | 0.85 | 0.57 | 68% | 193 | 76% |
| Comparative Example 10 | Formula (E) | 481 | 1.85 | 1.30 | 70% | 217 | 68% |
| Comparative Example 11 | Formula (F) | 481 | 1.85 | 1.30 | 70% | 213 | 64% |
| Comparative Example 12 | Formula (G) | 493 | 2.29 | 1.92 | 84% | 385 | 49% |
| Comparative Example 13 | Formula (H) | 497 | 2.26 | 1.75 | 77% | 292 | 57% |

### [Table 8]

**[table 8]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 74 | Example 3 Formula (32) | 498 | 1.11 | 0.76 | 68% | 73 | 97% |
| Example 75 | Example 6 Formula (47) | 499 | 1.37 | 0.97 | 71% | 131 | 94% |
| Example 76 | Example 9 Formula (57) | 451 | 0.75 | 0.49 | 66% | 83 | 95% |
| | | 565 | 0.88 | 0.56 | 65% | 84 | 96% |
| Example 77 | Example 10 Formula (59) | 510 | 1.32 | 0.87 | 66% | 75 | 94% |
| Example 78 | Example 12 Formula (66) | 501 | 1.40 | 0.93 | 67% | 82 | 94% |
| Example 79 | Example 13 Formula (68) | 512 | 1. 60 | 1.09 | 68% | 88 | 96% |
| Example 80 | Example 14 Formula (71) | 521 | 1. 93 | 1.36 | 70% | 117 | 94% |
| Example 81 | Example 20 Formula (85) | 503 | 1.23 | 0.86 | 70% | 86 | 93% |
| Example 82 | Example 45 Formula (94) | 506 | 1.24 | 0.88 | 71% | 71 | 92% |
| Example 83 | Example 47 Formula (102) | 521 | 1.27 | 0.84 | 66% | 64 | 88% |
| Example 84 | Example 48 Formula (106) | 513 | 1.39 | 0.90 | 65% | 40 | 93% |

### <Physical Property Evaluation for Photochromic Cured Product Prepared by Kneading Method>

### (Example 85)

### (Preparation of Curable Composition)

First, the photochromic compound obtained in Example 1, additives, and a polymerizable compound were mixed to obtain a curable composition.

As the polymerizable compound, a combination of the following polymerizable monomers was used.
1,3-bis(isocyanatomethyl)cyclohexane: 36.7 parts by mass Pentaerythritol tetrakis(3-mercaptopropionate): 39.4 parts by mass
Polyoxyethylene polyoxypropylene lauryl ether (Wonder Surf 140 manufactured by Aoki Oil Industrial Co., Ltd.): 17.4 parts by mass
1-decanethiol: 2.8 parts by mass
RX-1 adjusted according to the method described in Patent Document 10: 3.8 parts by mass
Note that the photochromic compound was added to the curable composition so that the amount of the photochromic compound was 0.106 mmol based on 100 g of the total amount of the polymerizable monomer.

The following additives were used:
Dimethyltin dichloride
   : 0.05 part by mass
Irganox 245: 0.1 part by mass
2-ethylhexyl 4-methoxycinnamate: 0.6 part by mass

### (Production of Cured Product)

The prepared curable composition was thoroughly defoamed, then injected into a glass mold having a 1 mm gap, and polymerized by cast polymerization. The polymerization was carried out in an air furnace while gradually raising the temperature from 27°C to 120°C over 18 hours. After the polymerization, the cured product was removed from the glass mold to obtain a photochromic cured product having a thickness of 1 mm. The obtained photochromic cured product was evaluated in the same manner as in Example 23. The results are presented in Table 9.

### (Examples 86 to 94, and Comparative Example 14)

Photochromic cured products were prepared using photochromic compounds presented in Table 9 in the same manner as in Example 85. The results are summarized in Table 9.

### [Table 9]

**[table 9]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 85 | Example 1 Formula (28) | 505 | 1.19 | 0.83 | 70% | 124 | 89% |
| Example 86 | Example 2 Formula (30) | 514 | 1.51 | 1.07 | 71% | 133 | 88% |
| Example 87 | Example 4 Formula (34) | 500 | 1.20 | 0.81 | 67% | 123 | 84% |
| Example 88 | Example 19 Formula (83) | 519 | 1.37 | 0.91 | 67% | 131 | 83% |
| Example 89 | Example 3 Formula (32) | 502 | 1.12 | 0.77 | 69% | 91 | 90% |
| Example 90 | Example 8 Formula (54) | 549 | 1.20 | 0.79 | 66% | 79 | 80% |
| Example 91 | Example 10 Formula (59) | 514 | 1.34 | 0.91 | 68% | 94 | 89% |
| Example 92 | Example 15 Formula (75) | 527 | 1.22 | 0.76 | 63% | 53 | 81% |
| Example 93 | Example 17 Formula (79) | 562 | 1.09 | 0.75 | 69% | 97 | 84% |
| Example 94 | Example 20 Formula (85) | 507 | 1.24 | 0.87 | 70% | 107 | 85% |
| Comparative Example 14 | Formula (A) | 476 | 1.75 | 1.22 | 70% | 247 | 60% |
| | | 573 | 0.82 | 0.57 | 70% | 248 | 65% |

### (Example 95)

### First Step

By referring to the method described in Patent Document 6, 20.8 g (45.0 mmol) of compound represented by formula (22A) below synthesized from 3-bromo-4-methoxybenzophenone, 550 mL of toluene,
7.51 g (63.0 mmol) of indoline, and 17.3 g (179.7 mmol) of t-butoxy sodium were mixed and stirred under reduced pressure, from which dissolved oxygen was removed. Subsequently, 0.41 g (0.4 mmol) of Pd₂(dba)₃ and 0.86 g (1.8 mmol) of X-Phos were added to the reaction liquid, which was heated to 80°C.

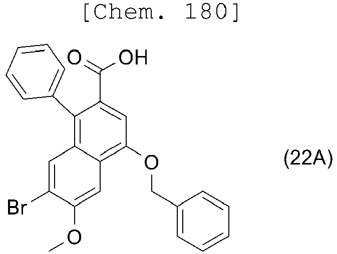

The heating was continued until the raw materials became invisible, and after completion of the reaction, the reaction liquid was cooled to room temperature, and then 700 mL of tetrahydrofuran and 10% hydrochloric acid was added to the reaction liquid, which was neutralized and then subjected to liquid separation. The resulting organic layer was concentrated and then purified by reslurry with 200 mL of methanol to obtain a carboxylic acid compound represented by formula (23A) below with a yield of 93%.

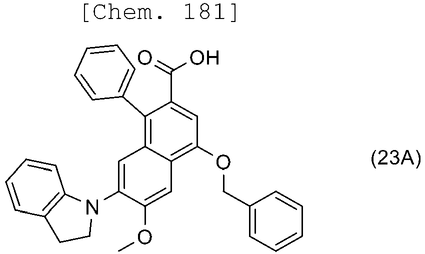

### Second Step

An iodine compound represented by formula (24A) below was obtained with a yield of 75% by referring to the method described in Patent Document 6 except that the carboxylic acid compound (23A) obtained in the first step was used.

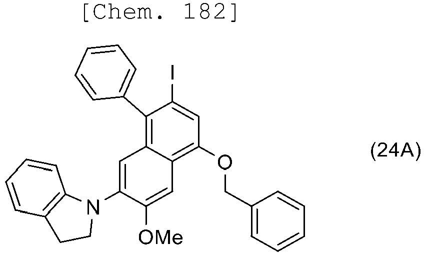

### Third Step

To 18.3 g (31.3 mmol) of compound represented by formula (24A) above obtained in the second step, 400 mL of toluene was added, and subjected to azeotropic dehydration until the water content in toluene decreased to 100 ppm or less. After the azeotropic dehydration, the mixture was slowly cooled to - 20°C, into which 24 mL of n-BuLi (1.6 mol/L hexane solution) was slowly dripped while maintaining the temperature at -15 to -20°C. After confirming that the raw materials had been consumed, 3.5 g of dehydrated acetone was dripped into the mixture. After the dripping, the temperature was slowly raised to room temperature. After the temperature raising, 200 mL of water was added to the mixture, which was subjected to liquid separation. The mixture was repeatedly washed with water until a pH of the water layer reached 7 to 8. From the obtained organic layer, the solvent was removed, and the organic layer was purified by a chromatography on silica gel to obtain a compound represented by formula (25A) below with a yield of 85%.

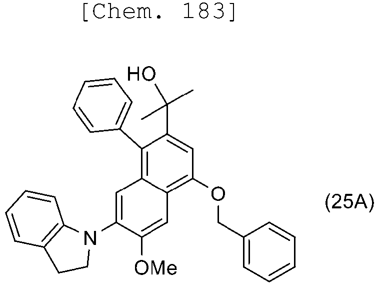

### Fourth Step

To 19.8 g (104.18 mmol) of p-toluenesulfonic acid monohydrate, 620 mL of toluene was added, which was subjected to azeotropic dehydration until the water content in toluene decreased to 300 ppm or less. Then, to this mixture, a toluene solution with 13.7 g (38.326.6 mmol) of the compound represented by formula (25A) above dissolved in 100 mL of toluene was slowly dripped while maintaining the temperature at 85 to 100°C, and after the dripping, the mixture was refluxed. After confirming that the raw materials had been consumed, the mixture was cooled to room temperature, to which 500 mL of water was added, which was subjected to liquid separation. This operation was repeated three times, the solvent was removed from the obtained organic layer, and the organic layer was purified by a chromatography on silica gel to obtain a naphthol compound represented by formula (26A) below with a yield of 74%.

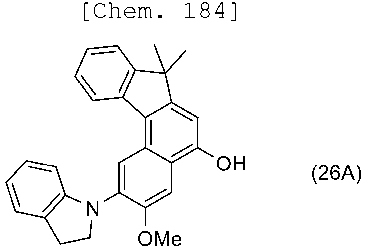

### Fifth Step

Into 40 mL of toluene, 2.04 g (5.0 mol) of the naphthol compound represented by formula (26A) above and 1.60 g (6.0 mmol) of propargyl alcohol represented by formula (27A) below were dissolved, additionally 0.12 g (0.5 mmol) of pyridinium p-toluenesulfonate was added, and the mixture was stirred at 85°C for 1 hour. After consuming the naphthol derivative as the raw material, the mixture was cooled to room temperature, and 40 mL of water was added to the mixture, which was subjected to liquid separation.

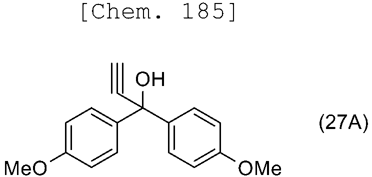

From the obtained organic layer, the solvent was removed, and the organic layer was purified by a chromatography on silica gel to obtain a photochromic compound represented by formula (28A) below with a yield of 78%.

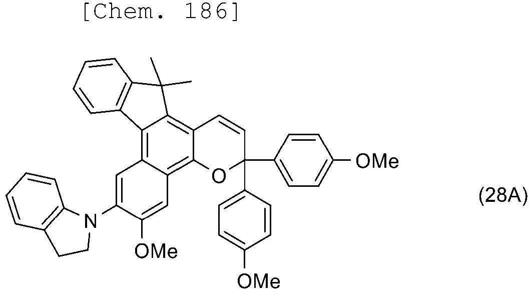

The photochromic compound represented by formula (28A) above had elemental analysis values of C: 82.16%, H: 6.01%, and N: 2.14%, which were considerably consistent with the C₄₅H₃₉NO₄ calculated values of C: 82.17%, H: 5.98%, and N: 2.13%.

In a proton nuclear magnetic resonance spectroscopy, the photochromic compound represented by formula (28A) showed a 6H peak attributed to a methyl group at around δ0.5 to 3.0 ppm, 13H peaks attributed to an indoline ring and a methoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (28A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 96)

### First Step

The same reaction as in the first step of Example 1 was performed except that 3-bromo-4-methylbenzophenone was used instead of 3-bromo-4-methoxybenzophenone, and the same reaction as in the third step of Example 1 was performed except that 4-heptanone was used instead of acetone, to synthesize a naphthol compound represented by formula (29A) below.

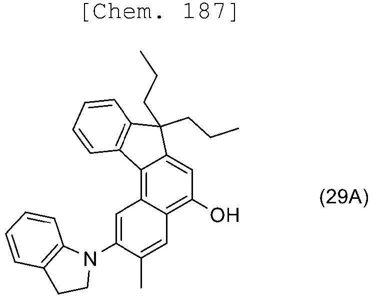

### Second Step

The same reaction as in the fifth step of Example 95 was performed except that the naphthol compound represented by formula (29A) above was used instead of the naphthol compound represented by formula (26A) above and a propargyl alcohol represented by formula (30A) below was used instead of the propargyl alcohol represented by formula (27A) above, to obtain a photochromic compound represented by formula (31A) below with a yield of 71%.

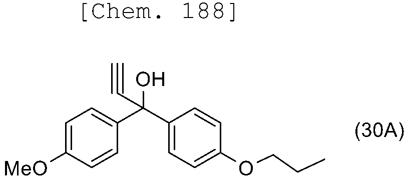

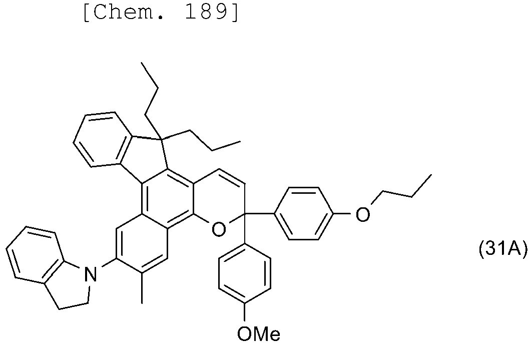

The photochromic compound represented by formula (31A) above had elemental analysis values of C: 84.35%, H: 7.09%, and N: 1.96%, which were considerably consistent with the C₃₁H₃₁NO₃ calculated values of C: 84.38%, H: 7.08%, and N: 1.93%.

In a proton nuclear magnetic resonance spectroscopy, the photochromic compound represented by formula (31A) showed 22H peaks attributed to a methyl group, a propyl group, and a propoxy group at around δ0.5 to 3.0 ppm, 9H peaks attributed to an indoline ring, a methoxy group, and a propoxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (31A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 97)

### First Step

By referring to the method described in Patent Document 3, the same reaction as in the first step of Example 1 was performed except that a benzophenone represented by formula (32A) below synthesized by a reaction of 4'-methyl(1,1'-biphenyl)-4-carbonyl chloride with 2-bromoanisole was used, and the same reaction as in the third step of Example 1 except that cyclooctanone was used instead of acetone, to synthesize a spiro compound represented by formula (33A) below.

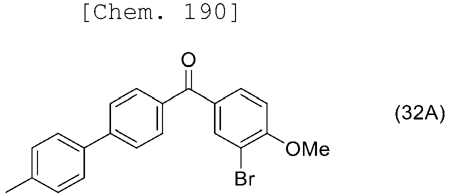

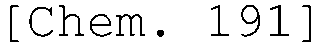

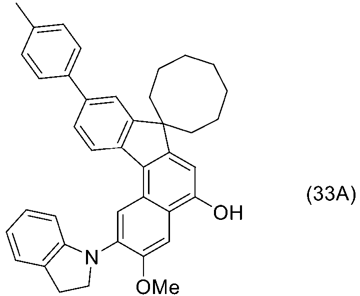

### Second Step

The same reaction as in the fifth step of Example 95 was performed except that the naphthol compound represented by formula (33A) above was used instead of the naphthol compound represented by formula (26A) above and a propargyl alcohol represented by formula (34A) below was used instead of the propargyl alcohol represented by formula (27A) above, to obtain a photochromic compound represented by formula (35A) below with a yield of 78%.

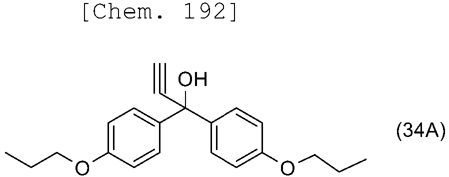

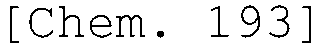

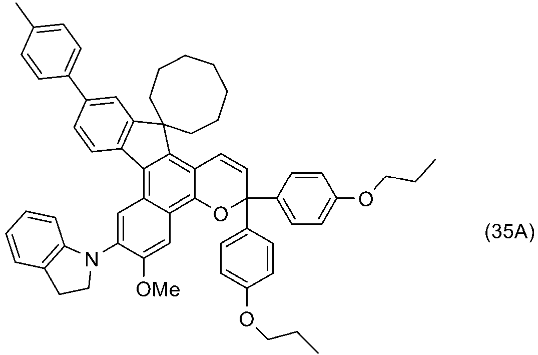

The photochromic compound represented by formula (35A) above had elemental analysis values of C: 84.04%, H: 7.01%, and N: 1.63%, which were considerably consistent with the C₆₁H₆₁NO₄ calculated values of C: 84.01%, H: 7.05%, and N: 1.61%.

In a proton nuclear magnetic resonance spectroscopy, the photochromic compound represented by formula (35A) showed 27H peaks attributed to a methyl group, a propoxy group, and a cyclooctane ring at around δ0.5 to 3.0 ppm, 11H peaks attributed to an indoline ring, a methoxy group, and a propoxy group at around δ3.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (35A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 98)

### First Step

The same reaction as in the first step of Example 95 was performed except that 3-methylindoline was used instead of indoline, to obtain an iodine compound represented by formula (36A) below.

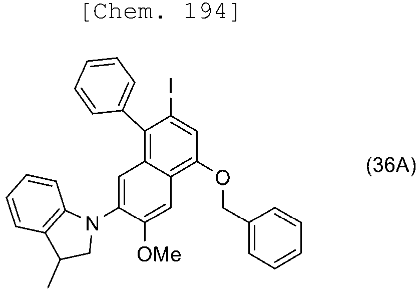

### Second Step

First, 25.8 g (43.1 mmol) of the iodine compound represented by formula (36A) above, 1.0 g (4.3 mmol) of palladium acetate, 42.3 g (431.0 mmol) of potassium acetate, 38.8 g (387.9 mmol) of potassium bicarbonate, 74.9 g (431.0 mmol) of dibromomethane, 260 mL of N,N-dimethylformamide (DMF), 86 mL of N,N-dimethylacetamide (DMAc), 4.2 mL of IPA, and 43 mL of water were mixed, and the mixture was heated and reacted at 80°C. After confirming that the iodine compound had been consumed, the mixture was allowed to cool to room temperature, and subjected to liquid separation using 100 mL of water and 300 mL of ethyl acetate. From the obtained organic layer, the solvent was removed, and then the organic layer was purified by a chromatography on silica gel to obtain a compound represented by formula (37A) below with a yield of 54%.

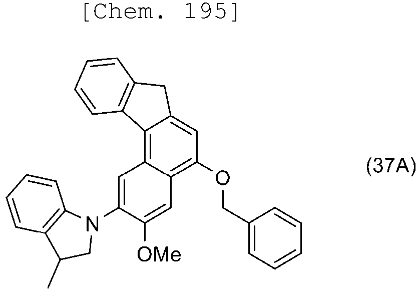

### Third Step

11.2 g (23.2 mmol) of the compound represented by formula (37A) above, 7.8 g (69.6 mmol) of potassium t-butoxide (tBuOK), and 400 mL of THF were mixed, and the mixture was cooled to 0 to 5°C. Into the mixture, 17.8 g (116 mmol) of 1-bromo-3-methoxypropane was slowly dripped over 15 minutes. After completion of the dripping, the mixture was stirred at room temperature. After confirming that the raw materials had been consumed, the mixture was cooled with ice, to which 80 mL of 2% hydrochloric acid and 500 mL of toluene were added, and the mixture was subjected to liquid separation. To the organic layer, 200 mL of water was added, and the mixture was washed with water. The mixture was repeatedly washed until a pH of a water layer reached 6 to 7. The solvent was removed from the obtained organic layer, and the 500 mL of THF and 0.3 g of 5% Pd/C (water content: 50%) were added to the organic layer, which was subjected to a pressurization reaction at 0.05 to 0.1 MPa with hydrogen gas. After confirming that the raw materials had been consumed, the Pd/C was filtered off from the organic layer, the solvent was removed from the obtained organic layer, and the organic layer was purified by a chromatography on silica gel to obtain a naphthol compound represented by formula (38A) below with a yield of 71%.

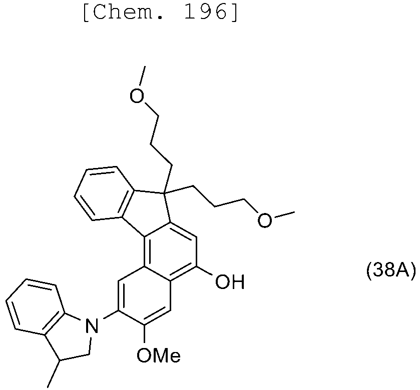

### Fourth Step

The same reaction as in the fifth step of Example 95 was performed except that the naphthol compound represented by formula (38A) above was used instead of the naphthol compound represented by formula (26A) above, to obtain a photochromic compound represented by formula (39A) below with a yield of 69%.

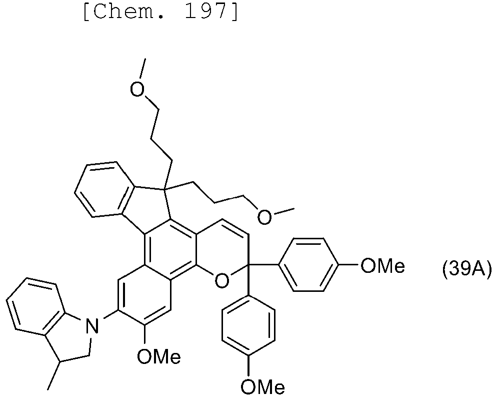

The photochromic compound represented by formula (39A) above had elemental analysis values of C: 79.29%, H: 6.76%, and N: 1.80%, which were considerably consistent with the C₅₂H₅₃NO₆ calculated values of C: 79.26%, H: 6.78%, and N: 1.78%.

In a proton nuclear magnetic resonance spectroscopy, the photochromic compound represented by formula (39A) showed 11H peaks attributed to a methyl group and a methoxypropyl group at around δ0.5 to 3.0 ppm, 22H peaks attributed to an indoline ring, a methoxy group, and a methoxypropyl group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (39A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 99)

### First Step

The same reaction as in Example 95 was performed except that 3-bromo-4-methyl-4'-methylbenzophenone was used instead of 3-bromo-4-methoxybenzophenone and diethylketone was used instead of acetone, to obtain a naphthol compound represented by formula (40A) below with a yield of 76%.

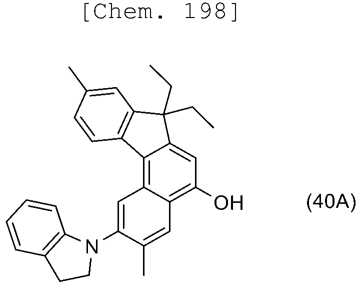

### Second Step

The same reaction as in the fifth step of Example 95 was performed except that the naphthol compound represented by formula (40A) above was used instead of the naphthol compound represented by formula (26A) above and a propargyl alcohol represented by formula (41A) below was used instead of the propargyl alcohol represented by formula (27A) above, to obtain a photochromic compound represented by formula (42A) below with a yield of 74%.

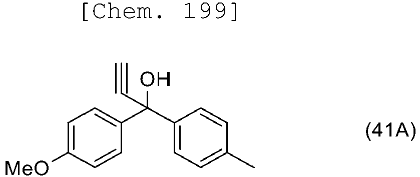

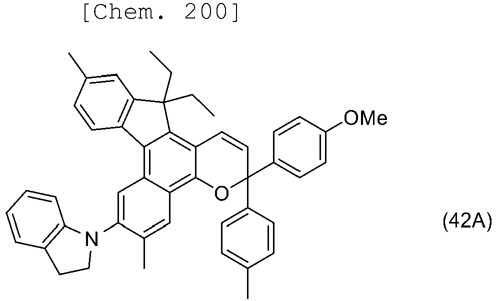

The photochromic compound represented by formula (42A) above had elemental analysis values of C: 86.30%, H: 6.79%, and N: 2.13%, which were considerably consistent with the C₄₈H₄₅NO₂ calculated values of C: 86.32%, H: 6.79%, and N: 2.10%.

In a proton nuclear magnetic resonance spectroscopy, the photochromic compound represented by formula (42A) showed 19H peaks attributed to a methyl group and an ethyl group at around 50.5 to 3.0 ppm, 7H peaks attributed to an indoline ring and a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (42A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 100)

The same reaction as in Example 98 was performed except that 3-bromo-4-methyl-4'-methylbenzophenone was used instead of 3-bromo-4-methoxybenzophenone and 1-bromo-3-trifluoromethylpropane was used instead of 1-bromo-3-methoxypropane, to obtain a naphthol compound represented by formula (43A) below with a yield of 67%.

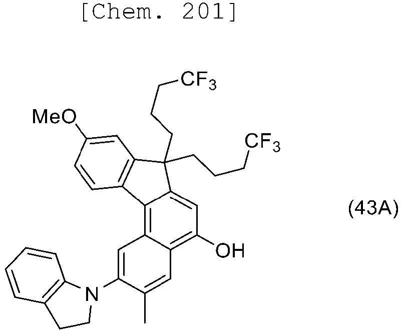

### Second Step

The same reaction as in the fifth step of Example 95 was performed except that the naphthol compound represented by formula (43A) above was used instead of the naphthol compound represented by formula (26A) above and a propargyl alcohol represented by formula (44A) below was used instead of the propargyl alcohol represented by formula (27A) above, to obtain a photochromic compound represented by formula (45A) below with a yield of 67%.

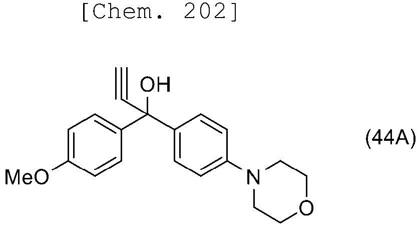

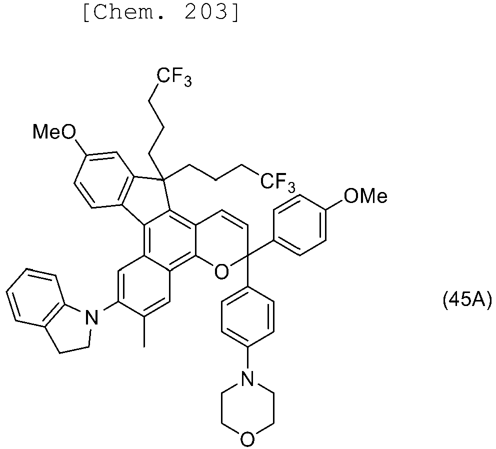

The photochromic compound represented by formula (45A) above had elemental analysis values of C: 71.80%, H: 5.67%, and N: 3.08%, which were considerably consistent with the C₅₅H₅₂F₆N₂O₄ calculated values of C: 71.88%, H: 5.70%, and N: 3.05%.

In a proton nuclear magnetic resonance spectroscopy, the photochromic compound represented by formula (45A) showed 19H peaks attributed to a methyl group, a 4-trifluoropropyl group, and a morpholino group at around δ0.5 to 3.0 ppm, 14H peaks attributed to an indoline ring, a methoxy group, and a morpholino group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (45A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 101)

### First Step

The same reaction as in the first step of Example 97 was performed except that 4'-trifluoromethyl(1,1'-biphenyl)-4-carbonyl chloride was used instead of 4'-methyl(1,1'-biphenyl)-4-carbonyl chloride and 5-methoxyindoline was used instead of indoline, and 3,3,5,5-tetramethylcyclohexanone was used instead of cyclooctanone, to obtain a naphthol compound represented by formula (46A) below with a yield of 69%.

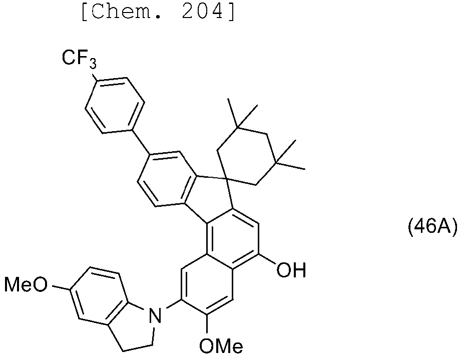

### Second Step

The same reaction as in the fifth step of Example 95 was performed except that the naphthol compound represented by formula (46A) above was used instead of the naphthol compound represented by formula (26A) above and a propargyl alcohol represented by formula (47) below was used instead of the propargyl alcohol represented by formula (27A) above, to obtain a photochromic compound represented by formula (48A) below with a yield of 52%.

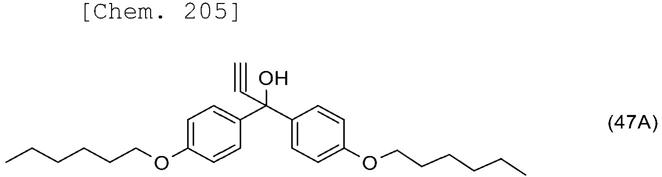

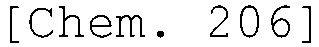

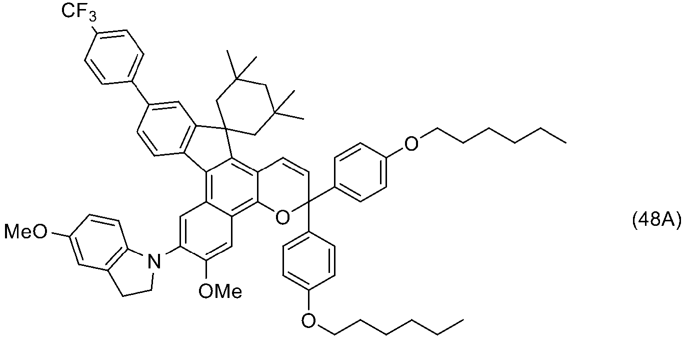

The photochromic compound represented by formula (48A) above had elemental analysis values of C: 78.68%, H: 7.20%, and N: 1.32%, which were considerably consistent with the C₇₀H₇₆F₃NO₅ calculated values of C: 78.70%, H: 7.17%, and N: 1.31%.

In a proton nuclear magnetic resonance spectroscopy, the photochromic compound represented by formula (48A) showed 40H peaks attributed to a 3,3,5,5-tetramethylcyclohexane ring and a hexyloxy group at around δ0.5 to 3.0 ppm, 14H peaks attributed to an indoline ring, a methoxy group, and a hexyloxy group at around δ3.0 to 5.0 ppm, and 22H peaks attributed to aromatic protons and protons of an alkene at around δ5.0 to 9.0 ppm.

Furthermore, in a ¹³C-nuclear magnetic resonance spectroscopy, the photochromic compound represented by formula (48A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 102)

### First Step

The same reaction as in the first step of Example 98 was performed except that 5-fluoroindoline was used instead of indoline and 2-[2-(2-methoxyethoxy)ethoxy]ethyl tosylate was used instead of 1-bromo-4-methoxypropane, to obtain a naphthol compound represented by formula (49A) below with a yield of 60%.

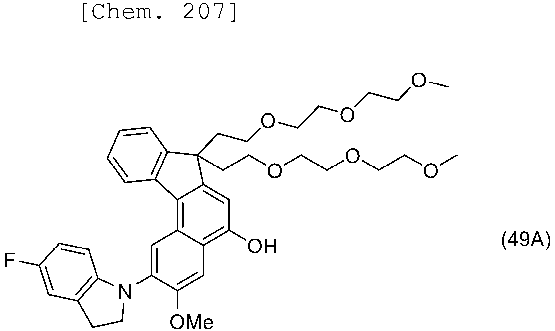

### Second Step

The same reaction as in the fifth step of Example 95 was performed except that the naphthol compound represented by formula (49A) above was used instead of the naphthol compound represented by formula (26A) above, to obtain a photochromic compound represented by formula (50A) below with a yield of 62%.

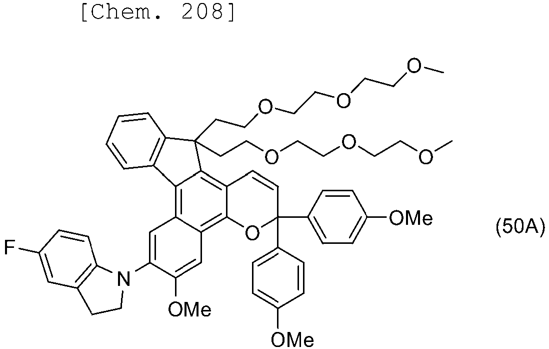

The photochromic compound represented by formula (50A) above had elemental analysis values of C: 72.80%, H: 6.67%, and N: 1.50%, which were considerably consistent with the C₅₇H₆₂FNO₁₀ calculated values of C: 72.82%, H: 6.65%, and N: 1.49%.

In a proton nuclear magnetic resonance spectroscopy, the photochromic compound represented by formula (50A) showed a 4H peak attributed to a 2-[2-(2-methoxyethoxy)ethoxy]ethyl group at around δ0.5 to 3.0 ppm, 39H peaks attributed to an indoline ring, a methoxy group, and a 2-[2-(2-methoxyethoxy)ethoxy]ethyl group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (50A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 103)

### First Step

The same reaction as in the first step of Example 95 was performed except that 5-methylindoline was used instead of indoline, to synthesize a naphthol compound represented by formula (51A) below. The obtained naphthol compound was reacted with the propargyl alcohol represented by formula (27A) above to obtain a photochromic compound represented by formula (52A) below with a yield of 72%.

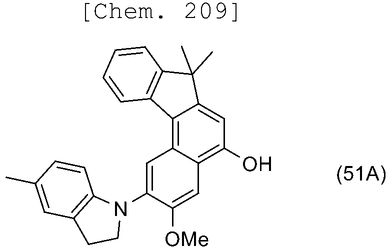

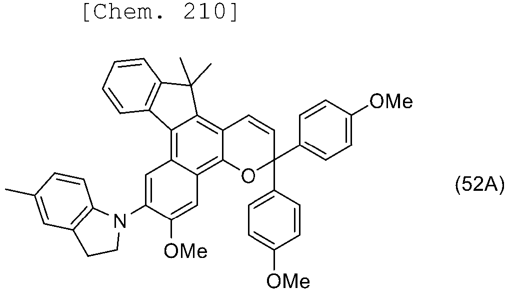

The photochromic compound represented by formula (52A) above had elemental analysis values of C: 82.21%, H: 6.17%, and N: 2.08%, which were considerably consistent with the C₄₆H₄₁NO₄ calculated values of C: 82.24%, H: 6.15%, and N: 2.08%.

In a proton nuclear magnetic resonance spectroscopy, the photochromic compound represented by formula (52A) showed a 9H peak attributed to a methyl group at around δ0.5 to 3.0 ppm, 13H peaks attributed to an indoline ring and a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (52A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 104)

### First Step

The same reaction as in Example 95 was performed except that 5-trifluoromethylindoline was used instead of indoline and 4-heptanone was used instead of acetone, to synthesize a naphthol compound represented by formula (53A) below with a yield of 69%.

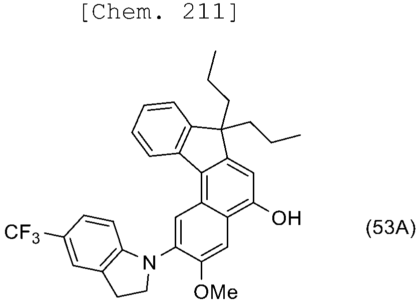

### Second Step

The same reaction as in the fifth step of Example 95 was performed except that the naphthol compound represented by formula (53A) above was used instead of the naphthol compound represented by formula (26A) above and the propargyl alcohol represented by formula (34A) above was used instead of the propargyl alcohol represented by formula (27A) above, to obtain a photochromic compound represented by formula (54A) below with a yield of 68%.

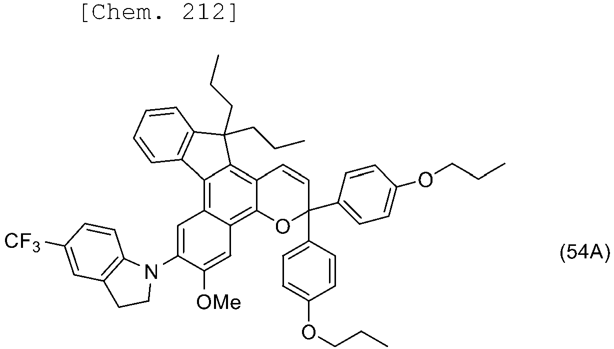

The photochromic compound represented by formula (54A) had elemental analysis values of C: 77.43%, H: 6.52%, and N: 1.65%, which were considerably consistent with the C₅₄H₅₄F₃NO₄ calculated values of C: 77.40%, H: 6.50%, and N: 1.67%.

In a proton nuclear magnetic resonance spectroscopy, the photochromic compound represented by formula (54A) showed a 9H peak attributed to a methyl group at around δ0.5 to 3.0 ppm, 13H peaks attributed to an indoline ring and a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (54A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 105)

### First Step

The same reaction as in the third step of Example 95 was performed except that spiro[5,5]undecan-3-one was used instead of acetone, to obtain a naphthol compound represented by formula (55A) below with a yield of 54%.

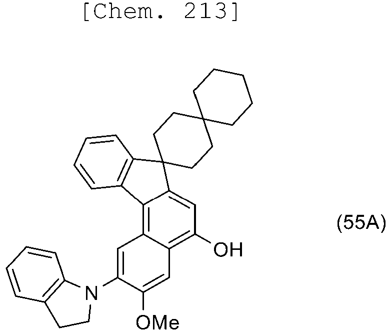

### Second Step

The same reaction as in the fifth step of Example 95 was performed except that the naphthol compound represented by formula (55A) above was used instead of the naphthol compound represented by formula (26A) above and a propargyl alcohol represented by formula (56A) below was used instead of the propargyl alcohol represented by formula (27A) above, to obtain a photochromic compound represented by formula (57A) below with a yield of 61%.

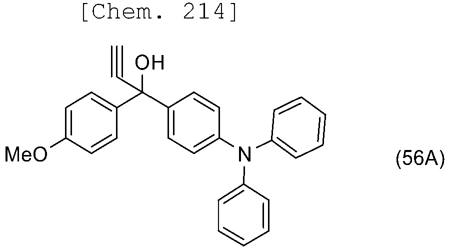

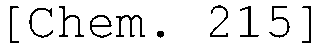

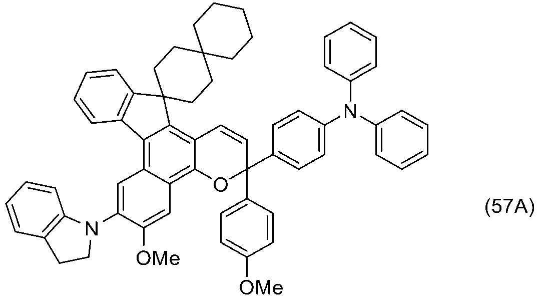

The photochromic compound represented by formula (57A) above had elemental analysis values of C: 85.11%, H: 6.46%, and N: 3.13%, which were considerably consistent with the C₆₄H₅₈N₂0₃ calculated values of C: 85.11%, H: 6.47%, and N: 3.10%.

In a proton nuclear magnetic resonance spectroscopy, the photochromic compound represented by formula (57A) showed a 18H peak attributed to a spiro[5,5]undecane ring at around δ0.5 to 3.0 ppm, 10H peaks attributed to an indoline ring and a methoxy group at around δ3.0 to 5.0 ppm, and 30H peaks attributed to aromatic protons and protons of an alkene at around δ5.0 to 9.0 ppm.

Furthermore, in a ¹³C-nuclear magnetic resonance spectroscopy, the photochromic compound represented by formula (57A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Example 106)

### First Step

The same reaction as in the first step of Example 97 was performed except that 4-phenylbenzoyl chloride was used instead of 4'-methyl(1,1'-biphenyl)-4-carbonyl chloride and 4,4-diethylcyclohexanone was used instead of cyclooctanone, to obtain a naphthol compound represented by formula (58A) below with a yield of 69%.

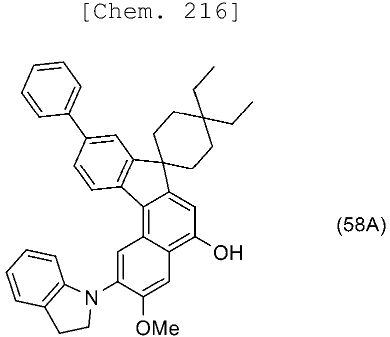

### Second Step

The same reaction as in the fifth step of Example 95 was performed except that the naphthol compound represented by formula (58A) above was used instead of the naphthol compound represented by formula (26A) above and a propargyl alcohol represented by formula (59A) below was used instead of the propargyl alcohol represented by formula (27A) above, to obtain a photochromic compound represented by formula (60A) below with a yield of 68%.

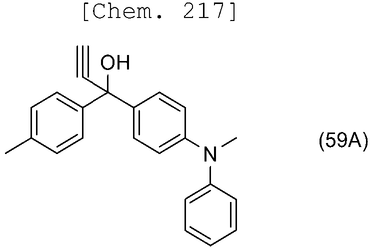

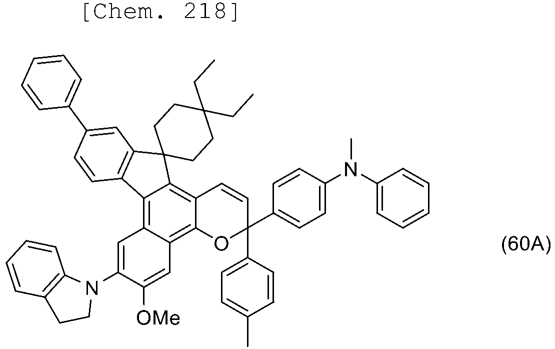

The photochromic compound represented by formula (60A) above had elemental analysis values of C: 86.42%, H: 6.78%, and N: 3.63%, which were considerably consistent with the C₆₄H₆₀N₂O₂ calculated values of C: 86.45%, H: 6.80%, and N: 3.60%.

In a proton nuclear magnetic resonance spectroscopy, the photochromic compound represented by formula (60A) showed 21H peaks attributed to a diethylcyclohexane ring and a methyl group at around δ0.5 to 3.0 ppm, 10H peaks attributed to an indoline ring, a methyl group, and a methoxy group at around δ3.0 to 5.0 ppm, and 29H peaks attributed to aromatic protons and protons of an alkene at around δ5.0 to 9.0 ppm.

Furthermore, in a ¹³C-nuclear magnetic resonance spectroscopy, the photochromic compound represented by formula (60A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### (Analysis Result of Naphthol Derivative)

The analysis results of the naphthol derivatives used in Examples 95 to 106 are summarized in Table 10.

### [Table 10]

**[table 10]**

| | Compound No. | Calculated value | | | Measured value | | | 1H-NMR |
|---|---|---|---|---|---|---|---|---|
| | | C | H | N | C | H | N | |
| Example 95 | Formula (28A) | 82.53 | 6.18 | 3.44 | 82.50 | 6.17 | 3.45 | δ0.5-5.0ppm 13H |
| | | | | | | | | δ5.0-9.0ppm 12H |
| Example 96 | Formula (31A) | 85.87 | 7.43 | 3.13 | 85.89 | 7.41 | 3.11 | δ0.5-5.0ppm 21H |
| | | | | | | | | 55.0-9.0ppm 12H |
| Example 97 | Formula (35A) | 84.92 | 6.95 | 2.48 | 84.90 | 6.94 | 2.50 | δ0.5-5.0ppm 24H |
| | | | | | | | | 55.0-9.0ppm 15H |
| Example 98 | Formula (39A) | 78.18 | 7.31 | 2.60 | 78.15 | 7.33 | 2.59 | δ0.5-5.0ppm 27H |
| | | | | | | | | 55.0-9.0ppm 12H |
| Example 99 | Formula (42A) | 85.87 | 7.21 | 3.23 | 85.89 | 7.22 | 3.25 | δ0.5-5.0ppm 20H |
| | | | | | | | | 55.0-9.0ppm 11H |
| Example 100 | Formula (45A) | 68.51 | 5.42 | 2.28 | 68.49 | 5.44 | 2.29 | δ0.5-5.0ppm 22H |
| | | | | | | | | 55.0-9.0ppm 11H |
| Example 101 | Formula (48A) | 76.20 | 6.25 | 2.07 | 76.22 | 6.24 | 2.06 | δ0.5-5.0ppm 28H |
| | | | | | | | | 55.0-9.0ppm 14H |
| Example 102 | Formula (50A) | 69.65 | 7.01 | 2.03 | 69.65 | 7.04 | 2.02 | δ0.5-5.0ppm 37H |
| | | | | | | | | δ5.0-9.0ppm 11H |
| Example 103 | Formula (52A) | 82.63 | 6.46 | 3.32 | 82.64 | 6.44 | 3.32 | δ0.5-5.0ppm 16H |
| | | | | | | | | δ5.0-9.0ppm 11H |
| Example 104 | Formula (54A) | 74.56 | 6.07 | 2.63 | 74.58 | 6.04 | 2.66 | δ0.5-5.0ppm 21H |
| | | | | | | | | δ5.0-9.0ppm 11H |
| Example 105 | Formula (57A) | 83.85 | 7.23 | 2.72 | 83.88 | 7.21 | 2.71 | δ0.5-5.0ppm 25H |
| | | | | | | | | δ5.0-9.0ppm 12H |
| Example 106 | Formula (60A) | 84.94 | 7.13 | 2.42 | 84.91 | 7.16 | 2.44 | δ0.5-5.0ppm 25H |
| | | | | | | | | 55.0-9.0ppm 16H |

### (Physical Property Evaluation for Photochromic Plastic Lens Prepared by Coating Method)

### (Examples 107 to 118, and Comparative Example 15)

In the same manner as in Example 23, each of the photochromic compounds obtained in Examples 95 to 106 was used to prepare a photochromic laminate of a compound represented by formula (I) below. Note that the compound (I) was synthesized by referring to the method described in PCT International Publication No. WO2011/034202.

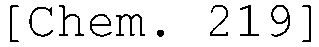

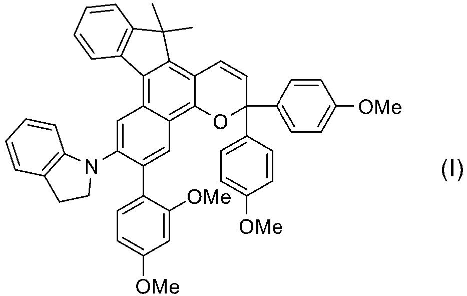

### <Evaluation Method>

The obtained photochromic laminates were evaluated in the same manner as in Example 23. The results of Examples 107 and 115, and Comparative Examples 1, 4, 5, 7, 8, and 15 are summarized in Table 11, and the results of Examples 108 to 114 and 116 to 118 are summarized in Table 12.

### [Table 11]

**[table 11]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 107 | Example 95 Formula (28A) | 532 | 1.57 | 1.16 | 74% | 125 | 74% |
| Example 115 | Example 103 Formula (52A) | 535 | 1. 68 | 1.26 | 75% | 140 | 76% |
| Comparative Example 1 | Formula (A) | 474 | 1.65 | 1.14 | 69% | 170 | 53% |
| | | 567 | 0.83 | 0.57 | 69% | 172 | 57% |
| Comparative Example 4 | Formula (D) | 449 | 0.69 | 0.44 | 64% | 95 | 74% |
| | | 569 | 0.87 | 0.55 | 63% | 95 | 73% |
| Comparative Example 5 | Formula (E) | 481 | 1.81 | 1.28 | 71% | 194 | 51% |
| Comparative Example 7 | Formula (G) | 493 | 2.24 | 1.89 | 84% | 345 | 38% |
| Comparative Example 8 | Formula (H) | 497 | 2.21 | 1.72 | 78% | 262 | 45% |
| Comparative Example 15 | Formula (I) | 522 | 1. 65 | 1 . 17 | 71% | 135 | 71% |

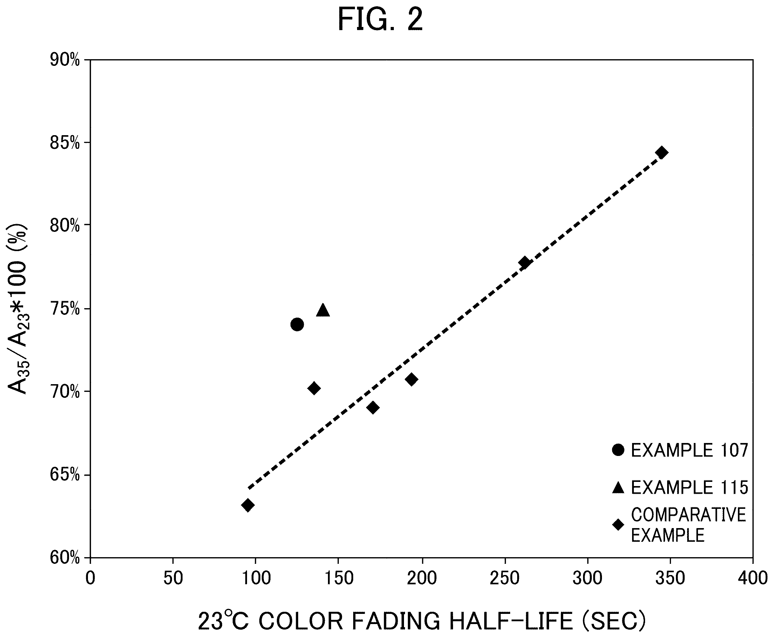

FIG. 2 is a graph presenting another example of the relationship between the color fading half-life at 23°C and the high-temperature color development rate of each photochromic laminate according to Examples and Comparative Examples. FIG. 2 is based on data of Example 107, Example 115, Comparative Example 1, Comparative Examples 4 to 8, and Comparative Example 15. The plots of the Comparative Examples correspond to, from the left, Comparative Example 4, Comparative Example 15, Comparative Example 1, Comparative Example 5, Comparative Example 8, and Comparative Example 7.

### [Table 12]

**[table 12]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 108 | Example 96 Formula (31A) | 520 | 1.36 | 0.89 | 65% | 63 | 67% |
| Example 109 | Example 97 Formula (35A) | 531 | 1.71 | 1.34 | 78% | 141 | 75% |
| Example 110 | Example 98 Formula (39A) | 533 | 1.50 | 1.12 | 75% | 83 | 71% |
| Example 111 | Example 99 Formula (42A) | 535 | 1. 62 | 1.21 | 75% | 139 | 70% |
| Example 112 | Example 100 Formula (45A) | 489 | 1.12 | 0.75 | 67% | 67 | 65% |
| | | 596 | 1.51 | 1.02 | 67% | 68 | 65% |
| Example 113 | Example 101 Formula (48A) | 539 | 1.07 | 0.66 | 62% | 31 | 69% |
| Example 114 | Example 102 Formula (50A) | 522 | 1.43 | 1.00 | 70% | 67 | 70% |
| Example 116 | Example 104 Formula (54A) | 486 | 0.99 | 0.66 | 67% | 60 | 69% |
| | | 592 | 0.96 | 0.64 | 66% | 59 | 69% |
| Example 117 | Example 105 Formula (57A) | 548 | 1. 68 | 1.18 | 70% | 137 | 65% |
| Example 118 | Example 106 Formula (60A) | 535 | 1.32 | 0.94 | 71% | 112 | 69% |
| | | 595 | 1.64 | 1.17 | 71% | 114 | 69% |

### Example 119

### First Step

By referring to the method described in Patent Document 3, the same reaction as in the first step of Example 95 was performed except that a benzophenone represented by formula (61A) below, synthesized by reacting 4-trifluoromethylbenzoyl chloride with 2-bromotoluene, was used, and the same reaction as in the third step of Example 95 was performed except that dibutylketone was used instead of acetone, to synthesize a spiro compound represented by formula (62A) below.

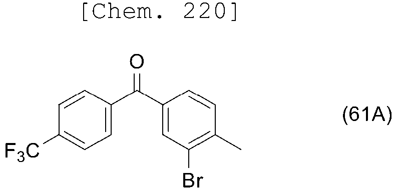

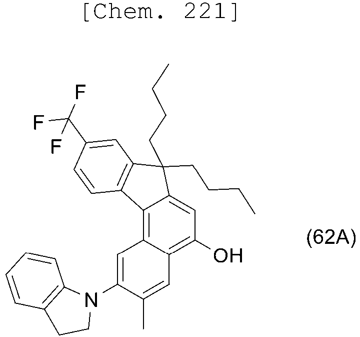

### Second Step

The same reaction as in the fifth step of Example 95 was performed except that the naphthol compound represented by formula (62A) above was used instead of the naphthol compound represented by formula (26A) above and a propargyl alcohol represented by formula (63A) below was used instead of the propargyl alcohol represented by formula (27A) above, to obtain a photochromic compound represented by formula (64A) below with a yield of 71%.

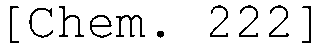

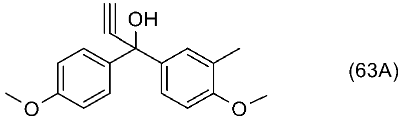

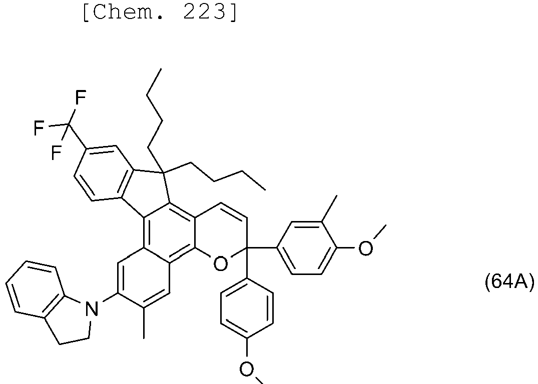

The photochromic compound represented by formula (64A) above had elemental analysis values of C: 78.75%, H: 6.51%, and N: 1.72%, which were considerably consistent with the C₅₃H₅₂F₃NO₃ calculated values of C: 78.78%, H: 6.49%, and N: 1.73%.

In a proton nuclear magnetic resonance spectroscopy, the photochromic compound represented by formula (64A) showed 24H peaks attributed to a methyl group and a butyl group at around δ0.5 to 3.0 ppm, 10H peaks attributed to an indoline ring and a methoxy group at around δ3.0 to 5.0 ppm, and 18H peaks attributed to aromatic protons and protons of an alkene at around δ5.0 to 9.0 ppm.

Furthermore, in a ¹³C-nuclear magnetic resonance spectroscopy, the photochromic compound represented by formula (64A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 120

### First Step

The same reaction as in the first step of Example 98 was performed except that indoline was used instead of 3-methylindoline, the same reaction as in the third step of Example 98 was performed except that 3-bromo-1,1,1-trifluoropropane was used instead of 1-bromo-3-methoxypropane, to synthesize a naphthol compound represented by formula (65A) below.

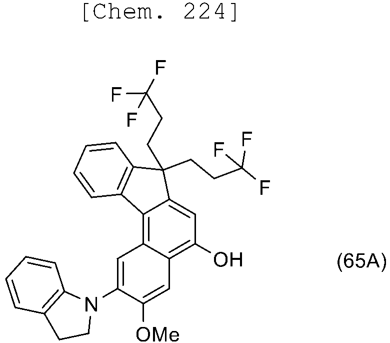

### Second Step

The same reaction as in the fifth step of Example 95 was performed except that the naphthol compound represented by formula (65A) above was used instead of the naphthol compound represented by formula (26A) above and a propargyl alcohol represented by formula (66A) below was used instead of the propargyl alcohol represented by formula (27A) above, to obtain a photochromic compound represented by formula (67A) below with a yield of 63%.

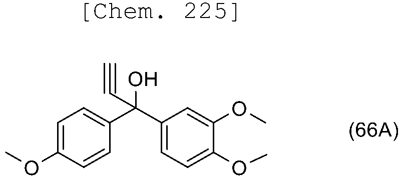

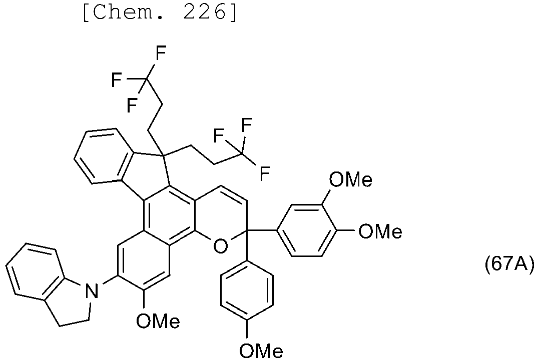

The photochromic compound represented by formula (67A) above had elemental analysis values of C: 70.46%, H: 5.07%, and N: 1.63%, which were considerably consistent with the C₅₀H₄₃F₆NO₅ calculated values of C: 70.50%, H: 5.09%, and N: 1.64%.

In a proton nuclear magnetic resonance spectroscopy, the photochromic compound represented by formula (67A) showed an 8H peak attributed to a 3-trifluoroethyl group at around δ0.5 to 3.0 ppm, 16H peaks attributed to an indoline ring and a methoxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (67A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 121

### First Step

The same reaction as in the first step of Example 97 was performed except that 4-phenoxybenzoyl chloride was used instead of 4'-methyl(1,1'-biphenyl)-4-carbonyl chloride and methylpropylketone was used instead of cyclooctanone, to synthesize a spiro compound represented by formula (68A) below.

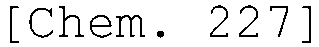

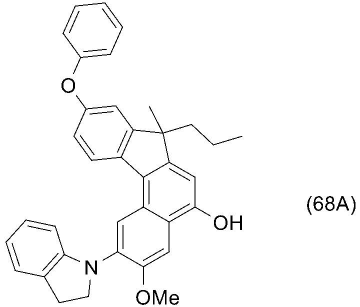

### Second Step

The same reaction as in the fifth step of Example 95 was performed except that the naphthol compound represented by formula (68A) above was used instead of the naphthol compound represented by formula (26A) above and a propargyl alcohol represented by formula (69A) below was used instead of the propargyl alcohol represented by formula (27A) above, to obtain a photochromic compound represented by formula (70A) below with a yield of 69%.

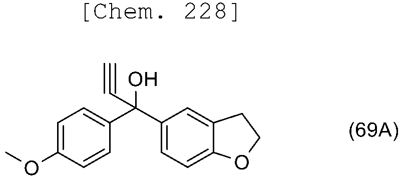

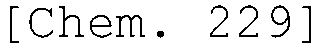

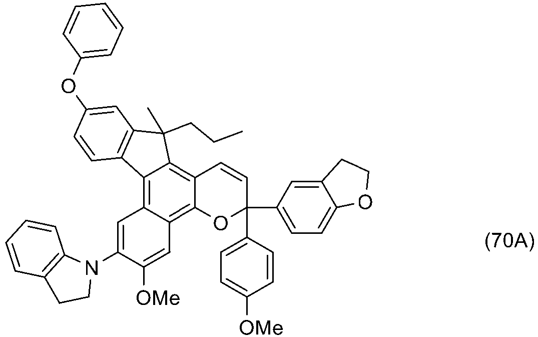

The photochromic compound represented by formula (70A) above had elemental analysis values of C: 82.12%, H: 6.01%, and N: 1.75%, which were considerably consistent with the C₅₄H₄₇NO₅ calculated values of C: 82.10%, H: 6.00%, and N: 1.77%.

In a proton nuclear magnetic resonance spectroscopy, the photochromic compound represented by formula (70A) showed 12H peaks attributed to a methyl group, a propyl group, and a dihydrobenzofuran group at around δ0.5 to 3.0 ppm, 12H peaks attributed to an indoline ring, a methoxy group, and a dihydrobenzofuran group at around δ3.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (70A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 122

### First Step

The same reaction as in the first step of Example 97 was performed except that 4-trifluoromethoxybenzoyl chloride was used instead of 4'-methyl(1,1'-biphenyl)-4-carbonyl chloride and 4,4-diethylcyclohexanone was used instead of cyclooctanone, to synthesize a spiro compound represented by formula (71A) below.

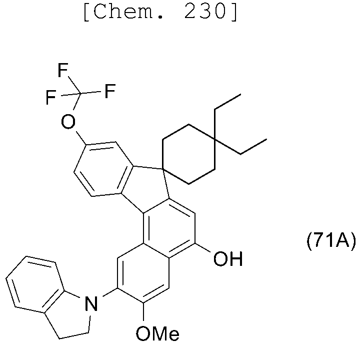

### Second Step

The same reaction as in the fifth step of Example 95 was performed except that the naphthol compound represented by formula (71A) above was used instead of the naphthol compound represented by formula (26A) above and a propargyl alcohol represented by formula (72A) below was used instead of the propargyl alcohol represented by formula (27A) above, to obtain a photochromic compound represented by formula (73A) below with a yield of 57%.

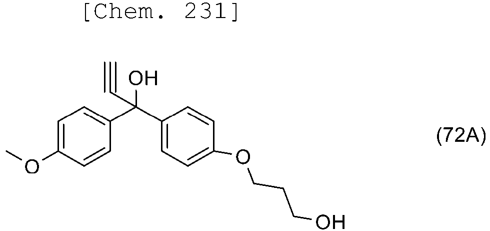

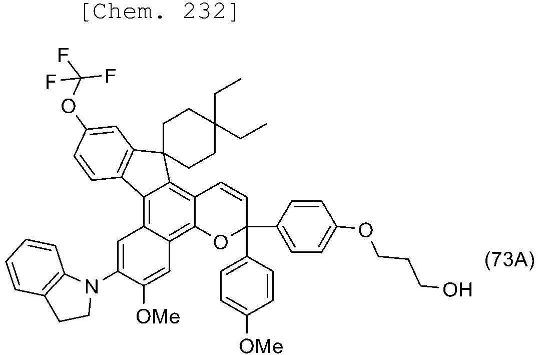

### Third Step

First, 4.4 g (5.0 mmol) of the photochromic compound represented by formula (73A) above and 0.75 g (11.0 mmol) of imidazole were dissolved in 50 mL of dimethylformamide. The internal temperature was decreased to 5°C or lower, and then, to the mixture, 10 mL of dimethylformamide solution containing 2.3 g (5.5 mmol) of [tris(trimethylsiloxy)silylethyl]dimethylchlorosilane was slowly dripped while maintaining the internal temperature at 5°C or lower. After the dripping, the mixture was stirred for 12 hours while slowly raising the temperature to room temperature. After completion of the reaction, 50 mL of water and 100 mL of toluene were added to the mixture, which was subjected to liquid separation. From the resulting organic layer, the solvent was removed, and the organic layer was purified by a chromatography on silica gel to obtain a photochromic compound represented by formula (74A) below with a yield of 89%.

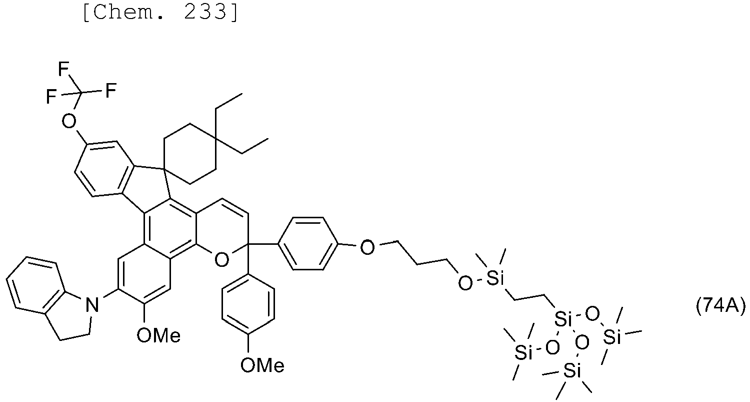

The photochromic compound represented by formula (74A) above had elemental analysis values of C: 64.64%, H: 7.19%, and N: 1.10%, which were considerably consistent with the C₆₈H₉₀F₃NO₉Si₅ calculated values of C: 64.67%, H: 7.18%, and N: 1.11%.

In a proton nuclear magnetic resonance spectroscopy, the photochromic compound represented by formula (74A) showed 57H peaks attributed to a methyl group, a 4,4-dietylcyclohexyl ring group, a propyloxy group, and a [tris(trimethylsiloxy)silylethyl]silyl group at around δ0.5 to 3.0 ppm, 14H peaks attributed to an indoline ring, a methoxy group, and a propyloxy group at around δ3.0 to 5.0 ppm, and 19H peaks attributed to aromatic protons and protons of an alkene at around δ5.0 to 9.0 ppm.

Furthermore, in a ¹³C-nuclear magnetic resonance spectroscopy, the photochromic compound represented by formula (74A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 123

### First Step

The same reaction as in the first step of Example 119 was performed except that 3-bromo-4-methylbenzoyl chloride was used instead of 4-trifluoromethylbenzoyl chloride and 2,3,4-trifluoro-1,1'-biphenyl was used instead of 2-bromotoluene, to synthesize a benzophenone represented by formula (75A) below with a yield of 83%.

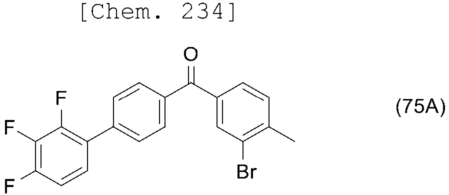

### Second Step

The same reaction as in the first step of Example 98 was performed except that the benzophenone represented by formula (75A) above was used instead of 3-bromo-4-methoxybenzophenon and indoline was used instead of 3-methylindoline, and the same reaction as in the third step of Example 98 was performed except that 1-bromo-4-methoxybutane was used instead of 1-bromo-3-methoxypropane, to synthesize a naphthol compound represented by formula (76A) below.

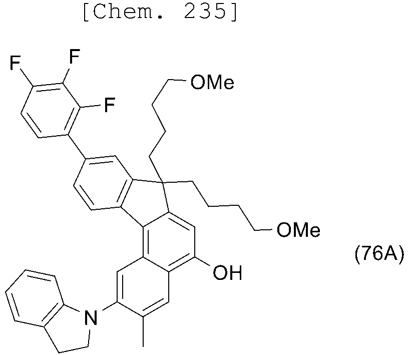

### Third Step

The same reaction as in the fifth step of Example 95 was performed except that the naphthol compound represented by formula (76A) above was used instead of the naphthol compound represented by formula (26A) above and a propargyl alcohol represented by formula (77A) below was used instead of the propargyl alcohol represented by formula (27A) above, to obtain a photochromic compound represented by formula (78A) below with a yield of 71%.

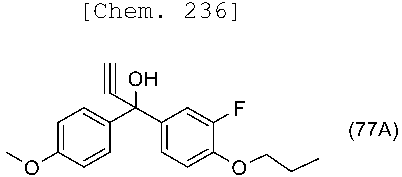

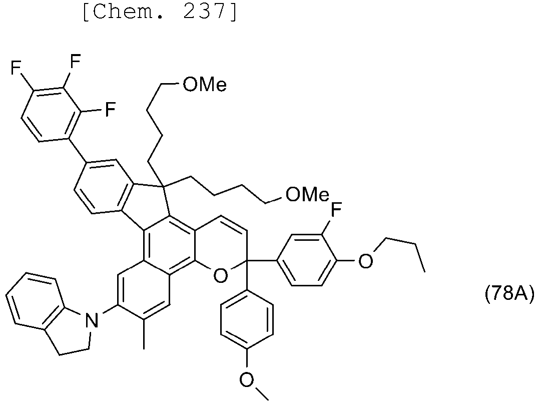

The photochromic compound represented by formula (78A) above had elemental analysis values of C: 76.13%, H: 6.17%, and N: 1.44%, which were considerably consistent with the C₆₁H₅₉F₄NO₅ calculated values of C: 76.15%, H: 6.18%, and N: 1.46%.

In a proton nuclear magnetic resonance spectroscopy, the photochromic compound represented by formula (78A) showed 20H peaks attributed to a methyl group, a 4-methoxybutyl group, and a propyloxy group at around δ0.5 to 3.0 ppm, 19H peaks attributed to an indoline ring, a methoxy group, and a propyloxy group at around δ3.0 to 5.0 ppm, and 20H peaks attributed to aromatic protons and protons of an alkene at around δ5.0 to 9.0 ppm.

Furthermore, in a ¹³C-nuclear magnetic resonance spectroscopy, the photochromic compound represented by formula (78A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 124

### First Step

The same reaction as in the third step of Example 95 was performed except that cyclooctanone was used instead of acetone, to synthesize a naphthol compound represented by formula (79A) below.

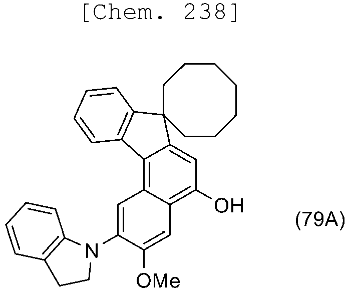

### Second Step

The same reaction as in the fifth step of Example 95 was performed except that the naphthol compound represented by formula (79A) above was used instead of the naphthol compound represented by formula (26A) above and the propargyl alcohol represented by formula (72A) above was used instead of the propargyl alcohol represented by formula (27A) above, to obtain a photochromic compound represented by formula (80A) below with a yield of 67%.

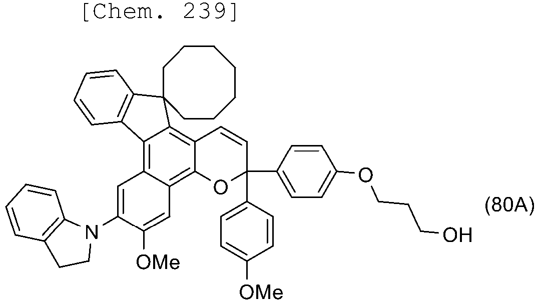

### Third Step

By referring to the method described in Patent Document 9 (PCT International Publication No. WO2019/013249), a compound represented by formula (81A) below synthesized from polytetramethylene glycol having a number-average molecular weight of 1000 was reacted with the photochromic compound represented by formula (80A) above to obtain a photochromic compound represented by formula (82A) below with a yield of 67%.

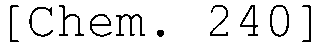

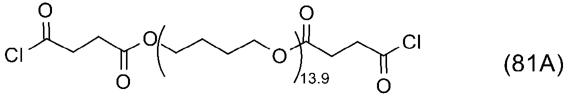

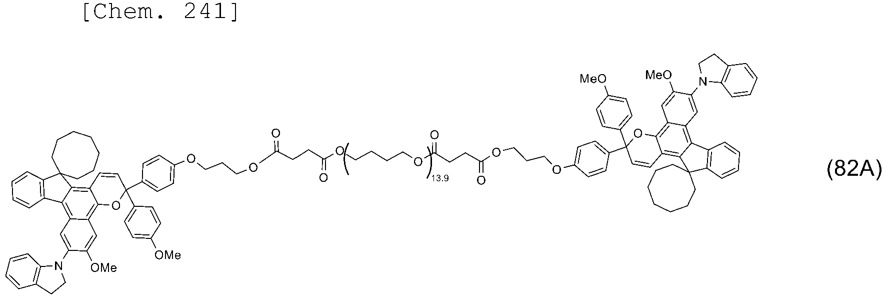

In a proton nuclear magnetic resonance spectroscopy, the photochromic compound represented by formula (82A) above showed about 96H peaks attributed to a cyclooctane ring, a propyloxy group, a succinic acid group, and a polytetramethylene glycol chain at around δ0.5 to 3.0 ppm, about 84H peaks attributed to an indoline ring, a methoxy group, a propyloxy group, and a polytetramethylene glycol chain at around δ3.0 to 5.0 ppm, and 40H peaks attributed to aromatic protons and protons of an alkene at around δ5.0 to 9.0 ppm.

### Example 125

### First Step

The same reaction as in the first step of Example 98 was performed except that (3-bromo-4-methoxyphenyl) [4'-(trifluoromethyl) [1,1'-biphenyl]-4-yl]methanone was used instead of 3-bromo-4-methoxybenzophenone and indoline was used instead of 3-methylindoline, and the same reaction as in the third step of Example 98 was performed except that 1-bromo-2-methoxyethane was used instead of 1-bromo-3-methoxypropane, to synthesize a naphthol compound represented by formula (83A) below.

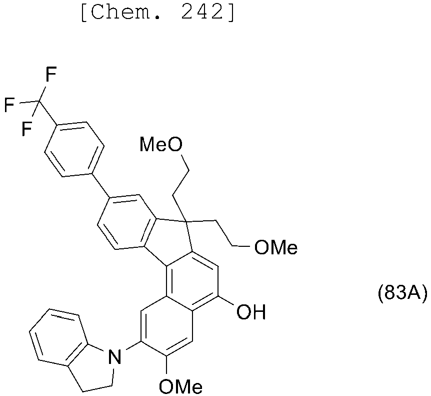

### Second Step

The same reaction as in the fifth step of Example 95 was performed except that the naphthol compound represented by formula (83A) above was used instead of the naphthol compound represented by formula (26A) above and a propargyl alcohol represented by formula (84A) below was used instead of the propargyl alcohol represented by formula (27A) above, to obtain a photochromic compound represented by formula (85A) below with a yield of 75%.

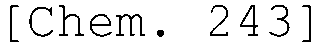

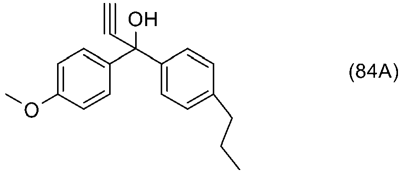

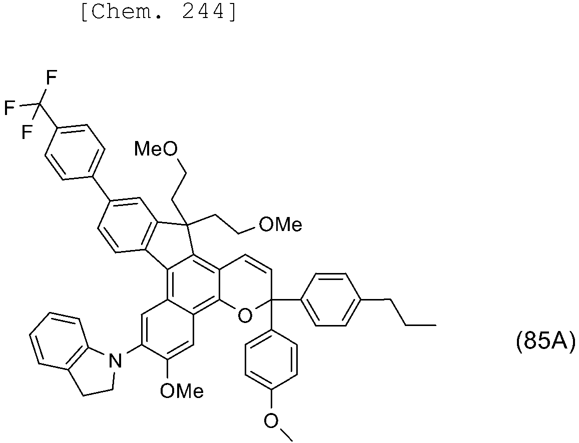

The photochromic compound represented by formula (85A) above had elemental analysis values of C: 77.21%, H: 6.02%, and N: 1.53%, which were considerably consistent with the C₅₈H₅₄F₃NO₅ calculated values of C: 77.23%, H: 6.03%, and N: 1.55%.

In a proton nuclear magnetic resonance spectroscopy, the photochromic compound represented by formula (85A) showed 11H peaks attributed to a 2-methoxyethyl group and a propyl group at around δ0.5 to 3.0 ppm, 20H peaks attributed to an indoline ring, a methoxy group, and a 2-methoxyethyl group at around δ3.0 to 5.0 ppm, and 23H peaks attributed to aromatic protons and protons of an alkene at around δ5.0 to 9.0 ppm.

Furthermore, in a ¹³C-nuclear magnetic resonance spectroscopy, the photochromic compound represented by formula (85A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

### Example 126

### First Step

The same reaction as in the first step of Example 119 was performed except that 3-bromo-4-methoxybenzoyl chloride was used instead of 4-trifluoromethylbenzoyl chloride and 4-(1-methylethoxy)-1,1'-biphenyl was used instead of 2-bromotoluene, to synthesize a benzophenone represented by formula (86A) below with a yield of 85%.

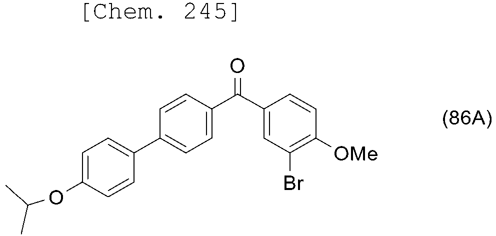

### Second Step

The same reaction as in the first step of Example 98 was performed except that the benzophenone represented by formula (86A) above was used instead of 3-bromo-4-methoxybenzophenone and indoline was used instead of 3-methylindoline, and the same reaction as in the third step of Example 98 was performed except that 1-bromo-4-fluorobutane was used instead of 1-bromo-3-methoxypropane, to synthesize a naphthol compound represented by formula (87A) below.

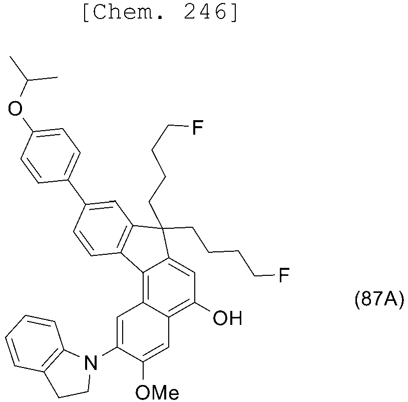

### Third Step

The same reaction as in the fifth step of Example 95 was performed except that the naphthol compound represented by formula (87A) above was used instead of the naphthol compound represented by formula (26A) above and the propargyl alcohol represented by formula (72A) above was used instead of the propargyl alcohol represented by formula (27A) above, to obtain a photochromic compound represented by formula (88A) below with a yield of 62%.

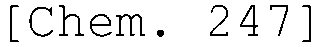

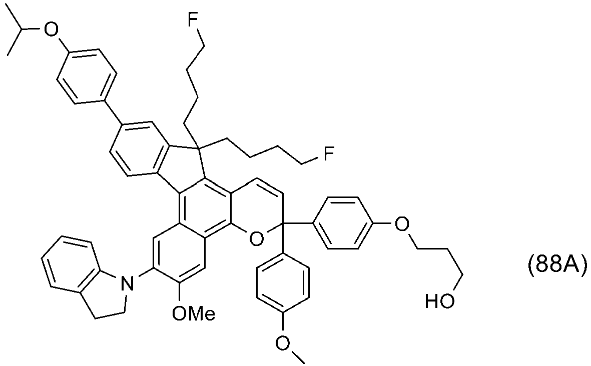

### Fourth Step

The same reaction as in the third step of Example 124 was performed except that sebacoyl chloride was used instead of the compound represented by formula (81A) above, to obtain a photochromic compound represented by formula (89A) below with a yield of 69%.

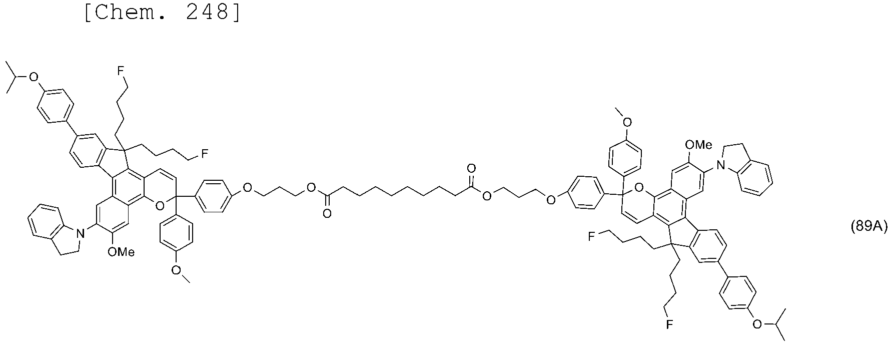

The photochromic compound represented by formula (89A) above had elemental analysis values of C: 77.44%, H: 6.78%, and N: 1.34%, which were considerably consistent with the C₁₃₄H₁₄₀F₄N₂O₁₄ calculated values of C: 77.43%, H: 6.79%, and N: 1.35%.

In a proton nuclear magnetic resonance spectroscopy, the photochromic compound represented by formula (89A) showed 64H peaks attributed to a 4-fluorobutyl group, an isopropyloxy group, a propyloxy group, and a sebacic acid group at around δ0.5 to 3.0 ppm, 30H peaks attributed to an indoline ring, a methoxy group, an isopropyloxy group, and a propyloxy group at around δ3.0 to 5.0 ppm, and 46H peaks attributed to aromatic protons and protons of an alkene at around δ5.0 to 9.0 ppm.

Furthermore, in a ¹³C-nuclear magnetic resonance spectroscopy, the photochromic compound represented by formula (89A) showed a peak attributed to carbon of an aromatic ring at around δ110 to 160 ppm, a peak attributed to carbon of an alkene at around δ80 to 140 ppm, and a peak attributed to carbon of an alkyl at δ20 to 60 ppm.

The analysis results of the naphthol derivatives used in Examples 119 to 126 are summarized in Table 13.

### [Table 13]

**[table 13]**

| | Compound No. | Calculated value | | | Measured value | | | 1H-NMR |
|---|---|---|---|---|---|---|---|---|
| | | C | H | N | C | H | N | |
| Example 119 | Formula (64A) | 77.32 | 6.67 | 2.58 | 77.30 | 6.66 | 2.59 | δ0.5-5.0ppm 25H |
| | | | | | | | | δ5.0-9.0ppm 11H |
| Example 120 | Formula (67A) | 67.25 | 4.76 | 2.45 | 67.23 | 4.74 | 2.45 | δ0.5-5.0ppm 15H |
| | | | | | | | | δ5.0-9.0ppm 12H |
| Example 121 | Formula (70A) | 81.95 | 6.30 | 2.65 | 81.96 | 6.31 | 2.64 | δ0.5-5.0ppm 17H |
| | | | | | | | | δ5.0-9.0ppm 16H |
| Example 122 | Formula (74A) | 73.58 | 6.17 | 2.38 | 73.56 | 6.16 | 2.37 | δ0.5-5.0ppm 25H |
| | | | | | | | | δ5.0-9.0ppm 11H |
| Example 123 | Formula (78A) | 75.77 | 6.36 | 2.10 | 75.76 | 6.34 | 2.10 | δ0.5-5.0ppm 29H |
| | | | | | | | | δ5.0-9.0ppm 13H |
| Example 124 | Formula (82A) | 83.33 | 6.99 | 2.94 | 83.34 | 7.00 | 2.94 | δ0.5-5.0ppm 21H |
| | | | | | | | | δ5.0-9.0ppm 12H |
| Example 125 | Formula (85A) | 73.22 | 5.67 | 2.19 | 73.20 | 5.66 | 2.18 | δ0.5-5.0ppm 21H |
| | | | | | | | | δ5.0-9.0ppm 15H |
| Example 126 | Formula (89A) | 78.04 | 6.85 | 2.12 | 78.05 | 6.83 | 2.13 | δ0.5-5.0ppm 30H |
| | | | | | | | | δ5.0-9.0ppm 15H |

### (Examples 127 to 134)

In the same manner as in Example 23, photochromic laminates were prepared using the photochromic compounds obtained in Examples 119 to 126. Note that, as for the photochromic compounds in Examples 124 and 126, the photochromic compound was added to the curable composition so that the amount of the photochromic compound was 0.125 mmol based on 100 g of the total amount of the radical-polymerizable monomers. The evaluation results are summarized in Table 14.

### [Table 14]

**[table 14]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 127 | Example 119 Formula (64A) | 523 | 1.10 | 0.71 | 65% | 66 | 64% |
| Example 128 | Example 120 Formula (67A) | 527 | 1.52 | 1.00 | 66% | 73 | 73% |
| Example 129 | Example 121 Formula (70A) | 536 | 1.81 | 1.39 | 77% | 142 | 71% |
| Example 130 | Example 122 Formula (74A) | 522 | 1.25 | 0.80 | 64% | 55 | 72% |
| Example 131 | Example 123 Formula (78A) | 524 | 1.34 | 0.91 | 68% | 70 | 64% |
| Example 132 | Example 124 Formula (82A) | 520 | 1.53 | 1.10 | 72% | 100 | 73% |
| Example 133 | Example 125 Formula (85A) | 530 | 1.59 | 1.11 | 69% | 56 | 65% |
| Example 134 | Example 126 Formula (89A) | 535 | 1.77 | 1.32 | 75% | 93 | 69% |

### <Physical Property Evaluation for Photochromic Layer Prepared by Binder Method>

### (Examples 135)

A binder sheet was prepared by the following method. The binder sheet consisted of a first optical sheet, a first adhesive layer, a photochromic layer, a second adhesive layer, and a second optical sheet, laminated in this order. The first optical sheet and the second optical sheet were made of a polycarbonate sheet with a thickness of 400 µm.

### (Preparation of Photochromic Layer Forming Composition)

A 2L four-necked flask equipped with agitation blades, a cooling tube, a thermometer, and a nitrogen gas introducing tube was charged with 315 parts by mass of polycarbonate diol having a number-average molecular weight of 1000, 100 parts by mass of isophorone diisocyanate, and 72 parts by mass of toluene, and the mixture was reacted under nitrogen atmosphere at 100°C for 7 hours to synthesize a urethane prepolymer having an isocyanate group on its terminal. After completion of the urethane prepolymer reaction, the reaction liquid was cooled to approximately 0°C, then dissolved in 205 parts by mass of t-butyl alcohol and 382 parts by mass of diethyl ketone, and then the liquid temperature was maintained at 0°C. Subsequently, into this reaction liquid, a mixture of 21.3 parts by mass of bis-(4-aminocyclohexyl)methane as a chain extender and 20 parts by mass of diethyl ketone was dripped over within 30 minutes, and the mixture was reacted at 0°C for 1 hour. Then, into the mixture, 8.1 parts by mass of 1,2,2,6,6-pentamethyl-4-aminopiperidine was dripped, and the mixture was reacted at 0°C for 1 hour to obtain a diethyl ketone solution of a terminal non-reactive urethaneurea resin.

To 100 parts by mass of the obtained terminal non-reactive urethaneurea resin solution, the photochromic compound in Example 95 (represented by formula (28A) above), 6.3 parts by mass of 4,4'-methylenebis(cyclohexyl isocyanate) isomer mixture (polyisocyanate compound), further 0.4 part by mass of ethylene bis(oxyethylene)bis[3-(5-tert-butyl-4-hydroxy-m-tolyl)propionate] as an antioxidant, and 0.06 part by mass of L-7001 manufactured by Dow Corning Toray Co., Ltd. as a surfactant were added, and the mixture was stirred and mixed at room temperature to obtain a photochromic layer forming composition.

Note that the addition amount of the photochromic compound was 0.25 mmol based on 100 parts by mass of the terminal non-reactive urethane urea resin.

### (Preparation of Adhesive Layer Composition)

A 5L separable flask (four-necked) equipped with agitation blades, a cooling tube, a thermometer, and a nitrogen gas introducing tube was charged with 400 parts by mass of polycarbonate diol having a number-average molecular weight of 1000, 175 parts by mass of isophorone diisocyanate, and 120 parts by mass of toluene, and the mixture was reacted under nitrogen atmosphere at 110°C for 7 hours to synthesize a urethane prepolymer having an isocyanate group on its terminal. After completion of the urethane prepolymer reaction, the reaction liquid was cooled to approximately 20°C, then dissolved in 2500 parts by mass of propylene glycol monomethyl ether, and then the liquid temperature was maintained at 20°C. Subsequently, into this reaction liquid, 60 parts by mass of isophorone diamine as a chain extender was dripped, and the mixture was reacted at 20°C for 1 hour. Then, into this mixture, 3 parts by mass of n-butylamine was dripped, and the mixture was reacted at 20°C for 1 hour to obtain a propylene glycol-monomethyl ether solution of a terminal non-reactive urethaneurea resin.

To 500 parts by mass of the obtained terminal non-reactive urethaneurea resin solution, 0.2 part by mass of L-7001 manufactured by Dow Corning Toray Co., Ltd. as a surfactant was added, and the mixture was stirred and mixed at room temperature to obtain an adhesive layer composition.

### (Production of Binder Sheet)

Using a coater (manufactured by TESTER SANGYO CO., LTD.), the adhesive layer composition was applied onto one main face of the first optical sheet at a coating speed of 0.5 m/min, and dried at a drying temperature of 110°C for 3 minutes to obtain a first optical sheet having a 5 µm-thick first coating film. In the same manner as above, the adhesive layer composition was applied onto one main face of the second optical sheet to obtain a second optical sheet having a second coating film.

Subsequently, using a coater (manufactured by TESTER SANGYO CO,. LTD.), a photochromic layer forming composition was applied onto a 50 µm-thick OPP film (stretched polypropylene film) at a coating speed of 0.3 m/min, and dried at a drying temperature of 100°C for 5 minutes. Thereby, a third coating film was obtained. Subsequently, the third coating film and the first optical sheet were bonded to each other so that the third coating film was in contact with the first coating film. From this structure, the OPP film was peeled off, and the second optical sheet and the third coating film were bonded to each other so that the exposed other main face of the third coating film was in contact with the second coating film. Subsequently, the resulting laminate was allowed to stand under vacuum at 40°C for 24 hours, then heated at 110°C for 60 minutes, then humidified at 60°C and 100% RH for 24 hours, and finally allowed to stand under vacuum at 40°C for 24 hours to obtain a binder sheet. The obtained binder sheet was evaluated in the same manner as in Example 23. The results are presented in Table 7.

### (Examples 136 to 146, and Comparative Examples 16)

Binder sheets were prepared by the same method as in Example 135 using photochromic compounds presented in Tables 15 and 16. The results of Examples 135, 136, and Comparative Examples 9, 10, 12, and 16 are summarized in Table 15, and the results of Examples 137 to 146 are summarized in Table 16.

### [Table 15]

**[table 15]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 135 | Example 95 Formula (28A) | 532 | 1. 60 | 1.19 | 74% | 140 | 88% |
| Example 136 | Example 103 Formula (52A) | 535 | 1.72 | 1.29 | 75% | 156 | 92% |
| Comparative Example 9 | Formula (A) | 474 | 1.69 | 1.16 | 69% | 189 | 70% |
| | | 566 | 0.85 | 0.57 | 68% | 193 | 76% |
| Comparative Example 10 | Formula (E) | 481 | 1.85 | 1.30 | 70% | 217 | 68% |
| Comparative Example 12 | Formula (G) | 493 | 2.29 | 1.92 | 84% | 385 | 49% |
| Comparative Example 16 | Formula (I) | 522 | 1. 69 | 1. 22 | 72% | 151 | 84% |

### [Table 16]

**[table 16]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 137 | Example 97 Formula (35A) | 531 | 1.75 | 1.37 | 78% | 157 | 90% |
| Example 138 | Example 98 Formula (39A) | 533 | 1.53 | 1.14 | 75% | 93 | 85% |
| Example 139 | Example 99 Formula (42A) | 535 | 1. 66 | 1.24 | 75% | 155 | 85% |
| Example 140 | Example 101 Formula (48A) | 539 | 1.09 | 0.67 | 62% | 35 | 84% |
| Example 141 | Example 102 Formula (50A) | 522 | 1.46 | 1. 02 | 70% | 75 | 84% |
| Example 142 | Example 105 Formula (57A) | 548 | 1.72 | 1.21 | 70% | 153 | 80% |
| Example 143 | Example 120 Formula (67A) | 527 | 1.55 | 1. 02 | 66% | 81 | 83% |
| Example 144 | Example 121 Formula (70A) | 535 | 1.85 | 1.42 | 77% | 159 | 82% |
| Example 145 | Example 122 Formula (74A) | 522 | 1.28 | 0.82 | 64% | 62 | 83% |
| Example 146 | Example 123 Formula (78A) | 523 | 1.37 | 0.93 | 68% | 78 | 82% |

### <Physical Property Evaluation for Photochromic cured product Prepared by Kneading Method>

### (Examples 147)

### (Preparation of Curable Composition)

First, the photochromic compound obtained in Example 95, additives, and a polymerizable compound were mixed to obtain a curable composition.

As the polymerizable compound, a compound composed of a combination of the following polymerizable monomers was used. 1,3-bis(isocyanatomethyl)cyclohexane: 36.7 parts by mass Pentaerythritol tetrakis(3-mercaptopropionate): 39.4 parts by mass
Polyoxyethylene polyoxypropylene lauryl ether (Wonder Surf 140 manufactured by Aoki Oil Industrial Co., Ltd.): 17.4 parts by mass
1-Decanethiol: 2.8 parts by mass
RX-1 adjusted according to the method described in Patent Document 10: 3.8 parts by mass

Note that the photochromic compound was added to the curable composition so that the amount of the photochromic compound was 0.106 mmol based on 100 g of the total amount of the polymerizable monomer.

The following additives were used:
Dimethyltin dichloride
   : 0.05 part by mass
Irganox 245: 0.1 part by mass
2-ethylhexyl 4-methoxycinnamate: 0.6 part by mass

### (Production of Cured Product)

The prepared curable composition was thoroughly defoamed, then injected into a glass mold having a 1 mm gap, and cast-polymerized. The polymerization was carried out in an air furnace while gradually raising the temperature from 27°C to 120°C over 18 hours. After the polymerization, the cured product was removed from the glass mold to obtain a photochromic cured product having a thickness of 1 mm. The obtained photochromic cured product was evaluated in the same manner as in Example 23. The results are presented in Table 17.

### (Examples 148 to 155, and Comparative Example 14 and 17)

Photochromic cured products were prepared using photochromic compounds presented in Table 17 in the same manner as in Example 147. The results are summarized in Table 17.

### [Table 17]

**[table 17]**

| | Compound No. | Maximum absorption wavelength (nm) | A23 (-) | A35 (-) | A35/A23 *100 (%) | 23°C color fading half-life (sec) | Residual rate (%) |
|---|---|---|---|---|---|---|---|
| Example 147 | Example 95 Formula (28A) | 536 | 1.61 | 1.21 | 75% | 176 | 79% |
| Example 148 | Example 97 Formula (35A) | 535 | 1.76 | 1.40 | 80% | 199 | 80% |
| Example 149 | Example 98 Formula (39A) | 537 | 1.54 | 1. 17 | 76% | 117 | 76% |
| Example 150 | Example 99 Formula (42A) | 539 | 1. 67 | 1.27 | 76% | 196 | 75% |
| Example 151 | Example 102 Formula (50A) | 526 | 1.47 | 1. 05 | 71% | 95 | 76% |
| Example 152 | Example 103 Formula (52A) | 539 | 1.73 | 1.32 | 76% | 198 | 82% |
| Example 153 | Example 120 Formula (67A) | 531 | 1.56 | 1. 05 | 67% | 103 | 75% |
| Example 154 | Example 121 Formula (70A) | 540 | 1.86 | 1.45 | 78% | 201 | 77% |
| Example 155 | Example 122 Formula (74A) | 526 | 1.29 | 0.84 | 65% | 78 | 77% |
| Comparative Example 14 | Formula (A) | 476 | 1.75 | 1.22 | 70% | 247 | 60% |
| | | 573 | 0.82 | 0.57 | 70% | 248 | 65% |
| Comparative Example 17 | Formula (I) | 526 | 1.70 | 1.25 | 73% | 191 | 74% |

Preferable aspects of the present invention will be described below.
[1] A photochromic compound having a skeleton represented by formula (1) below: 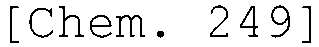 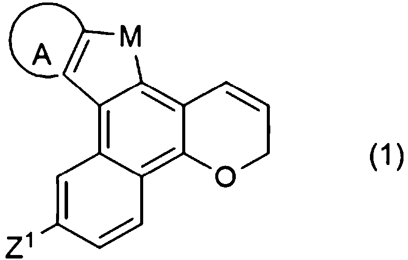 wherein, in formula (1) above,
   M is C, Si, or Ge,
   ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   Z¹ is a group represented by formula (1a) below or a group represented by formula (1b) below, 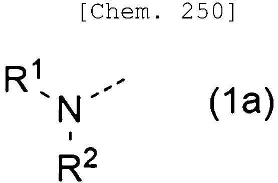 in formula (1a) above,
   R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
   R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below,

      -Q¹-(X¹Q²)a-X²Q³ (2a)
   in formula (2a) above,
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰, PR⁷⁰¹, or P(=O), R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
   a is an integer of 0, or 1 or more and 3 or less, 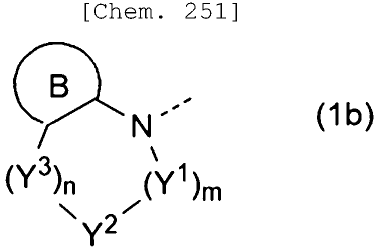 in formula (1b),
   ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Y¹ is a substituted or unsubstituted methylene group,
   Y² is a substituted or unsubstituted methylene group, O, S, SO₂, NR⁶⁰⁰, R⁶⁰¹C=CR⁶⁰², or CC,
   R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
   Y³ is a substituted or unsubstituted methylene group, and m is an integer of 1 to 4, n is an integer of 0 to 4, and m+n is an integer of 2 or more.
[2] The photochromic compound as described in [1], in which the photochromic compound has a skeleton represented by formula (2) below: 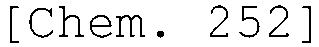 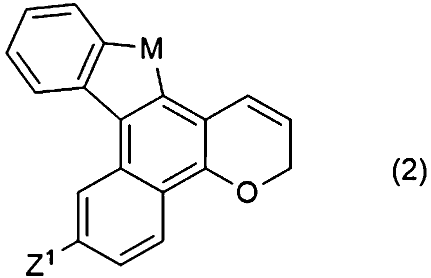 in formula (2) above, Z¹ and M are each the same as those in formula (1) above.
[3] The photochromic compound as described in [1] or [2], in which the photochromic compound has a skeleton represented by formula (3) below: 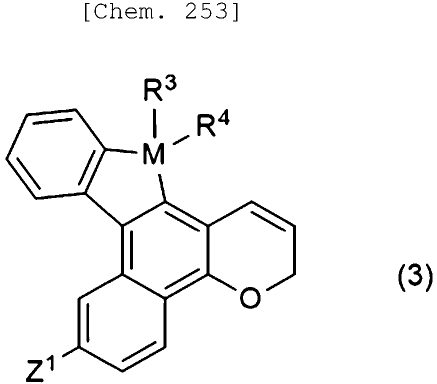 in formula (3) above, Z¹ and M are each the same as those in formula (1) above,
   R³ and R⁴ are each independently a hydrogen atom, a hydroxy group, a substituted or unsubstituted alkyl group, a haloalkyl group, a group represented by formula (2a) above, a substituted or unsubstituted cycloalkyl group, an alkoxy group, an alkoxyalkyl group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, or a substituted or unsubstituted heteroaryl group, or a group represented by formula (X3) below,

      L¹-R⁴⁰⁰ (X3)
   in formula (X3) above,
   R⁴⁰⁰ is a hydrogen atom, an alkyl group, an aryl group, a polymerizable group, a photochromic group, or a silyl group having, as a substituent, an alkyl group, an alkoxy group, or an aryl group,
   L¹ is a group represented by formula (X2) below, 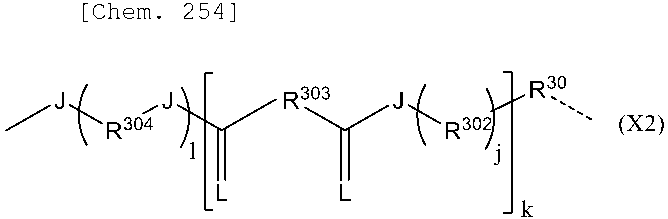 in formula (X2) above, R³⁰ is a group represented by formula (X2a) below, 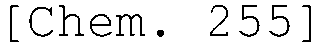 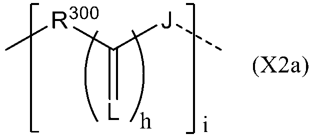 in formulas (X2) and (X2a) above,
   J represents a divalent group, each independently being directly bonded, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group or a silylene group having an alkyl group or an aryl group as a substituent,
   R³⁰², R³⁰³, and R³⁰⁴ are each independently an alkylene group, h, j, k, and 1 are each independently an integer of 0 or 1, i is an integer of 1 to 200,
   when i is 2 or more, the structures of a plurality of R³⁰ groups may be the same as or different from each other, and the dashed lines indicate bonds with R⁴⁰⁰, and
   R³ and R⁴, together with M, may collectively constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring.
[4] The photochromic compound as described in [3], in which the photochromic compound is represented by formula (4) below: 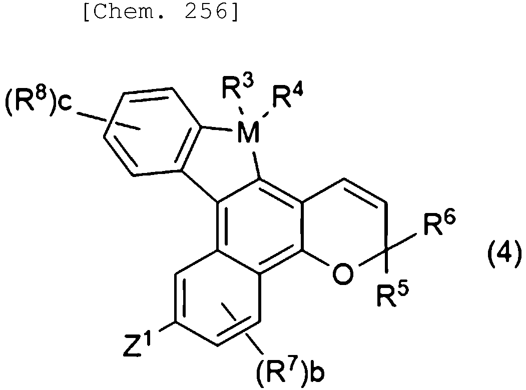 in formula (4) above, R³, R⁴, Z¹, and M are each the same as those in formula (3) above,
   R⁵ and R⁶ are each independently a substituted or unsubstituted aryl group, or a substituted or unsubstituted heteroaryl group,
   R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) above,
   b is an integer of 0 to 3, c is an integer of 0 to 4,
   when b is 2 to 3, a plurality of R⁷ groups may be the same as or different from each other,
   when c is 2 to 4, a plurality of R⁸ groups may be the same as or different from each other,
   when b is 2 to 3 and there are a plurality of R⁷ groups adjacent to each other, the two adjacent R⁷ groups, together with carbon atoms to which the R⁷ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, when c is 2 to 4 and there are a plurality of R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, 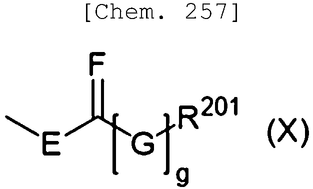 in formula (X),
   E is an oxygen atom or NR¹⁰¹, and R¹⁰¹ is a hydrogen atom or an alkyl group,
   F is an oxygen atom or a sulfur atom,
   G is an oxygen atom, a sulfur atom, or NR²⁰², and R²⁰² is a hydrogen atom, an alkyl group, a cycloalkyl group, an aryl group, or a heteroaryl group,
   g is 0 or 1,
   R²⁰¹ is a hydrogen atom, an alkyl group, a cycloalkyl group, an aryl group, or a heteroaryl group, and
   when G is an oxygen atom or a sulfur atom, R²⁰¹ is a group other than a hydrogen atom.
[5] The photochromic compound as described in [4], in which photochromic compound is represented by formula (5) below: 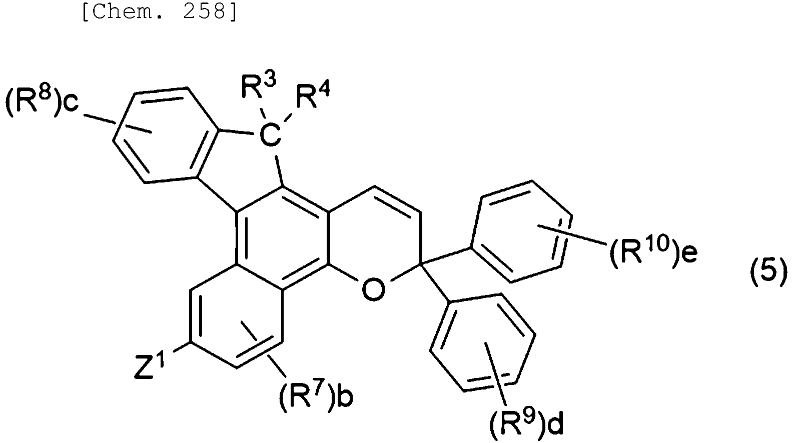 in formula (5) above,
   R³, R⁴, R⁷, R⁸, Z¹, b, and c are each independently the same as those in formula (4) above,
   R⁹ and R¹⁰ are each independently a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by (X3) above,
   d is an integer of 0 to 5,
   when d is 2 to 5, a plurality of R⁹ groups may be the same as or different from each other,
   when there are a plurality of R⁹ groups adjacent to each other, the two adjacent R⁹ groups, together with carbon atoms to which the R⁹ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   e is an integer of 0 to 5,
   when e is 2 to 5, a plurality of R¹⁰ groups may be the same as or different from each other, and
   when there are a plurality of R¹⁰ groups adjacent to each other, two adjacent R¹⁰ groups, together with carbon atoms to which the R¹⁰ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[6] The photochromic compound as described in any one of [1] to [5], wherein the photochromic compound has a skeleton represented by formula (7) below: 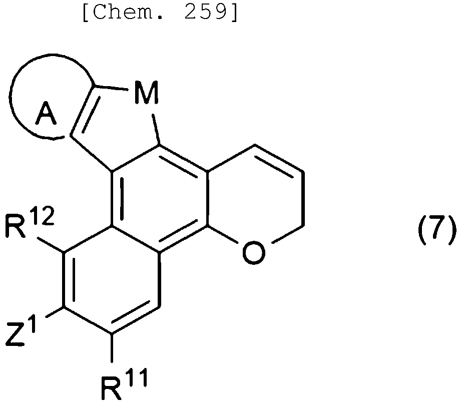 in formula (7) above, M, Z¹, and ring A are each the same as those in formula (1) above,
   R¹¹ is a hydrogen atom, a halogen atom, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a substituted or unsubstituted alkylthio group, a substituted or unsubstituted arylthio group, a substituted or unsubstituted aralkyl group, a substituted or unsubstituted aralkoxy group, or a substituted or unsubstituted aryloxy group, and
   R¹² is a hydrogen atom, a halogen atom, a substituted or unsubstituted alkyl group, a haloalkyl group, a substituted or unsubstituted alkoxy group, a substituted or unsubstituted amino group, a substituted or unsubstituted alkylthio group, a substituted or unsubstituted arylthio group, a substituted or unsubstituted aralkyl group, a substituted or unsubstituted aralkoxy group, or a substituted or unsubstituted aryloxy group.
[7] The photochromic compound as described in any one of [1] to [6], in which Z¹ has a group represented by formula (1a) above, and R¹ is a substituted or unsubstituted phenyl group.
[8] The photochromic compound as described in any one of [1] to [7], in which Z¹ has a group represented by formula (1a) above, and R² is a substituted or unsubstituted alkyl group having 1 or more and 5 or less carbon atoms or a substituted or unsubstituted phenyl group.
[9] The photochromic compound as described in any one of [1] to [6], in which Z¹ has a group represented by formula (1b) below, and ring B is a substituted or unsubstituted phenyl group.
[10] The photochromic compound as described in any one of [1] to [6] and [9], in which Z¹ has a group represented by formula (1b) below, and m and n are each 1.
[11] A curable composition including the photochromic compound as described in any one of [1] to [10], and at least one selected from a group consisting of a radical polymerizable monomer, a cationic polymerizable monomer, a compound having a polymerizable group, and a (thio)urethane(urea) polymer.
[12] A cured product of the curable composition as described in [11].
[13] An optical article including the cured product as described in [12].
[14] A lens including the photochromic compound as described in any one of [1] to [10].
[15] Eyeglasses including the lens as described in [14].
[16] A naphthol derivative having a skeleton represented by formula (6) below: 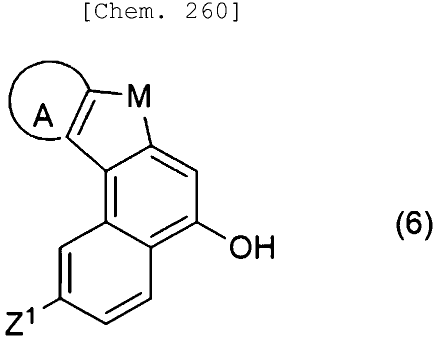 in which, in formula (6) above,
   M is C, Si, or Ge,
   ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   Z¹ is a group represented by formula (1a) below or a group represented by formula (1b) below, 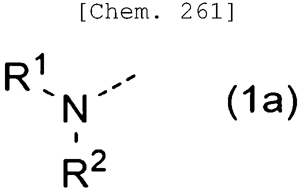
   in formula (1a) above,
   R¹ is a substituted or unsubstituted aryl group, or a substituted or unsubstituted heteroaryl group,
   R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below,

      -Q¹- (X¹Q²) a-X²Q³ (2a)
   in formula (2a) above,
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰R⁷⁰¹, PR⁷⁰²R⁷⁰³, or P(=O)R⁷⁰⁴,
   R⁷⁰⁰, R⁷⁰¹, R⁷⁰², R⁷⁰³, and R⁷⁰⁴ are each independently a hydrogen atom, a substituted or unsubstituted alkyl group, a substituted or unsubstituted cycloalkyl group, a substituted or unsubstituted aryl group, or a substituted or unsubstituted heteroaryl group,
   a is an integer of 0, or 1 or more and 3 or less, 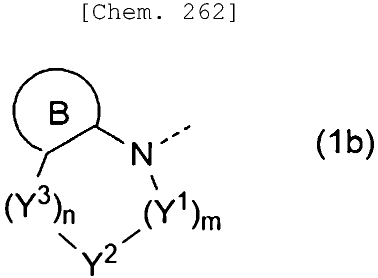
   in formula (1b) above,
   ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   Y¹ is a substituted or unsubstituted methylene group,
   Y² is a substituted or unsubstituted methylene group, O, S, SO₂, NR⁶⁰⁰, R⁶⁰¹C=CR⁶⁰², or CC,
   R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
   Y³ is a substituted or unsubstituted methylene group,
   m is an integer of 1 to 4, n is an integer of 0 to 4, and m+n is an integer of 2 or more.

[1A] A photochromic compound having a skeleton represented by formula (1A) below: 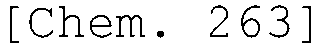 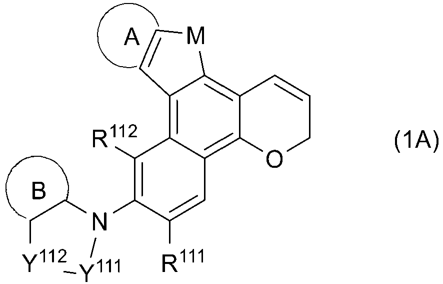 in which, in formula (1A) above,
   M is C, Si, or Ge,
   ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
      Y¹¹¹ is a substituted or unsubstituted methylene group, Y¹¹² is a substituted or unsubstituted methylene group, an oxygen atom, a sulfur atom, NR⁶⁰⁰, or SO₂,
      R⁶⁰⁰ is a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
      R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) below,

         -Q¹- (X¹Q²) a-X²Q³ (2a)

         in formula (2a) above,
         Q¹ is an alkylene group or a haloalkylene group,
         Q² is an alkylene group or a haloalkylene group,
         Q³ is an alkyl group or a haloalkyl group,
         X¹ and X² are each independently O, S, NR⁷⁰⁰, PR⁷¹¹, or P(=O), R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and
         a is 0, or 1 or more and 3 or less.
[2A] The photochromic compound as described in [1A], in which the photochromic compound has a skeleton represented by formula (2A) below: 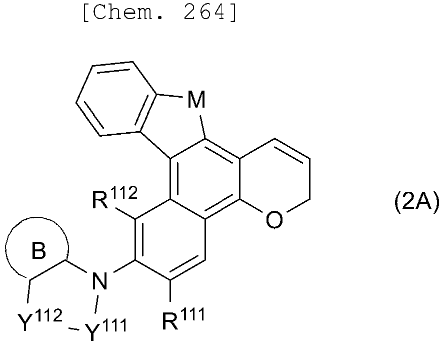 in which, in formula (2) above, ring B, Y¹¹¹, Y¹¹², R¹¹¹, R¹¹², and M are each the same as those in formula (1A) above.
[3A] The photochromic compound as described in [1A] or [2A], in which the photochromic compound has a skeleton represented by formula (3A) below: 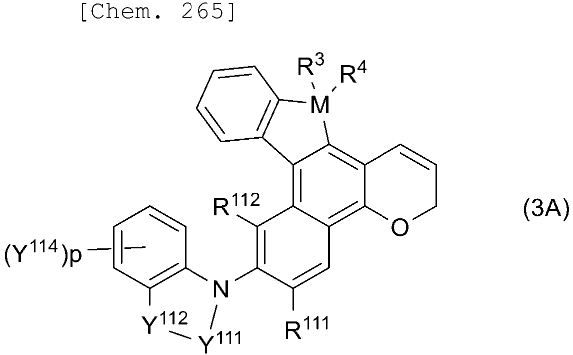 in formula (3) above, Y¹¹¹, Y¹¹², R¹¹¹, R¹¹², and M are each the same as those in formula (1A) above,
   R³ and R⁴ are each independently a hydrogen atom, a hydroxy group, a substituted or unsubstituted alkyl group, a substituted or unsubstituted haloalkyl group, a group represented by formula (2a) above, a substituted or unsubstituted cycloalkyl group, a substituted or unsubstituted alkoxy group, a substituted or unsubstituted alkoxyalkyl group, a formyl group, a hydroxycarbonyl group, a substituted or unsubstituted alkylcarbonyl group, a substituted or unsubstituted alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, or a group represented by formula (X3) below,

      L¹-R⁴⁰⁰ (X3)
   in formula (X3) above,
   R⁴⁰⁰ is a hydrogen atom, an alkyl group, an aryl group, a polymerizable group, a photochromic group, or a silyl group having, as a substituent, an alkyl group, an alkoxy group, or an aryl group,
   L¹ is a group represented by formula (X2) below, 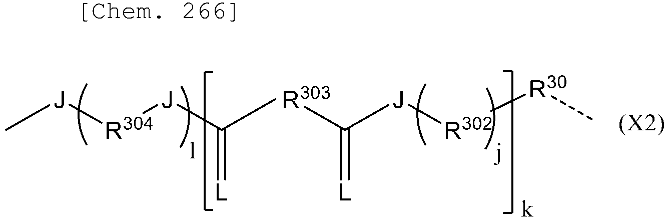 in formula (X2) above, R³⁰ is a group represented by formula (X2a) below, 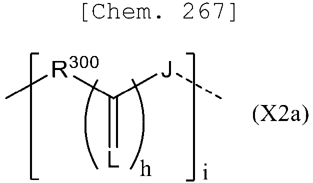 in formula (X2) above and formula (X2a) above,
   J represents a divalent group, each independently being directly bonded, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group, or a silylene group having an alkyl group or an aryl group as a substituent,
   R³⁰², R³⁰³, and R³⁰⁴ are each independently an alkylene group, h, j, k, and l are each independently an integer of 0 or 1, i is an integer of 1 to 200,
   when i is 2 or more, the structures of a plurality of R³⁰ groups may be the same as or different from each other, and the dashed lines indicate bonds with R⁴⁰⁰,
   R³ and R⁴, together with M, may collectively constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring,
   Y¹¹⁴ is a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a substituted or unsubstituted haloalkylthio group, a substituted or unsubstituted cycloalkylthio group, a substituted or unsubstituted silyl group, a substituted or unsubstituted oxysilyl group, or a group represented by formula (2a) above, and
   p is an integer of 0 to 4.
[4A] The photochromic compound as described in [3A], wherein the photochromic compound is represented by formula (4A) below: 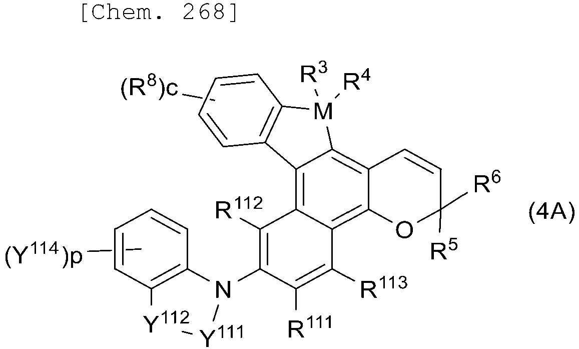 in which, in formula (4A) above, Y¹¹¹, Y¹¹², Y¹¹⁴, R³, R⁴, R¹¹¹, R¹¹², and M are each the same as those in formula (3A) above,
   R⁵ and R⁶ are each independently a substituted or unsubstituted aryl group or a substituted or unsubstituted heteroaryl group, R⁸ and R¹¹³ are each independently a hydrogen atom, a hydroxy group, a substituted or unsubstituted alkyl group, a substituted or unsubstituted haloalkyl group, a substituted or unsubstituted haloalkoxy group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group,
   a substituted or unsubstituted aryl group, a substituted or unsubstituted heteroaryl group, a thiol group, a substituted or unsubstituted alkoxyalkylthio group, a substituted or unsubstituted haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) above,
   c is an integer of 1 to 4,
   when c is 2 to 4, a plurality of R⁸ groups may be the same as or different from each other,
   R¹¹ and R¹³, together with carbon atoms to which R¹¹ and R¹³ bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   when c is 2 to 4 and there are a plurality of R⁸ groups adjacent to each other, the two adjacent R⁸ groups, together with carbon atoms to which the R⁸ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, 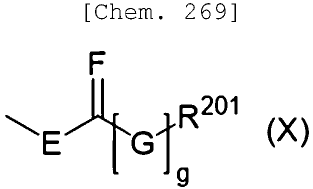 in formula (X),
   E is an oxygen atom or NR¹⁰¹, and R¹⁰¹ is a hydrogen atom or an alkyl group,
   F is an oxygen atom or a sulfur atom,
   G is an oxygen atom, a sulfur atom, or NR²⁰², and R²⁰² is a hydrogen atom, an alkyl group, a cycloalkyl group, an aryl group, or a heteroaryl group,
   g is 0 or 1,
   R²⁰¹ is a hydrogen atom, an alkyl group, a cycloalkyl group, an aryl group, or a heteroaryl group, and
   when G is an oxygen atom or a sulfur atom, R²⁰¹ is a group other than a hydrogen atom.
[5A] The photochromic compound as described in [4A], wherein the photochromic compound is represented by formula (5A) below: 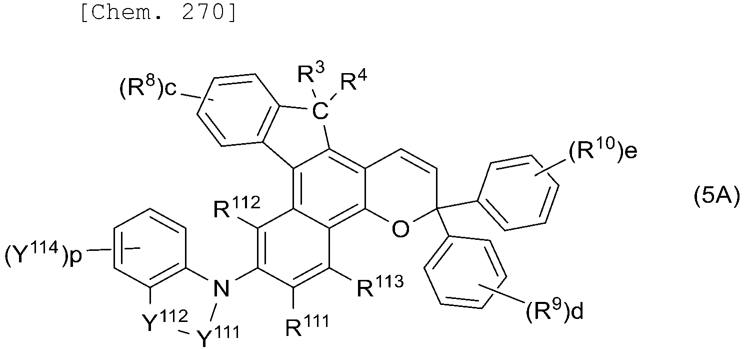 in which, in formula (5A) above,
   Y¹¹¹, Y¹¹², Y¹¹⁴, R³, R⁴, R¹¹¹, R¹¹², R¹¹³, c, and p are each independently the same as those in formula (4) above,
   R⁹ and R¹⁰ are each independently a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a heterocyclic group, a cyano group, a halogen atom, an alkylthio group having 1 to 6 carbon atoms, a substituted or unsubstituted arylthio group having 6 to 10 carbon atoms, a nitro group, a substituted or unsubstituted alkoxyalkylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by (2a) above, or a group represented by (X3) above,
   d is an integer of 0 to 5,
   when d is 2 to 5, the plurality of R⁹ groups may be the same as or different from each other,
   when there are a plurality of R⁹ groups adjacent to each other, the two adjacent R⁹ groups, together with carbon atoms to which the R⁹ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   e is an integer of 0 to 5,
   when e is 2 to 5, the plurality of R¹⁰ groups may be the same as or different from each other, and
   when there are a plurality of R¹⁰ groups adjacent to each other, two adjacent R¹⁰ groups, together with carbon atoms to which the R¹⁰ groups bind, may collectively constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.
[6A] The photochromic compound as described in any one of [1A] to [5A], in which R¹¹¹ and R¹¹² are a hydrogen atom, a halogen atom, a substituted or unsubstituted alkyl group, a haloalkyl group, a substituted or unsubstituted alkoxy group, a substituted or unsubstituted amino group, a substituted or unsubstituted heterocyclic group, a substituted or unsubstituted arylthio group, or a substituted or unsubstituted aryloxy group.
[7A] A curable composition including the photochromic compound as described in any one of [1A] to [6A], and at least one selected from a group consisting of a radical polymerizable monomer, a cationic polymerizable monomer, a compound having a polymerizable group, and a (thio)urethane(urea) polymer.
[8A] A cured product of the curable composition as described in [7A].
[9A] An optical article including the cured product as described in [8A].
[10A] A lens including the photochromic compound as described in any one of [1A] to [6A].
[11A] Eyeglasses including the lens as described in [10A].
[12A] A naphthol derivative having a skeleton represented by formula (6A) below: 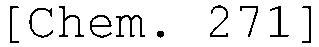 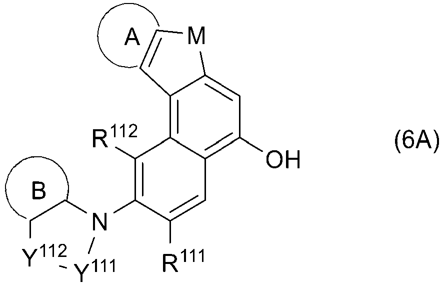 in which, in formula (6A) above,
   M is C, Si, or Ge,
   ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
   Y¹¹¹ is a substituted or unsubstituted methylene group,
   Y¹¹² is a substituted or unsubstituted methylene group, an oxygen atom, a sulfur atom, NR⁶⁰⁰, or SO₂,
   R⁶⁰⁰ is a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
   R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) below,

      -Q¹- (X¹Q²) a-X²Q³ (2a)
   in formula (2a) above,
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰, PR⁷⁰¹, or P(=O),
   R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and
   a is 0, or 1 or more and 3 or less.

## Claims

1. A photochromic compound having a skeleton represented by formula (1) or (1A) below: 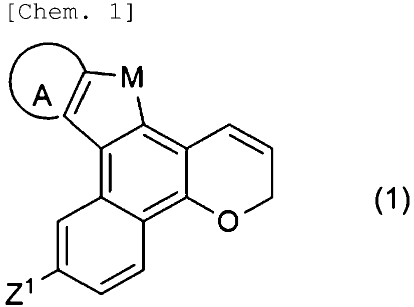 wherein, in formula (1) above,
M is C, Si, or Ge,
ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
Z¹ is a group represented by formula (1a) below or a group represented by formula (1b) below, 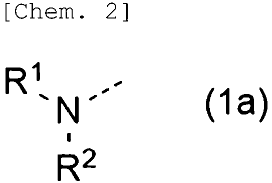
in formula (1a) above,
R¹ is a substituted or unsubstituted aryl group, a substituted or unsubstituted heteroaryl group, or a substituted or unsubstituted fused polycylic ring in which an aromatic ring or an aromatic heterocyclic ring is fused with these substituents,
R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below,
-Q¹- (X¹Q²) a-X²Q³ (2a)
in formula (2a) above,
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰, PR⁷⁰¹, or P(=O),
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group,
a is an integer of 0, or 1 or more and 3 or less, 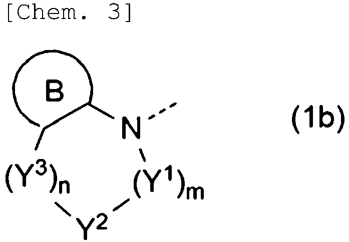
in formula (1b),
ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings. Y¹ is a substituted or unsubstituted methylene group,
Y² is a substituted or unsubstituted methylene group, O, S, SO₂, NR⁶⁰⁰, R⁶⁰¹C=CR⁶⁰², or CC,
R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
Y³ is a substituted or unsubstituted methylene group,
m is an integer of 1 to 4, n is an integer of 0 to 4, and m+n is an integer of 2 or more, 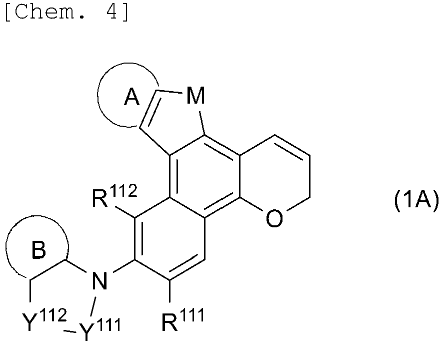
in formula (1A) above,
M, ring A, and ring B are each independently the same as those in formula (1) above,
Y¹¹¹ is a substituted or unsubstituted methylene group,
Y¹¹² is a substituted or unsubstituted methylene group, an oxygen atom, a sulfur atom, NR⁶⁰⁰, or SO₂, and R⁶⁰⁰ is the same as that in formula (1b) above, and
R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) above.

2. The photochromic compound according to claim 1, wherein the photochromic compound has a skeleton represented by formula (2) or (2A) below: 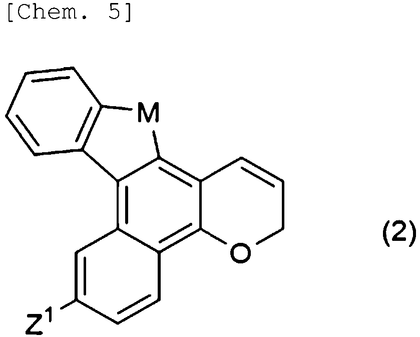 in formula (2) above, Z¹ and M are each the same as those in formula (1) above, 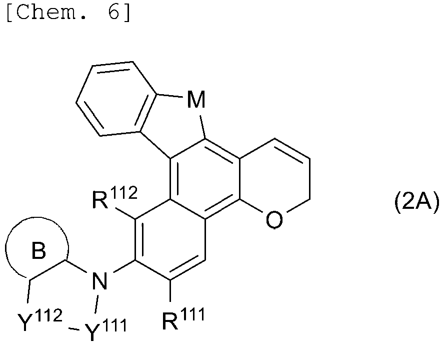 in formula (2A) above, ring B, M, R¹¹¹, R¹¹², Y¹¹¹, and Y¹¹² are each the same as those in formula (1A) above.

3. The photochromic compound according to claim 1 or 2, wherein the photochromic compound has a skeleton represented by formula (3) or (3A) below: 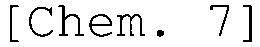 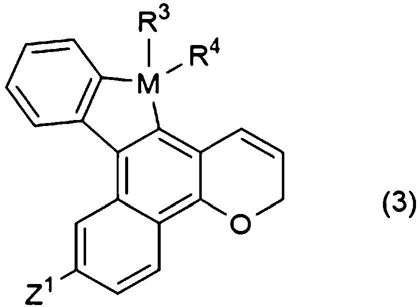
in formula (3) above, Z¹ and M are each the same as those in formula (1) above,
R³ and R⁴ are each independently a hydrogen atom, a hydroxy group, a substituted or unsubstituted alkyl group, a haloalkyl group, a group represented by formula (2a) above, a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, or a group represented by formula (X3) below,
L¹-R⁴⁰⁰ (X3)
in formula (X3) above,
R⁴⁰⁰ is a hydrogen atom, an alkyl group, an aryl group, a polymerizable group, a photochromic group, or a silyl group having, as a substituent, an alkyl group, an alkoxy group, or an aryl group,
L¹ is a group represented by formula (X2) below, 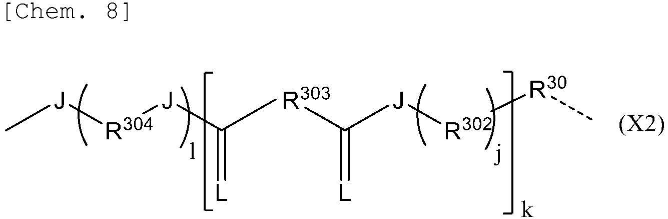
in formula (X2) above, R³⁰ is a group represented by formula (X2a) below, 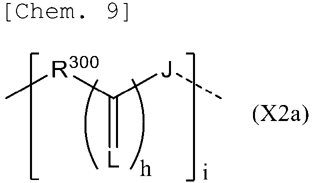
in formulas (X2) and (X2a) above,
J represents a divalent group, each independently being directly bonded, a substituted or unsubstituted methylene group, an oxygen atom, a sulfur atom, or NR³⁰¹, and R³⁰¹ is a hydrogen atom or an alkyl group,
L is an oxygen atom or a sulfur atom,
R³⁰⁰ is an alkylene group or a silylene group having an alkyl group or an aryl group as a substituent,
R³⁰², R³⁰³, and R³⁰⁴ are each independently an alkylene group,
h, j, k, and l are each independently an integer of 0 or 1,
i is an integer of 1 to 200, and when i is 2 or more, a plurality of R³⁰ groups may be the same as or different from each other, and dashed lines indicate bonds with R⁴⁰⁰, 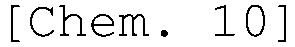 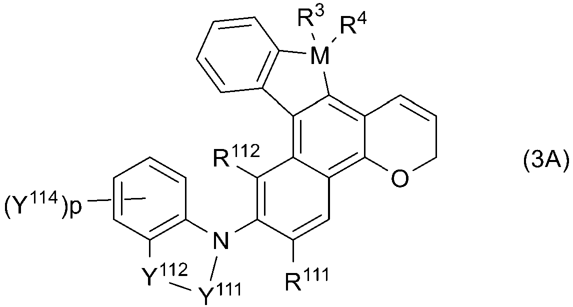
in formula (3A) above, R³ and R⁴ are each independently the same as those in formula (3) above,
R³ and R⁴, together with M, may constitute a substituted or unsubstituted aliphatic ring having 3 to 20 ring-membered carbon atoms, a substituted or unsubstituted fused polycylic ring in which an aromatic hydrocarbon ring or an aromatic heterocyclic ring is fused with the aliphatic ring, a substituted or unsubstituted heterocyclic ring having 3 to 20 ring-membered atoms, or a substituted or unsubstituted fused polycylic ring in which an aromatic ring or an aromatic heterocyclic ring is fused with the heterocyclic ring,
in formula (3A) above, Y¹¹¹, Y¹¹², R¹¹¹, R¹¹², and M are each the same as those in formula (1A) above,
Y¹¹⁴ is a hydroxy group, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a substituted or unsubstituted haloalkylthio group, a substituted or unsubstituted cycloalkylthio group, a substituted or unsubstituted silyl group, a substituted or unsubstituted oxysilyl group, or a group represented by formula (2a) above, and
p is an integer of 0 to 4.

4. The photochromic compound according to claim 3, wherein R³ and R⁴ are each independently a hydrogen atom, a hydroxy group, a substituted or unsubstituted alkyl group, a haloalkyl group, a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, or a group represented by formula (X3) above.

5. The photochromic compound according to claim 3, wherein the photochromic compound is represented by formula (4) or (4A) below: 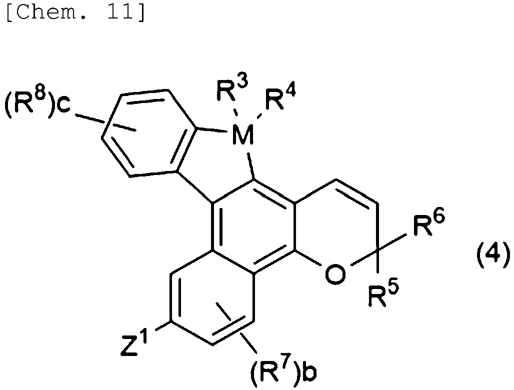
wherein in formula (4) above, R³, R⁴, Z¹, and M are each the same as those in formula (3) above,
R⁵ and R⁶ are each independently a substituted or unsubstituted aryl group, or a substituted or unsubstituted heteroaryl group,
R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, a group represented by formula (2a) above, a group represented by formula (X) below, or a group represented by formula (X3) above,
b is an integer of 0 to 3, and c is an integer of 0 to 4,
when b is 2 to 3, a plurality of R⁷ groups may be the same as or different from each other,
when c is 2 to 4, a plurality of R⁸ groups may be the same as or different from each other,
when b is 2 to 3 and there are a plurality of R⁷ groups adjacent to each other, two adjacent R⁷ groups, together with carbon atoms to which the R⁷ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
when c is 2 to 4 and there are a plurality of R⁸ groups adjacent to each other, the two adjacent R⁸ groups, together with carbon atoms to which the R⁸ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, 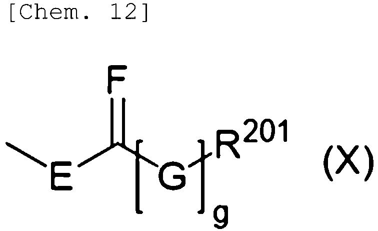
in formula (X) above,
E is an oxygen atom or NR¹⁰¹, and R¹⁰¹ is a hydrogen atom or an alkyl group,
F is an oxygen atom or a sulfur atom,
G is an oxygen atom, a sulfur atom, or NR²⁰², and R²⁰² is a hydrogen atom, an alkyl group, a cycloalkyl group, an aryl group, or a heteroaryl group,
g is 0 or 1,
R²⁰¹ is a hydrogen atom, an alkyl group, a cycloalkyl group, an aryl group, or a heteroaryl group,
when G is an oxygen atom or a sulfur atom, R²⁰¹ is a group other than a hydrogen atom, 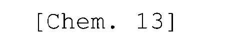 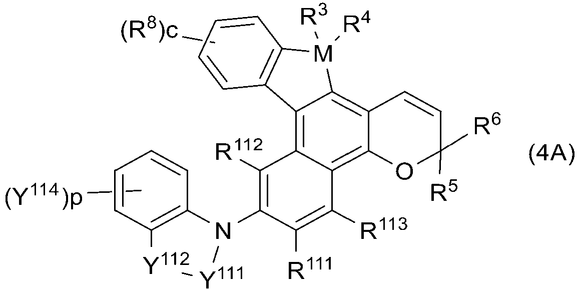
in formula (4A) above, Y¹¹¹, Y¹¹², Y¹¹⁴, R³, R⁴, R¹¹¹, R¹¹², M, and p are each the same as those in formula (3A) above,
R⁵, R⁶, R⁸, and c are the same as those in formula (4) above,
R¹¹³ is a hydrogen atom, a hydroxy group, a substituted or unsubstituted alkyl group, a substituted or unsubstituted haloalkyl group, a substituted or unsubstituted haloalkoxy group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a substituted or unsubstituted haloalkylthio group, a substituted or unsubstituted cycloalkylthio group, a substituted or unsubstituted silyl group, a substituted or unsubstituted oxysilyl group, a group represented by formula (2a) above, a group represented by formula (X) above, or a group represented by formula (X3) above, and
R¹¹¹ and R¹¹³, together with carbon atoms to which R¹¹¹ and R¹¹³ bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings.

6. The photochromic compound according to claim 5, wherein the photochromic compound is represented by formula (5) or (5A) below: 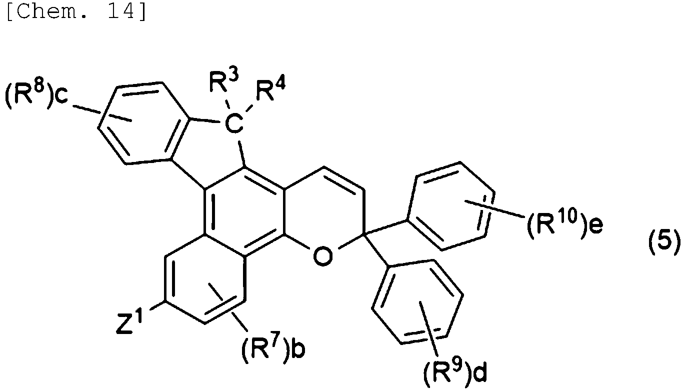 in formula (5) above,
R³, R⁴, R⁷, R⁸, Z¹, b, and c are each independently the same as those in formula (4) above,
R⁹ and R¹⁰ are each independently a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, a group represented by formula (2a) above, or a group represented by formula (X3) above,
d is an integer of 0 to 5,
when d is 2 to 5, the plurality of R⁹ groups may be the same as or different from each other,
when there are a plurality of R⁹ groups adjacent to each other, the two adjacent R⁹ groups, together with carbon atoms to which the R⁹ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
e is an integer of 0 to 5,
when e is 2 to 5, the plurality of R¹⁰ groups may be the same as or different from each other,
when there are a plurality of R¹⁰ groups adjacent to each other, the two adjacent R¹⁰ groups, together with carbon atoms to which the R¹⁰ groups bind, may constitute a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings, 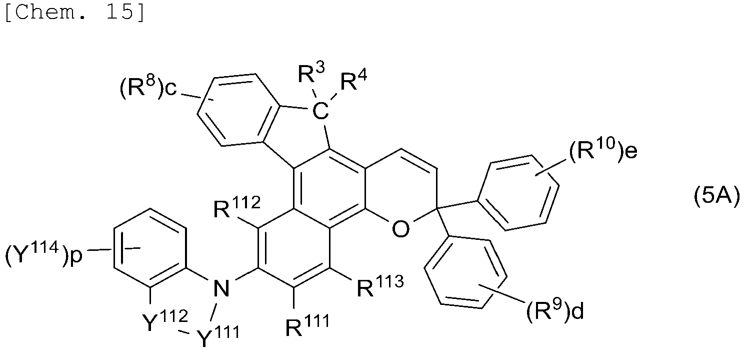
in formula (5A) above,
Y¹¹¹, Y¹¹², Y¹¹⁴, R³, R⁴, R⁸, R¹¹¹, R¹¹², R¹¹³, c, and p are each independently the same as those in formula (4A) above, and
R⁹, R¹⁰, d, and e are each independently the same as those in formula (5) above.

7. The photochromic compound according to claim 1, wherein the photochromic compound has a skeleton represented by formula (7) below: 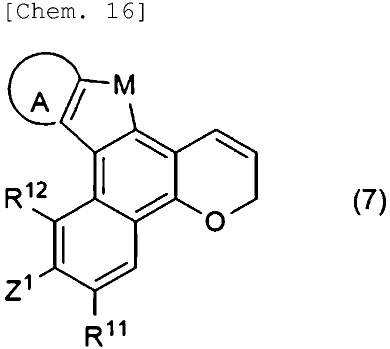
in formula (7) above, M, Z¹, and ring A are each the same as those in formula (1) above,
R¹¹ is a hydrogen atom, a halogen atom, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a substituted or unsubstituted alkylthio group, a substituted or unsubstituted arylthio group, a substituted or unsubstituted aralkyl group, a substituted or unsubstituted aralkoxy group, or a substituted or unsubstituted aryloxy group, and
R¹² is a hydrogen atom, a halogen atom, a substituted or unsubstituted alkyl group, a haloalkyl group, a substituted or unsubstituted alkoxy group, a substituted or unsubstituted amino group, a substituted or unsubstituted alkylthio group, a substituted or unsubstituted arylthio group, a substituted or unsubstituted aralkyl group, a substituted or unsubstituted aralkoxy group, or a substituted or unsubstituted aryloxy group.

8. The photochromic compound according to claim 1, wherein Z¹ has a group represented by formula (1a) above, and R¹ is a substituted or unsubstituted phenyl group.

9. The photochromic compound according to claim 1, wherein Z¹ has a group represented by formula (1a) above, and R² is a substituted or unsubstituted alkyl group having 1 or more and 5 or less carbon atoms or a substituted or unsubstituted phenyl group.

10. The photochromic compound according to claim 1, wherein Z¹ has a group represented by formula (1b) above, and ring B is a substituted or unsubstituted phenyl group.

11. The photochromic compound according to claim 1, wherein Z¹ has a group represented by formula (1b) above, and m and n are each 1.

12. A curable composition comprising the photochromic compound according to claim 1, and
at least one selected from a group consisting of a radical polymerizable monomer, a cationic polymerizable monomer, a compound having a polymerizable group, and a (thio)urethane(urea) polymer.

13. A cured product of the curable composition according to claim 12.

14. An optical article comprising the cured product according to claim 13.

15. A lens comprising the photochromic compound according to claim 1.

16. Eyeglasses comprising the lens according to claim 15.

17. A naphthol derivative having a skeleton represented by formula (6) or (6A) below: 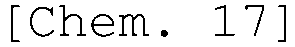 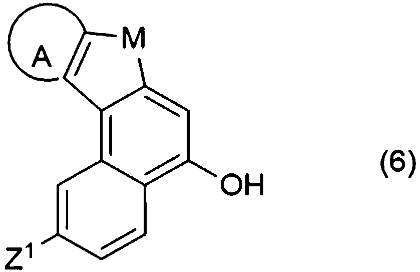 wherein, in formula (6) above,
M is C, Si, or Ge,
ring A is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
Z¹ is a group represented by formula (1a) below or a group represented by formula (1b) below, 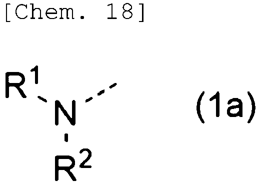
in formula (1a) above,
R¹ is a substituted or unsubstituted aryl group, or a substituted or unsubstituted heteroaryl group,
R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a) below,
-Q¹- (X¹Q²) a-X²Q³ (2a)
in formula (2a) above,
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰R⁷⁰¹, PR⁷⁰²R⁷⁰³, or P(=O)R⁷⁰⁴,
R⁷⁰⁰, R⁷⁰¹, R⁷⁰², R⁷⁰³, and R⁷⁰⁴ are each independently a hydrogen atom, a substituted or unsubstituted alkyl group, a substituted or unsubstituted cycloalkyl group, a substituted or unsubstituted aryl group, or a substituted or unsubstituted heteroaryl group,
a is an integer of 0, or 1 or more and 3 or less, 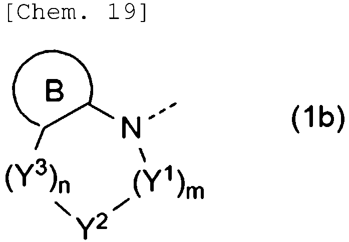
in formula (1b) above,
ring B is a substituted or unsubstituted aromatic ring, a substituted or unsubstituted aromatic heterocyclic ring, or a substituted or unsubstituted fused polycylic ring in which an aromatic ring or an aromatic heterocyclic ring is fused with these rings,
Y¹ is a substituted or unsubstituted methylene group,
Y² is a substituted or unsubstituted methylene group, O, S, SO₂, NR⁶⁰⁰, R⁶⁰¹C=CR⁶⁰², or CC,
R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
Y³ is a substituted or unsubstituted methylene group,
m is an integer of 1 to 4, n is an integer of 0 to 4, m+n is an integer of 2 or more, 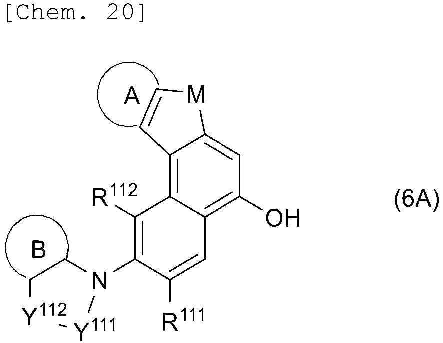
in formula (6A) above,
M, ring A, and ring B are each independently the same as those in formula (6) above,
Y¹¹¹ is a substituted or unsubstituted methylene group,
Y¹¹² is a substituted or unsubstituted methylene group, an oxygen atom, a sulfur atom, NR⁶⁰⁰, or SO₂, and R⁶⁰⁰ is the same as that in formula (1b) above, and
R¹¹¹ and R¹¹² are each independently a hydrogen atom, a halogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted amino group, a substituted or unsubstituted alkylthio group, a hydroxy group, a substituted or unsubstituted heterocyclic group, a cyano group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a thiol group, a substituted or unsubstituted haloalkylthio group, a substituted or unsubstituted haloalkoxy group, a substituted or unsubstituted cycloalkylthio group, a substituted or unsubstituted silyl group, a substituted or unsubstituted oxysilyl group, or a group represented by formula (2a) above.
